(19) Europäisches Patentamt
European Patent Office
Office européen des brevets

(11) EP 2 842 566 A1

(12) EUROPEAN PATENT APPLICATION

(43) Date of publication:
**04.03.2015 Bulletin 2015/10**

(21) Application number: **13306198.6**

(22) Date of filing: **03.09.2013**

(51) Int Cl.:
***A61K 38/17*** *(2006.01)* ***C07K 14/47*** *(2006.01)*
***G01N 33/50*** *(2006.01)* ***A61P 25/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Centre National de la Recherche Scientifique**
**75016 Paris (FR)**

(72) Inventors:
- **Gautreau, Alexis**
**91300 Massy (FR)**
- **Alexandrova, Antonina**
**Moscou 123098 (RU)**
- **Vacher, Sophie**
**92350 Le Plessis Robinson (FR)**
- **Sousa-Blin, Carla**
**EH16 5RG Ediburgh (GB)**
- **Derivery, Emmanuel**
**1202 Geneve (CH)**
- **Gorelik, Roman**
**91940 Les Ulis (FR)**

(74) Representative: **Leblois-Préhaud, Hélène Marthe Georgette**
**Cabinet Orès**
**36, rue de St Pétersbourg**
**75008 Paris (FR)**

# (54) USE OF ARPIN A NEW INHIBITOR OF THE ARP2/3 COMPLEX FOR THE DIAGNOSIS AND TREATMENT OF DISEASES

(57) The present invention relates to the diagnostic and therapeutic uses of Arpin, a protein from the Uncharacterized Protein Family UPF0552, which is a new inhibitor of the Arp2/3 that inhibits cell migration and is associated with the prognosis of cancer.

EP 2 842 566 A1

## Description

The present invention relates to various uses of Arpin (therapy, diagnosis, drug screening, research), a new inhibitor of the Arp2/3 complex that inhibits cell migration and is associated with the prognosis of cancer.

Cell migration is a critical process for every type of living organism. Cells in the body often move from place to place to complete their functions. Cell migration is important in many processes such as wound repair, cell differentiation, immune response. Furthermore, aberrant cell migration causes metastasis, a process associated with malignant cancer.

Cell migration requires the generation of branched actin networks that power the protrusion of the plasma membrane in lamellipodia[1,2]. The Arp2/3 complex is the molecular machine that nucleates these branched actin networks[3]. This machine is activated at the leading edge of migrating cells by the WAVE complex. The WAVE complex is itself directly activated by the small GTPase Rac, which induces lamellipodia[4-6]. However, how cells regulate the directionality of migration is poorly understood. The Arp2/3 complex is activated at different cellular locations by different Nucleation Promoting Factors (NPFs), WAVE at lamellipodia, N-WASP at clathrin coated pits, and WASH at endosomes[7,8]. NPFs share a characteristic C-terminal tripartite domain, referred to as the VCA[9]. The A motif (for Acidic) binds to the Arp2/3 complex and induces its conformational activation. Arp2/3 inhibitory proteins containing an A motif, PICK1 and Gadkin, were detected at endocytic pits and at endosomes[10,11]. Thus, while endocytic pits and endosomes possess antagonistic activities toward the Arp2/3 complex, it is not known whether lamellipodia harbor a similar Arp2/3 inhibitory protein to counteract WAVE and inhibit cell migration.

The inventors have identified a novel protein that inhibits the Arp2/3 complex *in vitro,* Arpin, and shown that Rac signalling recruits and activates Arpin at the lamellipodial tip, like WAVE. Consistently, upon depletion of the inhibitory Arpin, lamellipodia protrude faster and cells migrate faster. A major role of this inhibitory circuit, however, is to control directional persistence of migration. Indeed, Arpin depletion in both mammalian cells and *Dictyostelium discoideum* amoeba resulted in straighter trajectories, whereas Arpin microinjection in fish keratocytes, one of the most persistent systems of cell migration, induced these cells to turn. The coexistence of the Rac-Arpin-Arp2/3 inhibitory circuit with the Rac-WAVE-Arp2/3 activatory circuit can account for this conserved role of Arpin in steering cell migration. Loss of this inhibitory circuit promotes exploratory behaviors and commits carcinoma cells to the invasive state.

Therefore, the invention provides a new protein inhibitor of the Arp2/3 complex which can be used as a medicament to inhibit cell migration, as a prognostic biomarker for cancer, as a target for screening drugs that inhibit or promote cell migration, and as a research tool to study cell migration.

A first aspect of the invention relates to a product as a medicament for inhibiting cell migration, said product being selected from the group consisting of:

a) an Arpin protein,
b) a peptide of at least 13 consecutive amino acids from said Arpin protein, which comprises at least the acidic motif (A motif) of said Arpin protein, and
c) a polynucleotide encoding the Arpin protein in a) or peptide in b) in expressible form, and

wherein said Arpin protein in a) and peptide in b) inhibit the Arp2/3 complex.

The Arpin protein for the different uses according the invention is denominated Arpin or Arpin protein, and the corresponding gene is denominated Arpin gene.

The Arpin protein has the advantage to be easy to produce in large amounts using standard recombinant DNA techniques and also easy to introduce into cells in effective amounts to inhibit the Arp2/3 complex and thereby inhibit cell migration. In addition, the Arpin protein is an inhibitor of the Arp2/3 complex that is specific for cell migration.

The Arpin protein refers to any protein from the Arpin family and functional variants derived from said Arpin family. The Arpin family includes human Arpin and its orthologs from other species.

In the following description, the standard one letter amino acid code is used.

The Arpin family corresponds to the Uncharacterized Protein Family UPF0552 in the databases. The protein of amino acid sequence SEQ ID NO: 1 (GenBank Accession number AAH53602 or UniProtKB/Swiss-Prot Q7Z6K5) is the product of human C15orf38 gene (Gene ID 348110; location 15q26.1; complement of positions 90443832 to 90456222 on human chromosome 15). In the present invention, the protein of SEQ ID NO: 1 is denominated human Arpin or human Arpin protein and the C15orf38 gene is denominated human Arpin gene. Arpin orthologs are found in multiple animal species, including those shown in Table II, figure 3 and sequences SEQ ID NO: 2 to 6. The Arpin protein has a conserved structure characterized by a C-terminal A motif (figures 3 and 4). The A motif consists of a sequence of about 16 amino acids (usually 13 to 17 amino acids), comprising a tryptophan residue (W) at the antepenultimate or penultimate position and at least seven aspartic acid (D) or glutamic acid (E) residues, as shown in Table I, figure 1a and 3, and the sequences SEQ ID NO: 7 to 11.

Functional variants include natural variants resulting from Arpin gene polymorphism as well as artificial variants. Functional variants are derived from wild-type amino acid sequences by the introduction of one or more mutations (deletion, insertion, and/or substitution) at specific amino acid positions. Functional variants are able to bind to the Arp2/3 and prevents its activation (Arp2/3 complex inhibitory activity), and thereby inhibit cell migration.

The invention uses a natural, recombinant or synthetic protein/peptide which is pharmacologically active. Pharmacologically active refers to the inhibitory activity of the protein/peptide on the Arp2/3 complex and on cell migration. As demonstrated in the examples of the present Application, the A motif is necessary and sufficient to obtain an inhibitor of the Arp2/3 complex (figures 5 and 9).

The properties of the protein/peptide can be readily verified by technique known to those skilled in the art such as those described in the examples of the present application.

The polynucleotide encoding the protein/peptide in expressible form refers to a nucleic acid molecule which, upon expression in a cell or a cell-free system results in a functional protein/peptide.

According to a preferred embodiment, said Arpin protein comprises an amino acid sequence (I) which is at least 70 % identical to residues 1 to 226 of human Arpin amino acid sequence SEQ ID NO: 1 and which comprises an acidic motif.

The percent amino acid sequence identity is defined as the percent of amino acid residues in a Compared Sequence that are identical to the Reference Sequence SEQ ID NO: 1 after aligning the sequences and introducing gaps if necessary, to achieve the maximum sequence identity. The Percent identity is then determined according to the following formula: Percent identity = 100 x [1- (C/R)], wherein C is the number of differences between the Reference Sequence SEQ ID NO: 1 and the Compared sequence over the entire length of SEQ ID NO: 1 (i.e., positions 1 to 57 of SEQ ID NO: 1), wherein (i) each amino acid in the Reference Sequence that does not have a corresponding aligned amino acid in the Compared Sequence, (ii) each gap in the Reference Sequence, and (iii) each aligned amino acid in the Reference Sequence that is different from an amino acid in the Compared Sequence constitutes a difference; and R is the number amino acids in the Reference Sequence over the length of the alignment with the Compared Sequence with any gap created in the Reference Sequence also being counted as an amino acid.

Alignment for purposes of determining percent amino acid sequence identity can be achieved in various ways known to a person of skill in the art, for instance using publicly available computer software such as BLAST (Altschul et al., J. Mol. Biol., 1990, 215, 403-). When using such software, the default parameters, e.g., for gap penalty and extension penalty, are preferably used. For amino acid sequences, the BLASTP program uses as default a word length (W) of 3 and an expectation (E) of 10.

For example, the alignment of mouse Arpin (SEQ ID NO: 2; 226 amino acids) with human Arpin (SEQ ID NO: 1) shows that over the length of alignment between the Reference Sequence and the Compared Sequence, i.e., the entire length of SEQ ID NO: 1 (positions 1 to 226 of SEQ ID NO: 1), there are no gap in the Reference Sequence, no gap in the Compared Sequence and 26 amino acid in the Reference Sequence which are different from the aligned amino acid sequence in the Compared Sequence. Therefore, C = 26 and R = 226. The percent identity = 100 x [1-(26/226)]. Mouse Arpin comprises an amino acid sequence which is 88 % identical to positions 1 to 226 of SEQ ID NO: 1.

The invention encompasses the use of an Arpin protein/peptide comprising or consisting of natural amino acids (20 gene-encoded amino acids in a Land/or D-configuration) linked via a peptide bond as well as peptidomimetics of such protein where the amino acid(s) and/or peptide bond(s) have been replaced by functional analogues. Such functional analogues include all known amino acids other than said 20 gene-encoded amino acids. A non-limitative list of non-coded amino acids is provided in Table 1A of US 2008/0234183 which is incorporated herein by reference. The invention also encompasses modified proteins/peptides derived from the above proteins/peptides by introduction of any modification into one or more amino acid residues, peptide bonds, N-and/or C-terminal ends of the protein/peptide, as long as the Arp2/3 inhibitory activity is maintained in the modified protein/peptide. These modifications which are introduced into the protein/peptide by the conventional methods known to those skilled in the art, include, in a non-limiting manner: the substitution of a natural amino acid with a non-proteinogenic amino acid (D amino acid or amino acid analog); the modification of the peptide bond, in particular with a bond of the retro or retro-inverso type or a bond different from the peptide bond; the cyclization, and the addition of a chemical group to the side chain or the end(s) of the protein:peptide, in particular for coupling an agent of interest to the protein of the invention. These modifications may be used to label the protein/peptide, and/or to increase its affinity for Arp2/3 and/or its bioavailability.

The Arpin protein comprises or consists advantageously of an amino acid sequence (I) which is at least 75 %, 80 %, 85 %, 90 % or 95 % identical to residues 1 to 226 of SEQ ID NO: 1. Preferably, the sequence (I) is at least 85 % identical to residues 1 to 226 of SEQ ID NO: 1.

The sequence (I) has advantageously up to 500 amino acids, more preferably about 250 amino acids, and is selected from the group consisting of SED ID NO: 1 and a sequence which differs from SEQ ID NO: 1 by dispersed deletions and/or insertions of one to five amino acids in said sequence, amino acid substitutions, and/or N-terminal deletion(s) of one or more amino acids in said sequence. The amino acid substitution(s) in SEQ ID NO:1 are advantageously chosen from conservative substitutions, i.e., substitutions of one amino acid with another which has similar chemical or physical properties (size, charge or polarity), which generally does not modify the functional properties of the protein. More preferably, said conservative substitution(s) are chosen within one of the following five groups: Group 1-small aliphatic, non-polar or slightly polar residues (A, S, T, P, G); Group 2-polar, negatively charged residues and their amides (D, N, E, Q); Group 3-polar, positively charged residues (H, R, K); Group 4-large aliphatic, nonpolar residues (M, L, I, V, C); and Group 5-large, aromatic residues (F, Y, W).

In another preferred embodiment, said Arpin protein is a mammal Arpin, preferably human Arpin.

According to another preferred embodiment, the A motif consists of the sequence (II):

$X_1X_2X_3X_4X_5X_6X_7X_8X_9X_{10}X_{11}X_{12}X_{13}X_{14}X_{15}X_{16}X_{17}$, in which:

$X_1$ represents E, K or is absent, $X_2$ represents I, P, S or is absent, $X_3$ represents G or is absent, $X_4$ represents R, A or Q, $X_5$ represents E, G, A or Q, $X_6$ represents E or Q, $X_7$ represents G, N or Q, $X_8$ represents D or E; $X_9$ represents G or E, $X_{10}$ represents A or
E, $X_{11}$ represent D, G or E, $X_{12}$ represents D, $X_{13}$ represents D or E, $X_{14}$ represents E,
$X_{15}$ represents W, $X_{16}$ represents D or K and $X_{17}$ represents D or is absent, with the proviso that at least seven of said $X_1$,$X_5$,$X_6$,$X_8$ to $X_{14}$,$X_{16}$ and $X_{17}$ residues are E or D.
Preferably, the A motif consists of any one of SEQ ID NO: 7 to 11. More preferably the A motif consists of SEQ ID NO: 7.

In another preferred embodiment, the Arpin peptide comprises at least 16 consecutive amino acids from said sequence (I) including at least the A motif. In another preferred embodiment, the Arpin peptide consists of said A motif.

Examples of preferred Arpin proteins/peptides are SEQ ID NO: 1 to 11. Examples of more preferred proteins/peptides are SEQ ID NO: 1, 2 and 7.

In another preferred embodiment, the protein is a fusion or chimeric protein, comprising a sequence (III) fused to the N-terminal end of the sequence (I) and, optionally, another sequence (IV) fused to the C-terminal end of said sequence (I). The length of the protein is not critical to the invention as long as the Arp2/3 inhibitory activity is maintained. The sequences (III) and (IV) comprise one or more other protein/peptide moieties including those which allow the purification, detection, immobilization, and/or cellular targeting of the protein of the invention, and/or which increase the affinity for Arp2/3, the bioavailability, the production in expression systems and/or stability of said protein. These moieties may be selected from: (i) a labeling moiety such as a fluorescent protein (GFP and its derivatives, BFP and YFP), (ii) a reporter moiety such as an enzyme tag (luciferase, alkaline phosphatase, glutathione-S-transferase (GST), β-galactosidase), (ii) a binding moiety such as an epitope tag (polyHis6, FLAG, HA, myc.), a DNA-binding domain, a hormone-binding domain, a poly-lysine tag for immobilization onto a support, (iii) a stabilization moiety, and (iv) a targeting moiety for addressing the chimeric protein to a specific cell type or cell compartment. In addition, the sequence(s) (III) and/or (IIV) advantageously comprise a linker which is long enough to avoid inhibiting interactions between sequence (I) and sequences (III) and/or (IV). The linker may also comprise a recognition site for a protease, for example, for removing affinity tags and stabilization moieties from the purified chimeric protein according to the present invention.

The polynucleotide encoding the protein/peptide in expressible form is synthetic or recombinant DNA, RNA or combination thereof, either single- and/or double-stranded. Preferably the polynucleotide comprises a coding sequence which is optimized for the host in which the protein/peptide is expressed.

In another preferred embodiment, the polynucleotide comprises or consists of SEQ ID NO: 12.

In another preferred embodiment, the polynucleotide is inserted in a vector. Preferably, said recombinant vector is an expression vector capable of expressing said polynucleotide when transfected or transformed into a host cell such as a prokaryotic or eukaryotic cell. The polynucleotide is inserted into the expression vector in proper orientation and correct reading frame for expression. Preferably, the polynucleotide is operably linked to at least one transcriptional regulatory sequence and, optionally to at least one translational regulatory sequence. Recombinant vectors include usual vectors used in genetic engineering and gene therapy including for example plasmids and viral vectors.

Another aspect of the present invention relates to a product as defined above for use in treating a disease caused by aberrant cell migration. Preferably, said disease is cancer or a disease caused by aberrant cell migration of cells from the innate or adaptive immune system. Breast cancer is a non-limitative example of cancer. Chronic inflammatory diseases are non-limitative examples of diseases caused by aberrant cell migration of immune cells.

Another aspect of the invention is an inhibitor of a mammal Arpin protein, preferably human Arpin, as a promoter of cell migration, for use in treating injuries.

According to the invention said inhibitor decreases the activity or expression of said Arpin protein. Inhibitors of protein activity or expression are known in the art; any of such inhibitors can be adapted to the Arpin protein. Inhibitors of Arpin protein activity include small molecules and antibodies targeting the Arpin protein, in particular the A motif of said protein. Inhibitors of Arpin protein expression include oligonucleotides targeting the Arpin mRNAs such as for example, antisense oligonucleotides including morpholinos (phosphorodiamidate morpholino oligomers or PMOs), siRNAs, shRNAs and miRNAs. Preferably, said inhibitor is an oligonucleotide, more preferably a siRNA or a shRNA, targeting any one of the sequences SEQ ID NO: 13 to 17 from Arpin mRNA or a morpholino of SEQ ID NO: 19.

According to the invention, the protein/peptide, polynucleotide and/or vector, inhibitor, may be included in a pharmaceutical composition, further comprising a pharmaceutically acceptable carrier.

The pharmaceutical composition is formulated for administration by a number of routes, including but not limited to oral, parenteral and local. The pharmaceutically acceptable carriers are those conventionally used.

The pharmaceutical composition comprises a therapeutically effective amount of the protein/peptide/polynucleotide/vector/inhibitor, e.g., sufficient to show benefit to the indidual to whom it is administered. The pharmaceutically effective dose depends upon the composition used, the route of administration, the type of mammal (human or animal) being treated, the physical characteristics of the specific mammal under consideration, concurrent medication, and other factors, that those skilled in the medical arts will recognize.

The invention provides also a method for treating a patient having a disease caused by aberrant cell migration, comprising: administering a therapeutically effective amount of the protein/peptide, polynucleotide and/or vector to the patient.

The invention provides also a method for treating a patient having an injury, comprising: administering a therapeutically effective amount of the inhibitor to the patient.

Another aspect of the invention relates to a method *in vitro* for evaluating the prognosis of a cancer in a patient, comprising:

a) determining the level of an expression product of the Arpin gene in a biological sample from said patient, and
b) comparing the level in a) with a reference level for said expression product, wherein if the level in a) is lower than said reference level, then said patient suffers from an invasive cancer with an unfavorable prognosis.

Compared to other methods for the prognosis of cancer, the method of the present invention has the advantage of using a biomarker whose function is known (inhibition of cell migration) and associated with metastasis formation. Thus, under-expression of Arpin is associated with the formation of metastasis because cell migration is necessary for metastasis formation. In addition, the method of the invention requires one biomarker only to evaluate the prognosis of cancer.

"An expression product of the Arpin gene" refers to Arpin mRNA or protein.

"Biological sample" refers to a biological material likely to contain an expression product of the Arpin gene. The biological material which may be derived from any biological source is removed from the cancer patient by standard methods which are well-known to a person having ordinary skill in the art.

"Lower level" refers to a significant lower level, *i.e.,* p-value inferior to 0.1.

"Reference value" refers to a value established by statistical analysis of values obtained from a representative panel of individuals. The panel may for example depend from the nature of the sample, the type of cancer. The reference value can for example be obtained by measuring Arpin mRNA or protein expression level in a panel of normal individuals and/or individuals having a non-invasive cancer and determining a threshold value, for example the median concentration, which is used as reference value. When the method according to the invention aims at monitoring a patient, the reference value may be obtained from the patient previously tested.

In a preferred embodiment of the above method, said cancer is a carcinoma. A non-limitative example of carcinoma is breast cancer.

In another preferred embodiment of the above method, said patient is a human individual. In particular, said patient is a newly diagnosed individual.

Early evaluation of the prognosis of the cancer in the initial tumor of a patient using the method of the invention allows the choice of the most efficient therapy for the patient: local radiotherapy for a non-invasive tumor or systemic chemotherapy for an invasive tumor.

The biological sample is advantageously biopsied tumor cells or tissue, or a body fluid such as serum, plasma, blood, lymph, synovial, pleural, peritoneal, or cerebrospinal fluid, mucus, bile, urine saliva, tears and sweat.

In another more preferred embodiment of the above identified method, said biological sample is biopsied tumor cells or tissue.

Arpin gene product expression level may be assayed directly on the biological sample or following a standard pretreatment, according to pretreatment methods which are well-known to a person having ordinary skill in the art. Pretreatment may include for example lysing cells, extracting and precipitating RNA, and embedding biopsied tissue in plastic or paraffin.

Arpin gene product expression level can be measured using a variety of techniques for detecting and quantifying the expression of a gene, that are well-known to a person having ordinary skill in the art. Such techniques typically include methods based on the determination of the level of transcription (*i.e.,* the amount of mRNA produced) and methods based on the quantification of the protein encoded by the Arpin gene.

In another preferred embodiment of the above identified method, it comprises measuring human Arpin messenger RNA (mRNA) level in said biological sample, preferably biopsied tumor cells or tissue.

Arpin mRNA level may be measured, either by hybridization to a specific probe, eventually labeled with a detectable label and/or immobilized on the surface of a solid support (plate, slide, strip, wells, microparticles, fiber, gel), or by amplification using specific primers, eventually labeled with a detectable label. Preferably, the Arpin mRNA level is measured using an assay selected from the group consisting of: nucleic acid array- or tissue microarray-based assay, and quantitative reverse transcription polymerase chain reaction (qRT-PCR) assay. One skilled in the art will know which parameters may need to be manipulated to optimize detection and/or quantification of the Arpin mRNA using these techniques

In another preferred embodiment of the above identified method, it comprises measuring human Arpin protein level in said biological sample, preferably biopsied tumor cells or tissue.

Measurement of Arpin protein level may be achieved using several different techniques, many of which are antibody-based. Example of such techniques include with no limitations immunoassays, immunohistochemistry assays and antibody microarray-based assays. Preferably, Arpin protein level is measured using an immunohistochemistry assay. Arpin antibodies are prepared using conventional techniques, and various monoclonal and polyclonal antibodies can be obtained using these methods as shown in the examples of the present application. One skilled in the art will know which parameters may need to be manipulated to optimize detection and/or quantification of the Arpin protein with Arpin antibodies, using these techniques.

The reference value is advantageously obtained from the same type of biological sample and/or from a panel of patients with the same type of cancer, as the tested patient.

The method according to the present invention may be performed simultaneously or subsequently on biological samples from different patients.

The above mentioned method may further comprise, after the comparing step, a further step of sorting the cancer patient(s) into favorable and unfavorable prognosis based on Arpin level(s) in said biological sample(s).

Expression levels of other cancer biomarkers which are not biomarkers of cancer prognosis can be measured, in parallel for other purposes. Another aspect of the invention is a method for screening an inhibitor of cell migration, comprising:

- contacting at least one test molecule with a cell in which Arpin gene expression is inhibited, and
- identifying the molecules capable of increasing the level of expression of said Arpin gene in said cell, compared to a reference level of expression for said Arpin gene.

The screening of cell migration inhibitors is performed using standard assays for measuring gene expression at the mRNA or protein level which are well-known in the art such as those disclosed in the examples of the present application.

Another aspect of the invention is a method for screening a promoter of cell migration, comprising:

- contacting at least one test molecule with a cell expressing an Arpin protein, and
- identifying the molecules capable of inhibiting said Arpin protein.
  The molecule may inhibit the Arpin protein activity or expression. Preferably, the molecules which are selected are capable of blocking the inhibitory effect of Arpin on the Arp2/3 complex, for example by inhibiting the binding of Arpin to the Arp2/3 complex.

The screening of cell migration promoters is performed using standard assays such as those disclosed in the examples of the present application.

Another aspect of the invention is the use of an Arpin protein, a polynucleotide encoding said protein in expressible form, to study cell migration. The Arpin protein for research uses may be a mammal or a non-mammal Arpin, depending upon the cell system which is used to study cell migration.

The polynucleotide for use according to the invention is prepared by the conventional methods known in the art. For example, it is produced by amplification of a nucleic sequence by PCR or RT-PCR, by screening genomic DNA libraries by hybridization with a homologous probe, or else by total or partial chemical synthesis. The recombinant vectors are constructed and introduced into host cells by the conventional recombinant DNA and genetic engineering techniques, which are known in the art.

The protein/peptide for use according to the invention is prepared by the conventional techniques known to those skilled in the art, in particular by expression of a recombinant DNA in a suitable cell system (eukaryotic or prokaryotic)or by solid-phase or liquid-phase synthesis. More specifically, the protein and its derivatives are usually produced from the corresponding cDNA, obtained by any means known to those skilled in the art; the cDNA is cloned into a eukaryotic or prokaryotic expression vector and the protein produced in the cells modified with the recombinant vector is purified by any suitable means, in particular by affinity chromatography. The peptide and its derivatives are usually solid-phase synthesized, according to the Fmoc technique, originally described by Merrifield et al. (J. Am. Chem. Soc.,

1964, 85: 2149-) and purified by reverse-phase high performance liquid chromatography.

The practice of the present invention will employ, unless otherwise indicated, conventional techniques which are within the skill of the art. Such techniques are explained fully in the literature.

In addition to the above arrangements, the invention also comprises other arrangements, which will emerge from the description which follows, which refers to exemplary embodiments of the subject of the present invention, with reference to the attached drawings in which:

**Figure 1: Arpin inhibits Arp2/3 activation *in vitro.* a,** Alignment of acidic C-termini of 3 NPFs and of Arpin. The antepenultimate tryptophane is highlighted in light grey and acidic residues (D and E) are in grey. **b**, Arpin binds to the Arp2/3 complex through its acidic C-terminal region. Pulldown was performed with HeLa cell extract and GST fusions of Arpin full length (FL), Arpin deleted of its last 16 amino-acids (ΔA), the last 16 amino-acids (A) or the VCA domain of N-WASP as a positive control. Arp2/3 binding was assessed by western blot against the ArpC2 subunit. **c**, Spectrofluorimetry assay to monitor actin polymerisation. Conditions: 2 μM actin (10 % pyrene-labelled), 500 nM VCA, 20 nM Arp2/3 and Arpin full length or ΔA at the indicated concentrations (0, 400, 800, 1600 nM; 3, 7, 10 and 17 μM). **d**, Assembly of branched actin networks monitored by TIRF microscopy. Conditions: 1 μM actin (10 % rhodamine-labelled), 150 nM VCA, 80 nM Arp2/3 and Arpin at 5 μM when indicated. Scale bar: 5 μm. **e**, Arpin competes with the NPF for Arp2/3 binding. Pulldown was performed with HeLa cell extract and 5 μM GST N-WASP VCA immobilized on glutathione beads. Arp2/3 is displaced from its interaction with the VCA by full length Arpin, but not by ArpinΔA (18 μM and serial 2-fold dilutions).

**Figure 2: Arpin is ubiquitously expressed.** Lysates were prepared from the indicated murine tissues. Extract quality and equal quantity was verified by Coomassie blue staining. Arpin expression was detected by western blot.

**Figure 3: Conservation of Arpin and prediction of secondary structure elements and disordered regions.** A multiple alignment of the Arpin orthologs indicated in Table II was performed with MUSCLE[27] and displayed with Jalview[28]. Two methods relying on multiple alignments of Arpin orthologs were used to predict secondary structures and disordered regions, Psipred[29] and Disopred[30] respectively. The predicted secondary structure elements (SS) are indicated by arrows for β-strands, cylinders for α-helices and a line for coils; the associated confidence score (SS Conf) is displayed below, ranging from 0 to 9 for poor and high confidence, respectively. The confidence in predicting disorder is also scored from 0 to 10, by multiplying tenfold the Disopred probability. Amino acid conservation is indicated by a 0 to 10 score histogram bars.

**Figure 4: Nuclear Magnetic Resonance analysis of $^{15}$N labelled human Arpin.** Both views represent $^{1}$H-$^{15}$N HSQC spectra. Each peak corresponds to the $^{1}$H-$^{15}$N backbone amide bond of a specific residue. The position of a $^{1}$H-$^{15}$N peak in the spectrum depends on the chemical environment of the corresponding residue. **a**, Such a scattered distribution of peaks is characteristic of a folded protein. The last 20 residues were assigned to individual peaks and are displayed on the spectrum. These residues are clustered in the centre of the spectrum. **b**, Same HSQC spectrum displayed with a higher threshold to display only high peaks. The height of a peak depends on the mobility of the residue on a picosecond to millisecond timescale. This spectrum experimentally demonstrates that the 20 C-terminal residues are highly mobile. This result confirms that, as predicted, the Arp2/3 binding site of Arpin is exposed as a poorly structured tail of the protein.

**Figure 5: Arpin directly binds to the Arp2/3 complex with a dissociation constant of 200 nM. a,** Fluorescence anisotropy measurements of labelled ArpinA peptide binding at equilibrium to the purified Arp2/3 complex at the indicated concentrations. **b**, Labelled ArpinA peptide bound to the Arp2/3 complex was then titrated with purified Arpin FL, ArpinΔA or unlabelled ArpinA peptide as indicated. Arpin FL displaces the labelled ArpinA peptide more efficiently than the A peptide. ArpinΔA is unable to displace the ArpinA peptide. Curves that best fit the values yield the indicated equilibrium constants.

**Figure 6: Characterisation of recombinant Arpin.** Full length Arpin or ArpinΔA from human or zebrafish cDNA was expressed in *E.coli* and purified (see methods). **a**, Purity of the recombinant proteins was assessed by SDS-PAGE and coomassie staining. These human and zebrafish proteins were used for *in vitro* actin polymerisation assays and for fish keratocyte injection, respectively. **b**, Analysis of the molar mass of full length Arpins by Size Exclusion Chromatography coupled to Multi Angle Light Scattering (SEC-MALS). The UV measurement (left axis, dashed line) and the molar mass (right axis, horizontal solid line) were plotted as a function of column elution volume. **c**, SEC-MALS measures of masses indicate that both proteins are monomeric in solution.

**Figure 7: Arpin does not affect spontaneous actin nucleation and does not activate the Arp2/3 complex.** Conditions: 2 μM Actin (10 % pyrene-labelled), 20 nM Arp2/3, 500 nM Scar VCA, 5 μM Arpin.

**Figure 8: Determination of apparent Kd from Arpin inhibition of Arp2/3 nucleation. a**, Arpin inhibits Arp2/3 activation in the pyrene-actin assay. Part of this experiment is displayed in Fig.1c, more curves are plotted here (0, 100, 200, 400, 800 nM; 1.6, 2, 3, 5, 7, 9, 10 and 17 μM). **b**, The number of actin barbed ends is calculated from the slope at half-polymerisation using the relationship described in reference 31. Best fit of the values indicate an apparent Kd of 760 ± 156 nM for the Arp2/3 complex in a mixture including actin and the VCA.

**Figure 9: The ArpinA peptide is a moderate competitive inhibitor of the Arp2/3 complex. a**, ArpinA inhibits Arp2/3 activation in a dose dependent manner in the pyrene-actin assay. Conditions: 2 μM actin (10 % pyrene-labelled), 500 nM VCA, 20 nM Arp2/3 and ArpinA at the indicated concentrations (0, 1, 4, 10, 15, 30 and 60 μM). **b**, ArpinA competes with the NPF for Arp2/3 binding. Arp2/3 is displaced from its interaction with 5 μM GST N-WASP VCA immobilized on glutathione beads by the Arpin Acidic peptide (304 μM and serial 2-fold dilutions).

**Figure 10: Quantification of Arpin inhibition on the generation of branched actin networks.** Conditions as in Fig. 1d. Images were extracted from the TIRF movies, 5, 10, 15 and 20 min after initiation of the reaction. The number of branches and filaments were counted and the ratio determined. Arpin decreases the frequency of branches in actin networks.

**Figure 11: Arpin inhibits the Arp2/3 complex at the lamellipodium tip. a**, Arpin colocalises with Brk1, a subunit of the WAVE complex, at the lamellipodium tip of spreading MEF cells. Averaging intensity profiles along multiple line scans spanning lamellipodial outline revealed a perfect overlap in the distribution of both machineries (Mean ± sem, n = 24 line scans). Scale bar: 20 μm. **b**, Active Rac induces the association of Arpin with the Arp2/3 complex. 293T cells were co-transfected with GFP-Arpin and different forms of PC-tagged Rac, the dominant negative form T17N (TN), the wild type form (WT) and the constitutively active form Q61L (QL). Input lysates and GFP immunoprecipitates were western blotted with Arpin, ArpC2 or PC antibodies. PC-tagged Rac retains normal down-regulation of the endogenous protein, which is degraded by the ubiquitin proteasome pathway upon activation. **c**, Rac is required for the Arpin-Arp2/3 interaction. Input lysates and Arpin immunoprecipitates from Rac knock-out MEFs (KO) or floxed controls (WT) were western blotted with the indicated antibodies. **d**, Arpin depletion increases the speed at which lamellipodia protrude. RPE1 cells were transiently transfected with plasmids encoding a shRNA targeting Arpin and GFP fusion proteins as indicated. Spreading RPE1 cells were recorded using time lapse phase contrast imaging, and kymographs of lamellipodia were plotted. Arpin re-expression rescues the phenotype when full length, but not when the Arp2/3 interacting Acidic domain is removed. Mean ± sem; n = 40; * $P < 0.001$, ANOVA. e, Arpin circuitry. Rac activates the Arp2/3 complex through the WAVE complex and inhibits it through Arpin in an 'incoherent feedforward loop'.

**Figure 12: Arpin distribution relative to the Arp2/3 complex and cortactin.** Intensity profiles along multiple line scans encompassing the cell periphery were registered to the outer edge of the staining of a lamellipodial marker. This marker was Arpin in a and Cortactin in b. The multiple line scans were then averaged (Mean ± sem, $n \geq 16$). Scale bar : 20 μm.

**Figure 13: Confocal examination of Arpin staining.** Localisation of Arpin and Cortactin was examined by immunofluorescence in MEF cells. Acquisition of a single plane at the ventral surface using a confocal microscope confirms that Arpin indeed localises to the lamellipodium tip and not to a peripheral ruffle. Scale bar: 20 μm.

**Figure 14 : Lack of peripheral localisation of Arpin in Rac1 KO MEFs.**Rac1 KO MEFs that lack lamellipodia are completely devoid of Arpin staining at the cell periphery, in line with the complete absence of lamellipodia indicated here by the absence of cortactin staining. Arpin is normally expressed in the Rac1 KO MEFs (see Fig. 11c). Intensity profiles along multiple line scans encompassing the cell periphery were averaged after manual drawing of the cell edge. Scale bar : 20 μm.

**Figure 15: Rac does not directly bind to Arpin and does not regulate Arpin activity *in vitro.* a**, GST pull-down using a lysate of 293 cells overexpressing PC tagged Arpin and purified GST-Rac1 WT, GST-Rac1Q61L or GST alone as a negative control. Arpin did not associate with either type of Rac. In contrast, the endogenous WAVE complex bound to RacQ61L, but not Rac WT, as expected from a Rac effector. **b,** Untagged Rac1 was purified from *E.coli* and its purity analysed by SDS-PAGE and coomassie blue staining. Rac1 was then loaded with either GDP or GTPγS. Human Arpin (60 μM), Rac1 (120 μM) and mixture of these two proteins were analyzed by Size Exclusion Chromatography coupled to Multi Angle Light Scattering (SEC-MALS). The UV measurement (left axis, dashed line) and the molar mass (right axis, horizontal solid line) were plotted as a function of column elution volume. SEC was run in (20 mM Hepes, 100 mM NaCl, pH 7.4). The height of UV peaks was normalized to 1 in order to be displayed on the same figure. A single peak was detected in all cases. The measured masses indicate that no complex are formed between Arpin and Rac and that the single peak observed in the mixture corresponds to cofractionation of the two proteins of similar mass by SEC. c, Rac does not affect Arpin inhibitory activity on Arp2/3 mediated actin nucleation. Conditions: 2 μM Actin (10 % pyrene-labelled), 20 nM Arp2/3, 500 nM Scar VCA, 4 μM Arpin and the indicated concentrations of untagged Rac1 Q61L (0, 4 or 8 μM).

**Figure 16: Arpin regulates cell spreading through its interaction with the Arp2/3 complex.** Arpin was depleted from human RPE1 cells after transient transfection of shRNA plasmids and blasticidin-mediated selection of transfected cells. After 5 days, cells were either analysed by western blot or used for the spreading assay. Cells were serum-starved for 90 min in suspension in polyHEMA-coated dishes and then allowed to spread on collagen 1-coated coverslips for 2 hours. Phalloidin staining was used to calculate cell surface area of individual cells using ImageJ. Mean ± sem, n > 47 cells; * $P < 0.01$, *** $P < 0.001$; t-test or ANOVA when more than two conditions. **a**, Arpin depletion increases cell spreading. The same effect is obtained with 3 shRNAs targeting Arpin. **b**, This effect is rescued by GFP-Arpin expression in knock-down cells, but not by GFP-ArpinΔA expression. **c,** Combined depletion of Arpin and the Arp2/3 complex reverses the phenotype of Arpin depletion. The effect is seen with two shRNAs targeting ArpC2. The last two experiments indicate that Arpin exerts its effect on cell spreading through its ability to regulate the Arp2/3 complex.

**Figure 17: Arpin regulates protrusion frequency of prechordal plate cells and their collective migration in zebrafish embryos. a,** *In situ* hybridisation of *arpin* probe in zebrafish embryos at different stages. *arpin* mRNAs are maternally deposited. During gastrulation, *arpin* is expressed in hypoblast, which includes the prechordal plate. **b,** 3D trajectories of prechordal plate cells in embryos injected with control or *arpin* morpholino. During fish gastrulation, prechordal plate cells migrate collectively in a straight direction from the margin of the embryo towards the animal pole[32,33]. Loss of *arpin* function induces dispersion as evidenced by increased lateral cell displacement and a higher distance between cells. Lateral displacement is the cell movement perpendicular to main direction of the migration. Distance between cells refers to the average distance of the nucleus of a given cell to the nuclei of its 5 closest neighbors. Mean ± sem; $n \geq 1469$; *** $P < 0.001$, t-test. c, At the onset of gastrulation prechordal plate cells derived from morpholino injected embryos were transplanted into the prechordal plate of an untreated host embryo at the same stage in order to allow imaging of cell autonomous effects on protrusion formation. Donor embryos are injected with control or *arpin* morpholinos and mRNAs encoding Lifeact-mCherry as well as GFP-Arpin for the rescue. Time lapse imaging of injected cells is performed by epifluorescence to reveal Lifeact a marker of filamentous actin, which stains actin-based protrusions. For each cell, presence of a protrusion was assessed at each frame to deduce probability of protrusion presence and protrusion lifetimes. *arpin* loss of function increases the probability of presence of protrusions ($n \geq 8$, * $P < 0.05$, ANOVA) and their duration (in this case, n corresponds to the number of protrusions, $n \geq 40$, * $P < 0.05$, Kruskal-Wallis). Protrusions are indicated by arrowheads. Bar: 50 μm.

**Figure 18: Arpin depletion increases directional persistence of migration in mammalian cells and in the amoeba *Dictyostelium discoideum.* a,** Total extracts of stable clones of MDA-MB-231 cells depleted of Arpin or not were analysed by western blot. **b,** Arpin depleted MDA-MB-231 cells explore a wider territory than controls. Single cell trajectories of random migrating cells are plotted. **c,** Cell speed and directional persistence are increased in Arpin depleted cells. Rescue corresponds to the re-expression of GFP-Arpin in depleted cells. Mean ± sem; $n \geq 40$, * $P < 0.05$, Kruskal-Wallis. **d,** Single cell trajectories of random migrating amoeba are plotted. Arpin knock-out (KO) amoeba explores a wider territory than wild type (WT). Re-expression of *Dictyostelium* Arpin in KO amoeba reduces the migration relative to KO amoeba and to WT amoeba ($n \geq 45$; mean ± sem). **e,** Cell speed and directionality are both increased in Arpin KO amoeba relative to WT. Directionality is more than fully rescued by *Dictyostelium* Arpin expression. Directional persistence is thus the most important parameter controlled by Arpin. Mean ± sem; $n \geq 40$, * $P < 0.05$, Kruskal-Wallis.

**Figure 19: Arpin depletion increases cell migration in 3D.** Stable MDA-MB-231 clones depleted of Arpin or not were embedded in collagen gel. **a,** Single cell trajectories illustrate that control cells hardly move in this dense environment, unlike Arpin depleted cells which explore a significant territory, albeit at lower pace than in 2D, as evidenced by Mean Square Displacement (Fig. 20). **b,** Cell speed is significantly increased in the Arpin depleted clones. Mean ± sem; $n \geq 17$, * $P < 0.05$, Kruskal-Wallis. Directional persistence, calculated by d/D, is not significantly different in the clones depleted of Arpin or not. Direction autocorrelation (Fig. 21) shows however a complete lack of directionality at the earliest time points in the controls in 3D, but not in the Arpin depleted cells.

**Figure 20: Analysis of Mean Square Displacement (MSD) of the different migration experiments.** The Mean Square Displacement gives a measure of the area explored by cells for any given time interval. By setting a positional vector on the cellular trajectory at time t, the MSD is defined as: $MSD(t) = \langle [x(t + t_0) - x(t_0)]^2 + [y(t + t_0) - y(t_0)]^2 \rangle_{t, N}$, where brackets ⟨ ⟩ indicate averages over all starting times $t_0$ and all cells *N.* For each time interval ΔTime, mean and sem are plotted. Error bars corresponding to sem were always plotted, even if they are too small to be visible on some graphs. The grey area excludes the noisy part of curves corresponding to large time intervals where less data points are available. **a,** MDA-MB-231 depleted or not of Arpin in a two dimensional or three dimensional environment. Arpin depleted MDA-MB-231 cells explore a larger territory than the controls in time intervals examined (for 2D, $n \geq 40$, $P < 0.001$, two-way ANOVA with time and conditions; for 3D, $n \geq 17$, $P < 0.001$, two-way ANOVA with time and conditions). **b,** *Dictyostelium discoideum* KO amoebae explore a larger territory than controls and rescued amoebae ($n \geq 45$, $P < 0.001$, two-way ANOVA with time and conditions). **c,** Arpin injected fish keratocytes explore a smaller territory than the controls ($n \geq 8$, $P < 0.001$, two-way ANOVA with time and conditions).

**Figure 21: Analysis of direction autocorrelation of the different migration experiments. a,** Principle of the analysis. A hypothetical cell trajectory is depicted. Each step is represented by a vector of normalised length. θ is the angle between compared vectors. The plot illustrates the cosθ values for the putative trajectory of 4 steps. Averaging these cosθ values yields the Direction Autocorrelation (DA) function of time. This DA function measures the extent to which these vectors are aligned over time.The DA function is defined as:

$$DA(t) = <\square\upsilon(t_0) \bullet \upsilon(t_0+t)>_{t0,N} = <\cos\theta(t_0, t_0+t)>_{t0,N}$$

where $\upsilon(t_0)$ is the vector at the starting time to and $\upsilon(t_0+t)$ the vector at $t_0$+t. Brackets indicate that all calculated cosines are averaged over all possible starting times (to) and all cells (N). For each time interval t, vectors from all cell trajectories were used to compute average and sem. Error bars corresponding to sem were always plotted, even if they are too small to be visible on some graphs.**b,** Arpin depleted MDA-MB-231 clones turn less than control cells (for 2D, n ≥ 40, P < 0.05 between 10 and 40 min, Kruskal-Wallis; for 3D, n ≥ 17, P < 0.05 at time 10 min, Kruskal-Wallis). **c,** Arpin KO amoebae turn less than wild type amoebae and GFP-Arpin overexpressing KO amoebae (Rescue) turn more than wild type (n ≥ 45, P < 0.05 between 5 and 85 s, Kruskal-Wallis). **d,** Arpin injected fish keratocytes turn more than wild type and ArpinΔA injected keratocytes turn more than wild type but less than wild type Arpin injected keratocytes (n ≥ 8, P < 0.05 between 16 and 272 s, Kruskal-Wallis).

**Figure 22 : Generation of an Arpin knock-out in *D. discoideum.* a.** Schematic representation of the Arpin gene b. Construction of the targeting vector and generation of the knock-out mutant by recombination in the Arpin gene **c.** Recombination was assessed using diagnostic PCRs that distinguish KO from WT amoeba as indicated.

**Figure 23: Quantification of pseudopod dynamics in *Dictyostelium.*** Kymograph analysis was performed on movies of migrating *Dictyostelium* cells acquired with phase contrast optics using a 40x objective (1 frame every second for 15 min). Velocity, length and persistence of protrusion events were plotted. Mean ± sem, n ≥ 24, * P < 0.05, ANOVA after square root transformation. A statistically significant increase was seen only for pseudopod protrusion velocity of Arpin knock-out amoeba.

**Figure 24: Arpin microinjection induces fish keratocyte to turn. a,** Trout keratocytes were micro-injected with purified full length Arpin, ArpinΔA or buffer as control. Upon micro-injection of Arpin, lamellipodia were modified and the cells turned. Scale bar, 20 μm. **b,** Quantifications of the indicated parameters. Mean ± sem; n ≥ 8, * P < 0.05, *** P < 0.001, ANOVA. c, Kymograph of the Arpin microinjected keratocyte. The leading edge undergoes cycles of protrusions and retractions. **d,** Model. Directional persistence of migration in this cell system requires a positive feedback loop. Branched actin is sensed and activates Rac as a response. In this context, WAVE closes a positive feedback loop. In contrast, Arpin closes a negative feedback loop. These two nested positive and negative feedback loops can account for the oscillatory behavior of the leading edge observed upon Arpin micro-injection.

**Figure 25: Procedures to quantify fish keratocyte migration. a,** Kymograph analysis. Lamellipodial dynamics were studied along a one-pixel-wide line oriented in the direction of protrusions, using the MultiKymograph plug-in in ImageJ. Protrusion velocity was the average of all protrusion slopes at the front of the cell. Projections along the distance axis and the time axis, gave, respectively, length and duration of the protrusion. Cell speed was determined using the slope of the dashed line indicating the rear of the cell. **b,** Outline analysis. Cell contours were defined using CellTrack software (http://db.cse.ohio-state.edu/CellTrack) and manually adjusted when necessary. The cell from frame t is overlayed with the same cell in frame t+100 s, using their center of mass as a reference. The non-overlapping areas are summed up to give the cell deformation between these two frames. The angular deviation between three frames separated by 100 s is the angle made by the lines passing through two consecutive centers of mass, as indicated.

**Figure 26: Electroporation of various cells with purified Arpin.** A. MDA-MB-231. B. RPE-I and MEF cells. Western blot analysis was performed 1 hour after electroporation. The results show that Arpin can be successfully introduced into all these lines in a dose-dependent manner by electroporation.

**Figure 27: Electroporation of MDA-MB-231 cells with purified Arpin; analysis of cell migration.** While depletion of Arpin results in less idling (more active movement), electroporation of Arpin protein results in more idling (less active movement).

**Figure 28 : Arpin mRNA under-expression is associated with poor prognosis in breast cancer.** Metastasis-free survival (MFS) of patients was determined as the interval between initial diagnosis and detection of the first metastasis. Survival distributions in the different groups of gene expression were plotted using the Kaplan-Meier method, and the significance of the difference was ascertained with the log-rank test. Whereas Gadkin expression does not significantly correlate with MFS (P = 0.94), patients harbouring tumours with reduced Arpin mRNA expression have a significant poorer prognosis compared to the others (P = 0.022): 51.6 % MFS *vs* 75.5 % at 5 years (60 months) and 44.2 % *vs* 64.9 % at 10 years (120 months).

**Figure 29: Expression of the Arpin protein is reduced in breast carcinomas. a,** Normal alveolar ducts are composed of two epithelial layers. The inner luminal layer expresses keratin 8, whereas the surrounding basal myoepithelial layer expresses keratin 17. Arpin is highly expressed in both epithelial cell types compared to fibroblasts in the conjunctive tissue. Breast carcinomas *in situ* display proliferation of luminal epithelial cells, where Arpin expression is reduced. Invasive carcinomas display cells having lost all epithelial characteristics, even though they still express keratin 8. These invasive cells displayed strongly suppressed Arpin expression, even more than in situ carcinoma cells. Scale bars: 50 μm. **b**, Quantifications of Arpin fluorescence intensity in 14 patients suspected to develop breast cancer. 4 patients displayed only benign proliferation, whereas 10 patients had breast carcinoma. All examined carcinomas were estrogen and progesterone receptor positive and HER2 negative, the most frequent type. Arpin expression is not reduced when proliferation is benign. Arpin is significantly reduced in *in situ* carcinomas, and reduced to levels close to background in invasive carcinomas. *** $P < 0.001$, * $P < 0.05$, ANOVA on log10 transformed values.

**<u>Example 1:</u> Materials and methods**

### 1. Plasmids

Human and zebrafish Arpin were amplified by PCR from clones IMAGE:5770387 and IMAGE:7404342, respectively (GENESERVICE). *Dictyostelium discoideum* DdArpin was amplified from Ax2 cDNA. Human full length Arpin (residues 1-226), ArpinΔA (residues 1-210) or ArpinA (residues 211-226), zebrafish full length Arpin (residues 1-226), ArpinΔA (residues 1-210), murine N-WASP VCA fragment (residues 392-501)[34] were cloned into a modified pGEX vector with a TEV cleavage site between the restriction sites *Fse*I and *Asc*I. For expression in mammalian cells, Arpin inserts were cloned into a compatible plasmid pcDNAm PC-GFP blue[7]. Zebrafish full length Arpin was also inserted pBluescript to generate probes for *in situ* hybridisation and in pCS2-GFP for rescue experiments. Human Rac1 WT, T17N, Q61L, ArpC5A, and ArpC5B[35] were cloned into pcDNA5 His PC TEV blue[7]. For expression in amoeba, *Dictyostelium* Arpin was inserted into pDGFP-MCS-neo[36]. For shRNA expressing plasmids, two hybridised oligonucleotides (MWG) were cloned into psiRNA-h7SKblasti G1 (Invivogen) according to the manufacturer's protocol. Target sequences were the following:

- shArpin#1: GGAGAACTGATCGATGTATCT (SEQ ID NO: 13)
- shArpin#2: GCTTCCTCATGTCGTCCTACA (SEQ ID NO: 14)
- shArpin#3: GCCTTCCTAGACATTACATGA (SEQ ID NO: 15; targets the 3' UTR region of Arpin mRNA)
- shArpC2#1: CCATGTATGTTGAGTCTAA (SEQ ID NO: 16)
- shArpC2#2: GCTCTAAGGCCTATATTCA (SEQ ID NO: 17)

These plasmids were compared to the non-targeting control provided by Invivogen shControl: GCATATGTGCGTACCTAGCAT (SEQ ID NO: 18)

All constructs were verified by sequencing.

### 2. Protein purification

Arpin, ArpinΔA, ArpinA, N-WASP VCA fused to GST were purified from *E. coli* BL21* strain (LIFE TECHNOLOGIES) using standard purification protocols, dialysed against storage buffer (20 mM Tris-HCl, 50 mM NaCl, 1 mM DTT, pH 7.5), frozen in liquid nitrogen and stored at -80°C. When indicated, Arpin was cleaved by TEV protease off GST. Arpin bound to Glutathione sepharose 4B beads was cleaved by overnight incubation at 4°C using His-tagged TEV protease in 50 mM Tris pH 7.5, 2 mM β-mercaptoethanol, 100 mM NaCl, 5 mM $MgCl_2$. TEV was removed by incubation with $Ni^{2+}$ beads (GE HEALTHCARE). Arpin was further purified by size exclusion chromatography on a Superdex-200 column (GE HEALTHCARE) and concentrated on Vivaspin filters. Human Arpin was used for production of polyclonal antibodies and competition experiments. Zebrafish Arpin was similarly produced, purified, and used for keratocyte injection at 7.5 μg/μl in 15mM Tris-HCl, 150mM NaCl, 5mM $MgCl_2$, 1mM DTT, pH 7.5. Both proteins had an amino-terminal extension of 10 amino-acids (GAMAHMGRP) after TEV cleavage. ArpinA peptide (residues 211-226 of full length Arpin) was purchased from Proteogenix. For the SEC-MALS characterisation, proteins were separated in a 15-ml KW-803 column (Shodex) run on a Shimadzu HPLC system. MALS, QELS and RI measurements were achieved with a MiniDawn Treos (WYATT TECHNOLOGY), a WyattQELS (WYATT TECHNOLOGY) and an Optilab T-rEX (WYATT TECHNOLOGY), respectively. Molecular weight and hydrodynamic radius calculations were performed with the ASTRA VI software (Wyatt Technology) using a *dn*/*dc* value of 0.183 $mL.g^{-1}$.

### 3. Antibodies

Polyclonal antibodies targeting Arpin were obtained in rabbits (AGRO-BIO) against the purified human Arpin and purified by affinity purification on a HiTrap NHS-activated HP column (GE HEALTHCARE) coupled to the immunogen.

ArpC2 pAb and cortactin mAb (clone 4F11) were from MILLIPORe. ArpC5 mAb (clone 323H3) was from SYNAPTIC SYSTEMS. BrkI mAb (clone 231H9) was described earlier[37]. Tubulin mAb (clone E7) was obtained from Developmental Studies Hybridoma Bank. PC mAb (clone HPC4) was from ROCHE.

### 4. In vitro assays of actin polymerization

Pyrene actin assays and monitoring of the branching reaction were performed as described in reference 38 with the conditions described in figure legends. VCA refers to the VCA domain of WAVE1 purified as described[39].

### 5. Fluorescence anisotropy based determination of Kd

The ArpinA peptide was synthesized and labelled with 5-TAMRA at the N-terminus (PROTEOGENIX). The peptide was excited with polarised light at 549nm and emitted light was detected at 573 nm using a MOS450 fluorimeter (BIOLOGIC). Measurements were made for 60 s at 1 point/s and the average anisotropy was calculated with the Biologic software. Fits were performed as described in reference 40.

### 6. GST pull down, immunoprecipitations, SDS-PAGE and Western Blots

HeLa cells were lysed in 50 mM Tris-HCl, 150 mM NaCl, 1 mM EDTA, 1 mM DTT, 0.5% Triton-X100, 5% glycerol, pH 7.5. 20 μg of GST fusion protein associated with 20 μl of glutathione sepharose 4B beads (GE HEALTHCARE) were incubated with 1 ml of HeLa cell extracts for 2 h at 4°C. Beads were washed and analysed by western blot.

Co-immunoprecipitation of Arpin with the Arp2/3 complex was performed with either two 15 cm-dishes of MEF cells or one 10-cm dish of transfected 293T cells. Cell lysates prepared in 10 mM HEPES pH 7.7, 50 mM KCl, 1mM $MgCl_2$, 1 mM EGTA, 1% Triton X100 were incubated with 10 μg of non-immune Rabbit IgG or 10 μg of anti-Arpin antibodies coupled to tosyl-activated dynabeads (LIFE TECHNOLOGIES) or to GFP-trap beads (CHROMOTEK). Beads were incubated with extracts for 2 h at 4°C, washed and analysed by western blot.

SDS-PAGE was performed using NuPAGE 4-12% Bis-Tris gels (Life Technologies). For western blots, nitrocellulose membranes were developed using HRP-coupled antibodies, Supersignal kit (PIERCE) and a LAS-3000 imager (FUJIFILM).

### 7. Cells and transfections

hTERT immortalised RPE1 cells (CLONTECH) were grown in DMEM/HAM's F12, MEFs and 293T cells in DMEM, MDA-MB-231 cells were grown in RPMI, all media were supplemented with 10% FBS (media and serum from PAA LABORATORIES).

RPE1 cells were electroporated with ECM 630 BTX (HARVARD APPARATUS). $1x10^7$ cells were resuspended in 200 μl of serum free DMEM/HAM'S F12 medium containing 7.5 mM HEPES pH 7.5, mixed with 10 to 40 μg DNA plasmid in 50 μl of 210 mM NaCl and electroporated at 1500 μFD and 250 V. To isolate stable Arpin depleted clones, MDA-MB-231 cells were transfected with shRNA Arpin #3 or shControl using Lipofectamine 2000, and clones selected with 10 μg/ml blasticidin (Invivogen) were isolated with cloning rings and expanded. For rescue experiments, cells transfected with shRNA#3, which targets the 3'UTR, were transfected with GFP-Arpin, which lacks UTR sequences. To validate Arpin localisation, MEFs were transfected with non-targeting (D-001810-10) or Arpin targeting (J-059240-10; ON-TARGET plus siRNA, DHARMACON) using lipofectamine RNAiMax (LIFE TECHNOLOGIES), and examined after 2 days.

### 8. Immunofluorescence and live imaging of mammalian cells

Cells were fixed in 10% TCA, permeabilised with 0.2% Triton X-100 and processed for immunofluorescence. To draw radial line scans, a custom made ImageJ plug-in was developed, edge was determined using 'Isodata' thresholding, then a custom made VBA macro in Excel was used to align data relative to the edge. Lamellipodial dynamics and random migration were analysed with ImageJ using the plugins 'Kymograph' and 'MtrackJ', respectively. All imaging was done on a Axio Observer microscope (ZEISS) equipped with a Plan-Apochromat 63x/1.40 oil immersion objective, an EC Plan-Neofluar 40x/1.30 oil immersion objective and a Plan-Apochromat 20x/0.80 air objective, a Hamamatsu camera C10600 Orca-$R^2$ and a Pecon Zeiss incubator XL multi S1 RED LS (Heating Unit XL S, Temp module, $CO_2$ module, Heating Insert PS and $CO_2$ cover).

### 9. Fish keratocytes

Keratocytes were isolated from scales of freshly killed brook trout (*Salvelinus fontinalis*) as previously described [41] and imaged by phase contrast on an inverted Zeiss Axioscope using x63 optics, and a halogen lamp as light source. Microinjection was performed with a micromanipulator (LEITZ) and a micro-injector Femtojet (EPPENDORF) controlling backpressure and injection pulses. Contours were analysed using the CellTrack software (OHIO STATE UNIVERSITY).

### 10. Zebrafish

Embryos were obtained by natural spawning of *Tg(-1.8gsc:GFP)ml1* fish[42]. In these embryos, prechordal plate cells can be identified by their expression of GFP. In situ hybridisation was performed following standard protocols[43]. For loss of function experiments, a morpholino directed against *arpin* (GTTGTCATAAATACGACTCATCTTC; SEQ ID NO: 19), where the underlined anticodon corresponds to the initiating ATG codon) or a standard control morpholino (CCTCTTACCTCAGTTACAATTTATA; SEQ ID NO: 20) was injected at the one-cell stage, together with Histone2B-mCherry mRNAs or Lifeact-mCherry mRNAs, and GFP-Arpin mRNAs for rescue experiments. To analyse cell trajectories, confocal z-stacks were acquired every minute using a Nikon confocal spinning disk with an Evolve camera (PHOTOMETRICS). Nuclei were tracked using Imaris (BITPLANE). Further analyses were performed using custom routines in Matlab (MATHWORKS)[32].

### 11. *Dictyostelium discoideum*

Cultivation and transformation by electroporation of *D. discoideum* cells was performed as described[44]. To knock-out Arpin, 2 genomic fragments of the arpin gene were cloned into the pLPBLP vector[45]. Briefly, the coding sequences of *Dictyostelium discoideum* Arpin was amplified from the genome of an Ax2 wild type amoeba, using oligonucleotides DdArpin_BU CGCGGATCCGCATGAGTTCAAGTACAAATTATAGT (SEQ ID NO: 21) and DdArpin_SD CGCGTCGACTTTATTTCCATTCATCATCATCTTC (SEQ ID NO:22). The cloned PCR fragment was then used as a template to amplify a 5' fragment (using CGCGGATCCGCATGAGTTCAAGTACAAATTATAGT (SEQ ID NO: 22) and GCGCTGCAGCATCTGAAATTGCAACTGATAGTTG (SEQ ID NO: 23)) and a 3' fragment (using GCGAAGCTTTCTTCTTTACCTTCAAATTTTCAT (SEQ ID NO: 24) and CGCGTCGACGTTGGTTATTTGATTCTATTTGATC (SEQ ID NO: 25)). These 2 fragments were cloned as to flank a cassette carrying Blasticidin resistance in pLPBLP vector. The linearised vector was electroporated to induce recombination in the Arpin gene. Arpin knock-out clones were selected in HL5c-medium supplemented with 10μg/ml blasticidin S (INVIVOGEn). GFP-Arpin re-expressing KO lines were obtained after electroporation of pDGFP-Arpin and selection with 10 μg/ml geneticin (SIGMA). Two time series with more than 30 cells each were acquired per amoeba. 2 clones isolated after each transformation gave similar results.

### 12. qRT-PCR analysis of breast tumour RNA samples

All patients had primary unilateral non-metastatic breast carcinoma at the time of diagnosis and surgery in Institut Curie-Centre René Huguenin. Treatment consisted of modified radical mastectomy in 281 cases (61.9%) and breast-conserving surgery plus locoregional radiotherapy in 163 cases (35.9%). The patients had a physical examination and routine chest radiotherapy every 3 months for 2 years, then annually. Mammograms were done annually. Adjuvant therapy was administered to 354 patients, consisting of chemotherapy alone in 89 cases, hormone therapy alone in 173 cases and both treatments in 92 cases. During a median follow-up of 8.9 years (range 6 months to 29 years), tumours from 167 patients metastasized. Total RNA was extracted from breast samples containing more than 70% tumour cells. qRT-PCR was performed as previously described[46] using the following primers Arpin-U (5'- CTT CCT CAT GTC GTC CTA CAA GGT G -3' (SEQ ID NO: 26)) and Arpin-L (5'-CTG TCA GCG CGA GCA GCT CT-3' (SEQ ID NO: 27)) for *Arpin* gene, and TBP-U (5'- TGC ACA GGA GCC AAG AGT GAA -3'(SEQ ID NO: 28)) and TBP-L (5'-CAC ATC ACA GCT CCC CAC CA -3' (SEQ ID NO: 29)) for the *TBP* control gene.

### 13. Immunohistochemistry of patient biopsies

Biopsies from breast cancer patients of the Blokhin Institute, who had undergone mastectomy without preliminary therapy, were serially sectioned and frozen in liquid nitrogen. Sections were fixed with acetone-methanol and stained with Arpin rabbit polyclonal antibodies, keratin 8 (clone H1, IgG1) and keratin 17 (clone E3, IgG2b) mouse mAbs[47] followed by fluorescent anti-rabbit and isotype specific anti-mouse antibodies (SOUTHERN BIOTECHNOLOGY). Fluorescence intensity was calculated from specific regions (S) and surrounding regions (C) of the same size according to the formula I = (S-C)/C. An average per biopsy was calculated from 5 to 10 micrographs using 20 to 30 regions per micrograph.

### 14. Statistics

Statistical analysis of the results was carried out with SigmaStat software (SPSS inc., v2.03). When data satisfied the two criteria of normality and equal variance, parametric tests were used: t-test to compare two groups; ANOVA for more than two. Where indicated, a bijective transformation was applied to the data in order to pass the two criteria of normality and equal variance. When data did not satisfy both criteria even after transformation, non-parametric tests were applied: Mann-Whitney to compare two groups; Kruskal-Wallis for more than two. A representative experiment is plotted and results are expressed as means and standard error of the mean (sem) with respect to the number of cells (n).

For gene expression in tumours, distributions of target mRNA levels were characterised by their median values and ranges. Relationships between mRNA levels of the different target genes, and between mRNA levels and clinical parameters, were identified using non-parametric tests, namely the chi-square test (relation between two qualitative parameters), the Mann-Whitney U test (relation between one qualitative parameter and one quantitative parameter) and the Spearman rank correlation test (relation between two quantitative parameters). Survival distributions were estimated by the Kaplan-Meier method, and the significance of differences between survival rates was ascertained using the log-rank test.

Differences were considered significant at confidence levels greater than 95%. Three levels of statistical significance are distinguished: * P<0.05; ** P<0.01; *** P<0.001.

__Example 2:__ **Identification and molecular characterization of Arpin.**

To identify an Arp2/3 inhibitory protein to counteract WAVE at lamellipodia, a bioinformatics search was performed for proteins displaying the typical A motif of human NPFs, characterized by a tryptophan residue at the antepenultimate position in an acidic context. An uncharacterized protein (C15orf38) clustered with NPFs was identified with this procedure (Fig. 1 a; Table I).

**Table I: List of top hits for potential Arp2/3 binding proteins in the bioinformatic screen* (SEQ ID NO: 30, 33, 34, 7, 35 to 38, 32 and 39 to 41)**

| Accession ID | Last 16 amino-acids | Number of D and E | Description |
|---|---|---|---|
| *000401/WASL_HUMAN* | *DEDDEEDFEDDDEWED* | *14* | *Neural Wiskott-Aldrich syndrome protein - Homo sapiens* |
| *P42768/WASP_HUMAN* | *GEDQAGDEDEDDEWDD* | *11* | *Wiskott-Aldrich syndrome protein - Homo sapiens* |
| *Q9Y6W5/WASF2_HUMAN* | *DSEDDSSEFDEDDWSD* | *10* | *Wiskott-Aldrich syndrome protein family member 2 - Homo sapiens* |
| **Q7Z6K5\|CO038_HUMAN** | **EIREQGDGAEDEEWDD** | **9** | **UPF0552 protein C15orf38 - Homo sapiens** |
| *Q92558/WASF1_HUMAN* | *SDSEDDSEFDEVDWLE* | *9* | *Wiskott-Aldrich syndrome protein family member 1 - Homo sapiens* |
| *Q9UPY6/WASF3_HUMAN* | *SDSDDDSEFDENDWSD* | *9* | *Wiskott-Aldrich syndrome protein family member 3 - Homo sapiens* |
| *Q8IV90/WASF4_HUMAN* | *DSEDDSSEFDGDDWSN* | *8* | *Wiskott-Aldrich syndrome protein family member 4 - Homo sapiens* |
| Q5T1M5\|FKB15_HUMAN | PLFGDDDDDDDIDWLG | 8 | FK506-binding protein 15 - Homo *sapiens* |
| *A8K0Z3/WASH1_HUMAN* | *PPQQPQAEEDEDDWES* | *7* | *WAS protein family homolog 1 - Homo sapiens* |
| *Q6VEQ5/WASH2_HUMAN* | *PPQQPQAEEDEDDWES* | *7* | *WAS protein family homolog 2 - Homo sapiens* |
| *A8MWX3/WASH4_HUMAN* | *PPPQQPQAEDEDDWES* | *6* | *Putative WAS protein family homolog 4 - Homo sapiens* |
| Q7RTN6\|STRAD_HUMAN | GLVTNLEELEVDDWEF | 6 | STE20-related adapter protein - Homo *sapiens* |
| Q9HBV2\|SACA1_HUMAN | PTEMPGEDDALSEWNE | 6 | Sperm acrosome membrane-associated protein 1 precursor- Homo sapiens |

*** Arpin is in bold; NPFs are in itallics**

This protein was named Arpin. The Arpin family has been annotated as the Uncharacterized Protein Family UPF0552. Arpin was detected in a variety of animals, but not in plants, nor in yeasts. In any given organism, only a single Arpin gene was identified. Thus, Arpin is encoded by a single gene in metazoans and amoeba (Table II).

**Table II: Arpin orthologs are found in multiple species**

| Species | Description | Accession # | Length (aa) | % Identity |
|---|---|---|---|---|
| *H. sapiens* | Human | AAH53602 | 226 | 100 |
| *M. musculus* | Mouse | AAH31379 | 226 | 88 |
| *X. tropicalis* | Frog | AAH75312 | 226 | 64 |
| *D. rerio* | Zebrafish | AAH93306 | 226 | 58 |
| *S. purpuratus* | Sea urchin | XP_787889 | 226 | 48 |
| *D. discoideum* | Amoeba | XP_635450 | 224 | 22 |

The Arpin protein is expressed in many mouse tissues (Figure 2). Predictions and NMR analysis indicate that this protein of about 220 residues is structured with the exception of its highly mobile 20 C-terminal residues, which contain the putative Arp2/3 binding site (Figures 3-4).

Indeed, Arpin directly binds to Arp2/3, mostly through its Acidic motif (Fig.1b; Kd in Figure 5).

The molecular function of Arpin on Arp2/3 activity was assayed by spectrofluorimetry and TIRF microscopy using purified proteins (Figure 6). Arpin did not affect spontaneous actin nucleation, nor elongation, and was unable to activate the Arp2/3 complex consistent with its lack of VC motifs (Figure 7). However, when Arp2/3 was activated by VCA, Arpin, but not its truncated form lacking the Acidic motif (ArpinΔA), inhibited actin polymerisation in a dose-dependent manner (Fig.1c; Figure 8). The Acidic peptide was sufficient for this inhibition, although it was less effective than full length Arpin, in line with its lower affinity for the Arp2/3 complex (Figure 9). Arpin inhibited Arp2/3 activation, since we observed by TIRF microscopy the generation of fewer actin branched junctions in the presence of Arpin (Fig.1d; Figure 10 for quantifications). Since Arpin and NPFs share a homologous acidic motif, it was tested whether Arpin can prevent the VCA from binding the Arp2/3 complex. Indeed, full length Arpin and its Acidic motif, but not ArpinΔA, compete with VCA for Arp2/3 binding (Fig.1e; Figure 9). Therefore Arpin is a new competitive inhibitor of the Arp2/3 complex. The name Arpin is a mnemonic for its activity (<u>Arp</u>2/3 inhibition).

The subcellular localisation of Arpin was examined by immunofluorescence in spreading Mouse Embryonic Fibroblasts (MEFs). Arpin was detected in restricted segments of the plasma membrane (Figure 11a). These Arpin positive segments of the plasma membrane were also stained by three lamellipodial markers, the WAVE complex, the Arp2/3 complex and cortactin (Fig 11a; Figs. 12-13). The lamellipodial staining of Arpin was specific, since it was lost upon siRNA-mediated depletion of Arpin (Fig. 12). Radial line scans of immunofluorescence pictures through lamellipodial outlines were generated, registered with the edge as a reference, and averaged to reveal the relative distributions of these different lamellipodial components. Arpin overlapped perfectly with the distribution of the WAVE complex, a tip component (Fig.2a, ref. 12). Arpin is thus localised at the lamellipodium tip, where new actin branches are nucleated by WAVE and Arp2/3 complexes. As expected, Arp2/3 and cortactin distribution extended rearwards compared to Arpin (Fig.12), since Arp2/3 and cortactin correspond to branches of the lamellipodial actin network undergoing retrograde flow with respect to the protruding membrane[9,13]. The branched junction undergoes retrograde flow like actin itself due to actin filament elongation[9,12]. Cortactin recognizes Arp2/3 at the branch junction and is thought to stabilise branched actin networks[13]. As a marker of the branched junction, cortactin stains the width of lamellipodia, like the Arp2/3 complex.

<u>**Example 3:**</u> **Regulation of Arpin activity**

The role of Rac, the master controller of lamellipodium formation, was examined to understand the regulation of Arpin activity. 293T cells were co-transfected with different forms of Rac and GFP-Arpin and then the interaction of Arpin with the Arp2/3 complex was analysed through GFP immunoprecipitations. The active form of Rac1, which is sufficient to induce lamellipodia, was also sufficient to induce Arp2/3 co-immunoprecipitation with Arpin (Fig. 11b). Rac1 knock-out MEFs that lack lamellipodia (Steffen et al., J. Cell. Sci., 2013, July 31) were employed to examine whether Rac is required for Arpin activation. The absence of Rac abrogated the peripheral localisation of Arpin in all knock-out MEF cells examined (Fig. 14). Endogenous Arpin was then immunoprecipitated from Rac1 knock-out MEFs. The Arp2/3 complex co-immunoprecipitated with Arpin in control MEF cells, but not in Rac-deficient cells (Fig.2c). Rac does not directly bind to Arpin and thus activates it indirectly through a signalling pathway (Fig. 15). Together these results show that, in response to Rac signalling, Arpin inhibits the Arp2/3 complex at the lamellipodium tip, i.e. where Rac also stimulates actin polymerisation through the WAVE complex.

This counter-intuitive finding suggests that Arpin would be a built-in brake of protrusions. Thus, lamellipodial dynamics was examined in cells depleted of Arpin. shRNA expressing plasmids that efficiently deplete human Arpin were designed and validated (Fig. 16). The immortalized human RPE1 cells were transiently transfected and selected by blasticidin. Arpin depletion using several different shRNAs increased lamellipodia-mediated cell spreading (Fig. 16).

Arpin depletion increased protrusion velocity of lamellipodia, consistent with its Arp2/3 inhibitory role (Fig.11d). This effect was fully rescued by Arpin re-expression, but only when Arpin harboured the Acidic motif. Arpin thus provides a paradoxical negative circuit downstream of Rac. Such a circuitry, where Rac induces and inhibits actin polymerisation, generates a so-called 'incoherent feedforward loop' (Fig.11e), which can favour temporal regulations[14].

**Example 4: Effect of Arpin expression on cell migration in physiological conditions**

To examine whether the Arpin circuit is physiologically relevant for cell migration, the expression of the *arpin* gene was impaired in zebrafish embryos using morpholinos. During gastrulation, prechordal plate cells undergo a collective migration towards the animal pole. Upon *arpin* loss of function, cell movements were less coordinated (Fig. 17). Prechordal plate cell transplants revealed a cell autonomous effect of Arpin on protrusions. Protrusions were more frequent and more persistent over time in the absence of Arpin. This observation is consistent with the incoherent feedforward loop, a circuitry that can suppress the protrusion it creates.

To further understand the role of the incoherent feedforward loop in cell migration, Arpin loss-of-function experiments were performed in cell systems migrating as individual cells. The set of shRNA plasmids targeting Arpin were first used to select stably depleted clones from the breast invasive human cell line MDA-MB-231 (Fig. 18a). Migration of these cells was analysed by video microscopy in 2D or 3D (Fig.18b; Fig. 19). In both cases, the tracks illustrate that Arpin-depleted cells explored a larger territory than control cells, an observation substantiated by mean square displacements (Fig. 20). Increased exploration was not only due to increased speed, but also to increased directional persistence, measured as the ratio of the distance between two points by the actual trajectory (Fig.18c) or as the direction autocorrelation function (Fig.21). Since Arpin is conserved in amoeba, its function was analysed by generating a knock-out of the orthologous Arpin gene in *Dictyostelium discoideum,* a model organism for cell migration (Fig. 22). Arpin knock-out *Dictyostelium* amoebae explored a wider territory than the controls (Fig.18d). As in mammalian cells, both cell speed and protrusion velocity of pseudopods were increased (Fig.18e; Fig. 23). In amoeba, Arpin removal had a stronger effect on directionality than on cell speed. Overexpression of GFP-Arpin in knock-out amoeba restricted the explored territory as compared to wild type (Fig.18d; Fig. 20). Directional persistence, which is more than fully rescued by GFP-Arpin expression, can account for this effect (Fig.18e; Supplementary Fig. 21). These loss-of-function experiments in distant systems thus indicated that the major function of Arpin at the cell level is to restrict exploration by decreasing both cell speed and directional persistence of migration.

Arpin could thus be a 'steering factor'. The fish keratocyte model was selected to test this hypothesis directly, in a gain-of-function experiment. These cells are characterized by fast migration based on a wide fan-shaped lamellipodium with high directional persistence (Fig.24a, Figures 20, 21, 25). Zebrafish Arpin and Arpin∆A were purified from *E. coli* (Fig. 6) and these recombinant proteins were microinjected into migrating trout keratocytes. Injection of Arpin, but not of Arpin∆A, caused pronounced cell shape changes (Fig.24a). Strikingly, Arpin did not prevent lamellipodia protrusion, but resulted in cycles of suppression of existing lamellipodia followed by formation of new, ectopic ones (Fig.24b). Lamellipodial instability caused keratocytes to reduce their speed and to deviate from their initial direction of migration (Fig.24c; Fig. 21).

Purified recombinant Arpin can be introduced efficiently into various cell types by electroporation as shown in figure 26 with retinal pigment epithelial (RPE-1) cells, mouse embryonic fibroblasts (MEFs) and breast invasive human cell line MDA-MB-231. MDA-MB-231 cells depleted of Arpin migrates faster and more directionally than their respective non-targeting controls (shCtrl). However, when electroporated with purified Arpin, their speed and persistence decreased (figure 27).

Collectively, the experiments performed in different systems of cell migration thus supported a role for Arpin in promoting cell steering: Arpin slows down cells and allows them to turn.

In a computational model of efficient and persistent cell migration, the lamellipodium spatially determines where the WAVE and the Arp2/3 complexes will next polymerise actin, thus maintaining the front at the front over time (Fig.24d)[15]. Such a feedback, sensing where branched actin is polymerised and activating Rac as a response, has recently been identified: it involves Coronin1A and the Rac exchange factor β-Pix[16]. In this feedback, the WAVE complex closes a positive feedback loop that maintains efficient directional migration over time, whereas Arpin closes a concurrent negative feedback loop, which induces braking and allows turning. These two nested feedback loops, positive and negative, can account for the emergence of oscillations in lamellipodium protrusion/retraction, as observed in fish keratocytes upon Arpin injection, and for various travelling actin waves described in different systems[17-20]

**Example 5: Arpin underexpression correlates with poor prognosis in cancer**

The negative feedback loop contributed by Arpin provides a homeostatic mechanism, whereas the positive feedback loop contributed by WAVE commits cells toward an exploratory behaviour. Thus tumours, which can form metastases when tumour cells escape the primary tumour were examined and surrounding tissues were explored.

Expression of the three Arp2/3 inhibitors, Arpin, PICK1 and Gadkin was examined in a large series of about 450 breast tumours from patients and in 10 normal breast tissue from women undergoing cosmetic breast surgery. mRNA values were quantified using qRT-PCR. Values of breast cancer samples were normalised to the median of the 10 normal breast tissue values.

**Table III: Characteristics of breast tumours relative to Arpin and Gadkin mRNA expression**

| | Prognosis status | | | Arpin status | | | Gadkin status | | | |
|---|---|---|---|---|---|---|---|---|---|---|
| | Total population (%) | Metastases | *P-value*[a] | Arpin mRNA under expressed | Arpin mRNA normally expressed | *P-value*[b] | Total population (%) | Gadkin mRNA normally expressed | Gadkin mRNA over expressed | *P-value*[b] |
| *Total* | 454 (100.0) | 167 (36.8) | | 33 (7.3) | 421 (92.7) | | 446 (100.0) | 408 (91.5) | 38 (8.5) | |
| *Age* | | | | | | | | | | |
| ≤50 | 97 (21.4) | 36 (21.6) | 0.63 (NS) | 9 (9.3) | 88 (90.7) | 0.39 (NS) | 94 (21.1) | 92 (97.9) | 2 (2.1) | **0.012** |
| >50 | 357 (78.6) | 131 (78.4) | | 24 (6.7) | 333 (93.3) | | 352 (78.9) | 316 (89.8) | 36 (10.2) | |
| *SBR histological grade*[c,a] | | | | | | | | | | |
| I | 58 (13.0) | 8 (4.9) | **0.00011** | 1 (1.7) | 57 (98.3) | **0.014** | 57 (13.0) | 54 (94.7) | 3 (5.3) | **0.014** |
| II | 228 (51.2) | 83 (50.9) | | 13 (5.7) | 215 (94.3) | | 223 (51.0) | 195 (87.4) | 28 (12.6) | |
| III | 159 (35.7) | 72 (44.2) | | 19 (11.9) | 140 (88.1) | | 157 (35.9) | 150 (95.5) | 7 (4.5) | |
| *Lymph node status*[e] | | | | | | | | | | |
| 0 | 119 (26.3) | 3 (21.0) | **0.0000027** | 10 (8.4) | 109 (91.6) | 0.85 (NS) | 118 (26.5) | 105 (89) | 13 (11) | 0.46 (NS) |
| 1-3 | 237 (52.3) | 77 (46.1) | | 16 (6.8) | 221 (93.2) | | 232 (52.1) | 213 (91.8) | 19 (8.2) | |
| >3 | 97 (21.4) | 55 (32.9) | | 7 (7.2) | 90 (92.8) | | 95 (21.3) | 89 (93.7) | 6 (6.3) | |
| *Macroscopic tumour size*[f] | | | | | | | | | | |
| ≤25mm | 222 (49.8) | 62 (37.3) | **0.000017** | 11 (5.0) | 211 (95.0) | **0.050** | 220 (50.2) | 200 (90.9) | 20 (9.1) | 0.50 (NS) |
| >25mm | 224 (50.2) | 104 (62.7) | | 22 (9.8) | 202 (90.2) | | 218 (49.8) | 202 (92.7) | 16 (7.3) | |
| *ERastatus* | | | | | | | | | | |
| Negative | 116 (25.6) | 48 (28.7) | **0.021** | 11 (9.5) | 105 (90.5) | 0.29 (NS) | 115 (25.8) | 113 (98.3) | 2 (1.7) | **0.0025** |
| Positive | 338 (74.4) | 119 (71.3) | | 22 (6.5) | 316 (93.5) | | 331 (74.2) | 295 (89.1) | 36 (10.9) | |

(continued)

| | Prognosis status | | | Arpin status | | | Gadkin status | | | |
|---|---|---|---|---|---|---|---|---|---|---|
| | Total population (%) | Metastases | *P-value*[a] | Arpin mRNA under expressed | Arpin mRNA normally expressed | *P-value*[b] | Total population (%) | Gadkin mRNA normally expressed | Gadkin mRNA over expressed | *P-value*[b] |
| *PR status* | | | | | | | | | | |
| Negative | 192 (42.3) | 83 (49.7) | **0.0024** | 19 (9.9) | 173 (90.1) | 0.065 (NS) | 190 (42.6) | 177 (93.2) | 13 (6.8) | 0.27 (NS) |
| Positive | 262 (57.7) | 84 (50.3) | | 14 (5.3) | 248 (94.7) | | 256 (57.4) | 231 (90.2) | 25 (9.8) | |
| *ERBB2 status* | | | | | | | | | | |
| Negative | 357 (78.6) | 127 (76.0) | 0.17 (NS) | 28 (7.8) | 329 (92.2) | 0.37 (NS) | 353 (79.1) | 323 (91.5) | 30 (8.5) | |
| Positive | 97 (21.4) | 40 (24.0) | | 5 (5.2) | 92 (94.8) | | 93 (20.9) | 85 (91.4) | 8 (8.6) | 0.97 (NS) |
| *Molecular subtypes*[g] | | | | | | | | | | |
| HR- ERBB2- | 68 (15.0) | 26 (15.6) | **0.032** | 8 (11.8) | 60 (88.2) | 0.38 (NS) | 68 (15.2) | 66 (97.1) | 2 (2.9) | **0.014** |
| HR-ERBB2+ | 43 (9.5) | 21 (12.6) | | 3 (7.0) | 40 (93.0) | | 42 (9.4) | 42 (100) | 0 (0) | |
| HR+ ERBB2- | 289 (63.7) | 101 (60.5) | | 20 (6.9) | 269 (93.1) | | 285 (63.9) | 257 (90.2) | 28 (9.8) | |
| HR+ERBB2+ | 54 (11.9) | 19 (11.4) | | 2 (3.7) | 52 (96.3) | | 51 (11.4) | 43 (84.3) | 8 (15.7) | |

NS: not significant

[a] Log-rank test.

[b] $\chi^2$ Test

[d] Information available for only 445 patients.

[e] Information available for only 453 patients.

[g] HR refers to Hormone Receptors (both estrogen receptor $\alpha$ and progesterone receptor)

**Table IV: Relationships between PICK1 transcript levels and clinical biological parameters**

| | Total population (%) | PICK1 transcript levels relative to normal breast Median (range) | *P-value*[a] |
|---|---|---|---|
| *Total* | 446 (100.0) | 0.99 (0.10-4.46) | |
| *Age* | | | |
| ≤50 | 94 (21.1) | 0.98 (0.39-4.13) | 0.48 (NS) |
| >50 | 352 (78.9) | 0.99 (0.10-4.46) | |
| *SBR histological grade*[b,c] | | | |
| I | 57 (13.0) | 1.05 (0.45-2.76) | 0.26 (NS) |
| II | 223 (51.0) | 0.97 (0.10-3.67) | |
| III | 157 (35.9) | 1.00 (0.35-4.46) | |
| *Lymph node status*[d] | | | |
| 0 | 118 (26.5) | 1.07 (0.37-4.13) | 0.53 (NS) |
| 1-3 | 232 (52.1) | 0.99 (0.19-4.46) | |
| >3 | 95 (21.3) | 0.96 (0.10-3.19) | |
| *Macroscopic tumor size*[e] | | | |
| ≤25mm | 220 (50.2) | 1.00 (0.35-4.13) | 0.99 (NS) |
| >25mm | 218 (49.8) | 0.98 (0.10-4.46) | |
| *ER□□status* | | | |
| Negative | 115 (25.8) | 1.01 (0.40-4.46) | 0.28 (NS) |
| Positive | 331 (74.2) | 0.99 (0.10-3.67) | |
| *PR status* | | | |
| Negative | 190 (42.6) | 0.95 (0.10-4.46) | 0.93 (NS) |
| Positive | 256 (57.4) | 1.01 (0.35-3.67) | |
| *ERBB2 status* | | | |
| Negative | 353 (79.1) | 0.96 (0.10-4.46) | **0.0062** |
| Positive | 93 (20.9) | 1.06 (0.44-3.42) | |
| *Molecular subtypes* | | | |
| HR- ERBB2- | 68 (15.2) | 0.92 (0.40-4.46) | **0.047** |
| HR- ERBB2+ | 42 (9.4) | 1.07 (0.53-3.42) | |
| HR+ ERBB2- | 285 (63.9) | 0.97 (0.10-3.67) | |
| HR+ ERBB2+ | 51 (11.4) | 1.03 (0.44-3.05) | |

NS : not significant
[a] Kruskal Wallis's H Test.
[b] Scarff Bloom Richardson classification.
[c] Information available for 437 patients.
[d] Information available for 445 patients.
e Information available for 438 patients.
f Spearman rank correlation.

**Table V: Arpin mRNA under-expression is associated with prognosis in breast cancer**

| Number of tumours (%) | Arpin* | PICK1** | Gadkin** |
|---|---|---|---|
| Under-expressing tumours (N target < 0.33) | 33 **(7.3)** | 2 (0.4) | 5 (1.1) |
| Normo-expressing tumours (0.33 ≤ N target ≤ 3) | 414 **(91.2)** | 436 **(97.8)** | 403 (**90.4**) |
| Over-expressing tumours (N target > 3) | 7 (1.5) | 8 (1.8) | 38 **(8.5)** |
| * information available for 454 tumours<br>** information available for 446 tumours | | | |

PICK1 expression did not significantly vary among the breast tumours (Table IV and V). In contrast, expression of Arpin (C15orf38) and Gadkin (AP1AR) genes varies in a significant number of tumours, Arpin being under-expressed and Gadkin overexpressed (Table III, and V). Furthermore, the down-regulation of Arpin expression at the mRNA level which was found in 7 % of the patients, was associated with poor prognosis for the patients, characterised by reduced metastasis-free survival time (Fig.28). Such a correlation with patient survival was not observed with the other Arp2/3 inhibitors, Gadkin and PICK1.

Arpin was then localised by immunohistochemistry in breast biopsies. Arpin expression was strongly reduced in all 10 invasive carcinomas examined (Fig. 29), suggesting that loss of Arpin expression is a common event in breast cancer progression and that it should frequently occur through post-transcriptional mechanisms.

***Conclusions***

Other proteins were previously shown to regulate cell steering[21,22]. Knock-down of Rac1 or of cofilin, a protein that depolymerises and severs actin filaments, increases directional persistence of mammalian cells[23,24]. These proteins are required, however, for lamellipodial protrusion and actin based motility[25,26] Arpin is unique in that it regulates cell steering, while being dispensable for lamellipodial protrusion and efficient migration. Arpin is a prime candidate to fine-tune numerous physiological migrations biased by diverse cues[22]. Arpin also appears to play a role in preventing cells from migrating. In this respect, dissection of the mechanisms regulating Arpin expression in physiology and pathology is a major challenge ahead of us.

**REFERENCES**

1 Wu, C. et al. Arp2/3 is critical for lamellipodia and response to extracellular matrix cues but is dispensable for chemotaxis. Cell 148, 973-987 (2012).

2 Suraneni, P. et al. The Arp2/3 complex is required for lamellipodia extension and directional fibroblast cell migration. J Cell Biol 197, 239-251 (2012).

3 Mullins, R. D., Heuser, J. A. & Pollard, T. D. The interaction of Arp2/3 complex with actin: nucleation, high affinity pointed end capping, and formation of branching networks of filaments. Proc Natl Acad Sci USA 95, 6181-6186 (1998).

4 Insall, R. H. & Machesky, L. M. Actin dynamics at the leading edge: from simple machinery to complex networks. Dev Cell 17, 310-322 (2009).

5 Padrick, S. B. & Rosen, M. K. Physical mechanisms of signal integration by WASP family proteins. Annu Rev Biochem 79, 707-735 (2010).

6 Ridley, A. J. Life at the leading edge. Cell 145, 1012-1022 (2011).

7 Derivery, E. et al. The Arp2/3 activator WASH controls the fission of endosomes through a large multiprotein complex. Dev Cell 17, 712-723 (2009).

8 Suetsugu, S. & Gautreau, A. Synergistic BAR-NPF interactions in actin-driven membrane remodeling. Trends Cell Biol 22, 141-150 (2012).

9 Pollard, T. D. Regulation of actin filament assembly by Arp2/3 complex and formins. Annu Rev Biophys Biomol Struct 36, 451-477 (2007).

10 Rocca, D. L., Martin, S., Jenkins, E. L. & Hanley, J. G. Inhibition of Arp2/3-mediated actin polymerization by PICK1 regulates neuronal morphology and AMPA receptor endocytosis. Nat Cell Biol 10, 259-271 (2008).

11 Maritzen, T. et al. Gadkin negatively regulates cell spreading and motility via sequestration of the actin-nucleating ARP2/3 complex. Proc Natl Acad Sci U S A 109, 10382-10387 (2012).

12 Lai, F. P. et al. Arp2/3 complex interactions and actin network turnover in lamellipodia. Embo J 27, 982-992 (2008).

13 Cai, L., Makhov, A. M., Schafer, D. A. & Bear, J. E. Coronin 1B antagonizes cortactin and remodels Arp2/3-containing actin branches in lamellipodia. Cell 134, 828-842 (2008).

14 Hart, Y. & Alon, U. The utility of paradoxical components in biological circuits. Mol Cell 49, 213-221 (2013).

15 Neilson, M. P. et al. Chemotaxis: a feedback-based computational model robustly predicts multiple aspects of real cell behaviour. PLoS Biol 9, e1000618 (2011).

16 Castro-Castro, A. et al. Coronin 1A promotes a cytoskeletal-based feedback loop that facilitates Rac1 translocation and activation. Embo J 30, 3913-3927 (2011).

17 Machacek, M. & Danuser, G. Morphodynamic profiling of protrusion phenotypes. Biophys J 90, 1439-1452 (2006).

18 Weiner, O. D., Marganski, W. A., Wu, L. F., Altschuler, S. J. & Kirschner, M. W. An actin-based wave generator organizes cell motility. PLoS Biol 5, e221 (2007).

19 Brandman, O. & Meyer, T. Feedback loops shape cellular signals in space and time. Science 322, 390-395 (2008).

20 Allard, J. & Mogilner, A. Traveling waves in actin dynamics and cell motility. Curr Opin Cell Biol (2012).

21 Gosh, M. et al. Cofilin promotes actin polymerization and defines the direction of cell motility. Science 304, 743-746 (2004).

22 Petrie, R. J., Doyle, A. D. & Yamada, K. M. Random versus directionally persistent cell migration. Nat Rev Mol Cell Biol 10, 538-549 (2009).

23 Pankov, R. et al. A Rac switch regulates random versus directionally persistent cell migration. J Cell Biol 170, 793-802 (2005).

24 Sidani, M. et al. Cofilin determines the migration behavior and turning frequency of metastatic cancer cells. J Cell Biol 179, 777-791 (2007).

25 Li, A. et al. Rac1 drives melanoblast organization during mouse development by orchestrating pseudopod- driven motility and cell-cycle progression. Dev Cell 21, 722-734 (2011).

26 Loisel, T. P., Boujemaa, R., Pantaloni, D. & Carlier, M. F. Reconstitution of actin-based motility of Listeria and Shigella using pure proteins. Nature 401, 613-616 (1999).

27 Edgar, R. C. MUSCLE: multiple sequence alignment with high accuracy and high throughput. Nucleic Acids Res 32, 1792-1797 (2004).

28 Clamp, M., Cuff, J., Searle, S. M. & Barton, G. J. The Jalview Java alignment editor. Bioinformatics 20, 426-427 (2004).

29 McGuffin, L. J., Bryson, K. & Jones, D. T. The PSIPRED protein structure prediction server. Bioinformatics 16, 404-405 (2000).

30 Ward, J. J., McGuffin, L. J., Bryson, K., Buxton, B. F. & Jones, D. T. The DISOPRED server for the prediction of protein disorder. Bioinformatics 20, 2138-2139 (2004).

31 Blanchoin, L. et al. Direct observation of dendritic actin filament networks nucleated by Arp2/3 complex and WASP/Scar proteins. Nature 404, 1007-1011 (2000).

32 Dumortier, J. G., Martin, S., Meyer, D., Rosa, F. M. & David, N. B. Collective mesendoderm migration relies on an intrinsic directionality signal transmitted through cell contacts. Proc Natl Acad Sci USA 109, 16945-16950 (2012).

33 Montero, J. A., Kilian, B., Chan, J., Bayliss, P. E. & Heisenberg, C. P. Phosphoinositide 3-kinase is required for process outgrowth and cell polarization of gastrulating mesendodermal cells. Curr Biol 13, 1279-1289 (2003).

34 Lommel, S. et al. Actin pedestal formation by enteropathogenic Escherichia coli and intracellular motility of Shigella flexneri are abolished in N-WASP-defective cells. EMBO Rep 2, 850-857. (2001).

35 Millard, T. H., Behrendt, B., Launay, S., Futterer, K. & Machesky, L. M. Identification and characterisation of a novel human isoform of Arp2/3 complex subunit p16-ARC/ARPC5. Cell Motil Cytoskeleton 54, 81-90 (2003).

36 Dumontier, M., Hocht, P., Mintert, U. & Faix, J. Rac1 GTPases control filopodia formation, cell motility, endocytosis, cytokinesis and development in Dictyostelium. J Cell Sci 113 (Pt 12), 2253-2265 (2000).

37 Derivery, E. et al. Free Brickl is a trimeric precursor in the assembly of a functional wave complex. PLoS ONE 3, e2462 (2008).

38 Michelot, A. et al. Actin-filament stochastic dynamics mediated by ADF/cofilin. Curr Biol 17, 825-833 (2007).

39 Machesky, L. M. et al. Scar, a WASp-related protein, activates nucleation of actin filaments by the Arp2/3 complex. Proc Natl Acad Sci USA 96, 3739-3744. (1999).

40 Marchand, J. B., Kaiser, D. A., Pollard, T. D. & Higgs, H. N. Interaction of WASP/Scar proteins with actin and vertebrate Arp2/3 complex. Nat Cell Biol 3, 76-82 (2001).

41 Urban, E., Jacob, S., Nemethova, M., Resch, G. P. & Small, J. V. Electron tomography reveals unbranched networks of actin filaments in lamellipodia. Nat Cell Biol 12, 429-435 (2010).

42 Doitsidou, M. et al. Guidance of primordial germ cell migration by the chemokine SDF-1. Cell 111, 647-659 (2002).

43 Hauptmann, G. & Gerster, T. Two-color whole-mount in situ hybridization to vertebrate and Drosophila embryos. Trends Genet 10, 266 (1994).

44 Schirenbeck, A., Bretschneider, T., Arasada, R., Schleicher, M. & Faix, J. The Diaphanous-related formin dDia2 is required for the formation and maintenance of filopodia. Nat Cell Biol 7, 619-625 (2005).

45 Faix, J., Kreppel, L., Shaulsky, G., Schleicher, M. & Kimmel, A. R. A rapid and efficient method to generate multiple gene disruptions in Dictyostelium discoideum using a single selectable marker and the Cre-loxP system. Nucleic Acids Res 32, e143 (2004).

46 Bieche, I. et al. Quantification of estrogen receptor alpha and beta expression in sporadic breast cancer. Oncogene 20, 8109-8115 (2001).

47 Troyanovsky, S. M., Guelstein, V. I., Tchipysheva, T. A., Krutovskikh, V. A. & Bannikov, G. A. Patterns of expression of keratin 17 in human epithelia: dependency on cell position. J Cell Sci 93 (Pt 3), 419-426 (1989).

## Claims

1. A product as a medicament for inhibiting cell migration, said product being selected from the group consisting of:

   a) a protein, denominated Arpin, which is a protein from the Uncharacterized Protein Family UPF0552 or a functional variant thereof,
   b) a peptide of at least 13 consecutive amino acids from the Arpin protein in a) which comprises at least the acidic motif of said Arpin, and
   c) a polynucleotide encoding the Arpin protein in a) or peptide in b) in expressible form, and
   wherein said Arpin protein in a) and peptide in b) inhibit the Arp2/3 complex.

2. The product as a medicament according to claim 1, wherein said protein comprises an amino acid sequence (I) which is at least 70 % identical to residues 1 to 226 of human Arpin amino acid sequence SEQ ID NO: 1 and which comprises an acidic motif.

3. The product as a medicament according to claim 1 or claim 2, wherein said acidic motif consists of the sequence (II):

   $X_1X_2X_3X_4X_5X_6X_7X_8X_9X_{10}X_{11}X_{12}X_{13}X_{14}X_{15}X_{16}X_{17}$, in which:

   $X_1$ represents E, K or is absent, $X_2$ represents I, P, S or is absent, $X_3$ represents G or is absent, $X_4$ represents R, A or Q, $X_5$ represents E, G, A or Q, $X_6$ represents E or Q, $X_7$ represents G, N or Q, $X_8$ represents D or E; $X_9$ represents G or E, $X_{10}$ represents A or E, $X_{11}$ represents D, G or E, $X_{12}$ represents D, $X_{13}$ represents D or E, $X_{14}$ represents E, $X_{15}$ represents W, $X_{16}$ represents D or K and $X_{17}$ represents D or is absent, with the proviso that at least seven of said $X_1$, $X_5$, $X_6$, $X_8$ to $X_{14}$, $X_{16}$ and $X_{17}$ residues are E or D.

4. The product as a medicament according to claim 3, wherein said sequence (II) is SEQ ID NO: 7.

5. The product as a medicament according to any one of claims 1 to 4, wherein said protein or peptide is selected from the group consisting of SEQ ID NO: 1, 2 and 7.

6. The product according to any one of claims 1 to 5 for use in treating a disease caused by aberrant cell migration.

7. The product for use in treating a disease according to claim 6, wherein said disease is cancer.

8. A method *in vitro* for evaluating the prognosis of a cancer in a patient, comprising:

   a) determining the level of an expression product of the Arpin gene in a biological sample from said patient, and
   b) comparing the level in a) with a reference level for said expression product, wherein if the level in a) is lower than said reference level, then said patient suffers from an invasive cancer with an unfavorable prognosis.

9. The method of claim 8, wherein said cancer is a carcinoma.

10. The method of claim 8 or 9, wherein said expression product is human Arpin protein.

11. The method of any one of claims 8 to 10, wherein said sample is a tumor biopsy.

12. An inhibitor of human Arpin protein, as a promoter of cell migration, for use in treating injuries.

13. A method for screening an inhibitor of cell migration, comprising:

   - contacting at least one test molecule with a cell in which Arpin gene expression is inhibited, and

- identifying the molecules capable of increasing the level of expression of said Arpin gene in said cell, compared to a reference level of expression for said Arpin gene.

**14.** A method for screening a promoter of cell migration, comprising:

- contacting at least one test molecule with a cell expressing an Arpin protein, and
- identifying the molecules capable of inhibiting said Arpin protein.

**15.** Use of an Arpin protein to study cell migration.

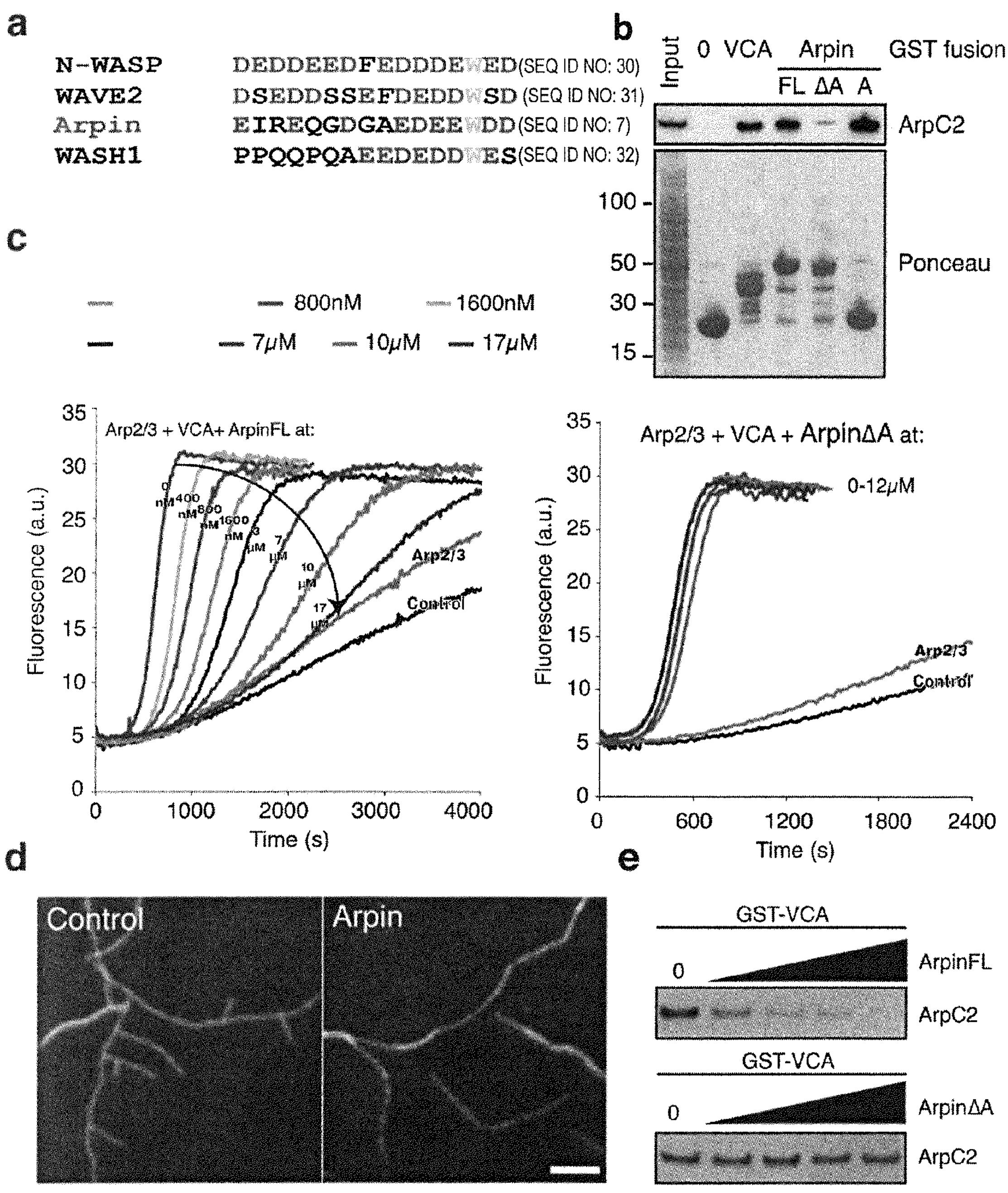
a
N-WASP DEDDEEDFEDDDE WED (SEQ ID NO: 30)
WAVE2 DSEDDSSEFDEDD WSD (SEQ ID NO: 31)
Arpin EIREQGDGAEDEE WDD (SEQ ID NO: 7)
WASH1 PPQQPQAEEDEDD WES (SEQ ID NO: 32)
b
Input
0
VCA
Arpin
GST fusion
FL
ΔA
A
ArpC2
100
50
30
15
Ponceau
c
800nM
1600nM
7μM
10μM
17μM
Arp2/3 + VCA+ ArpinFL at:
Fluorescence (a.u.)
Time (s)
Arp2/3
Control
Arp2/3 + VCA + ArpinΔA at:
0-12μM
d
Control
Arpin
e
GST-VCA
0
ArpinFL
ArpC2
GST-VCA
0
ArpinΔA
ArpC2

FIGURE 1

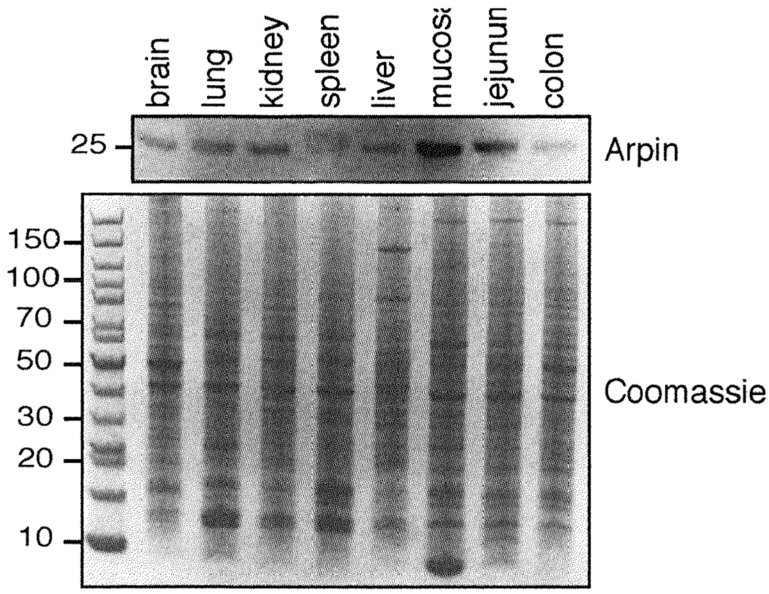
brain
lung
kidney
spleen
liver
mucosa
jejunum
colon
25
Arpin
150
100
70
50
30
20
10
Coomassie

FIGURE 2

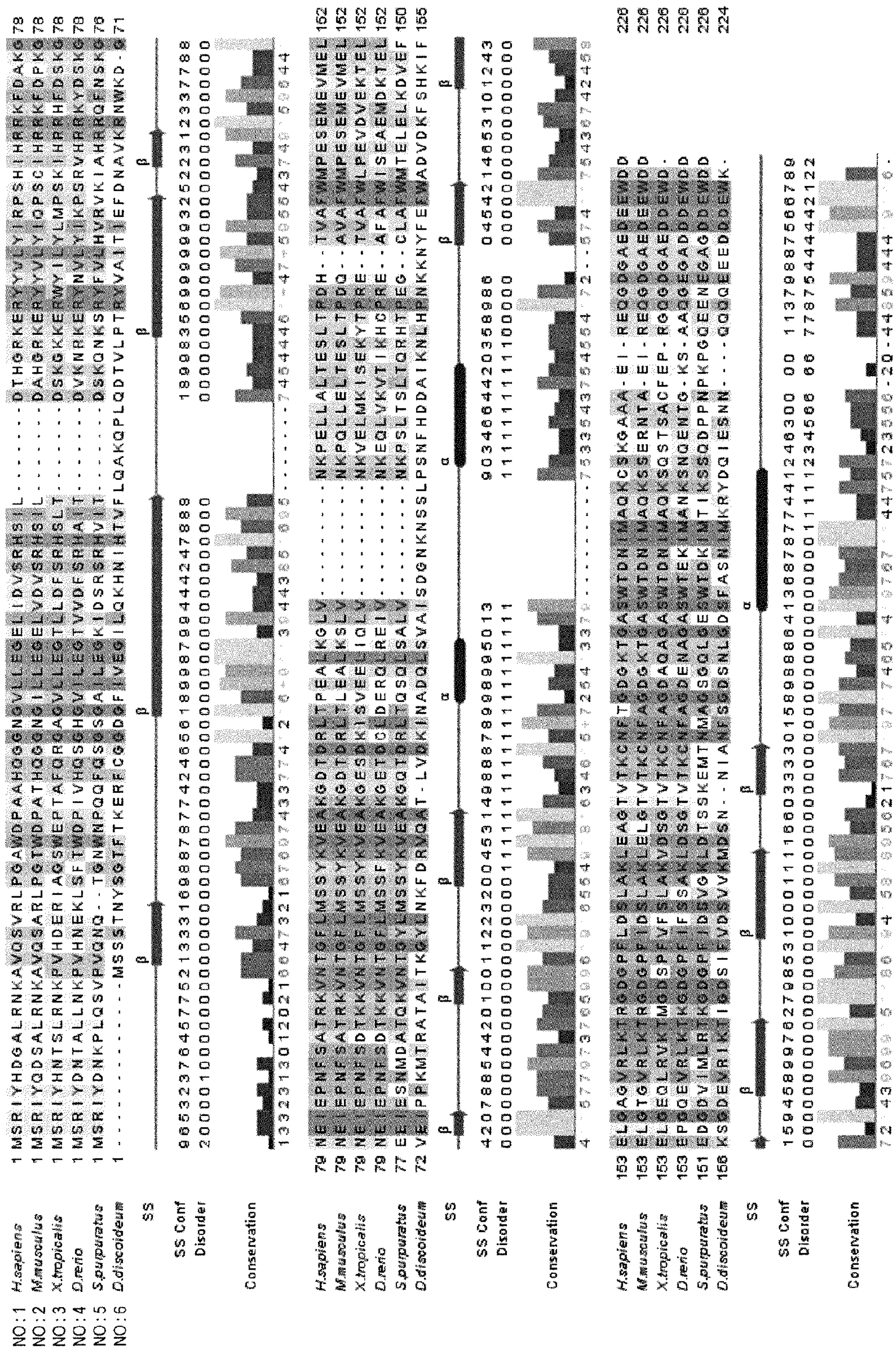

FIGURE 3

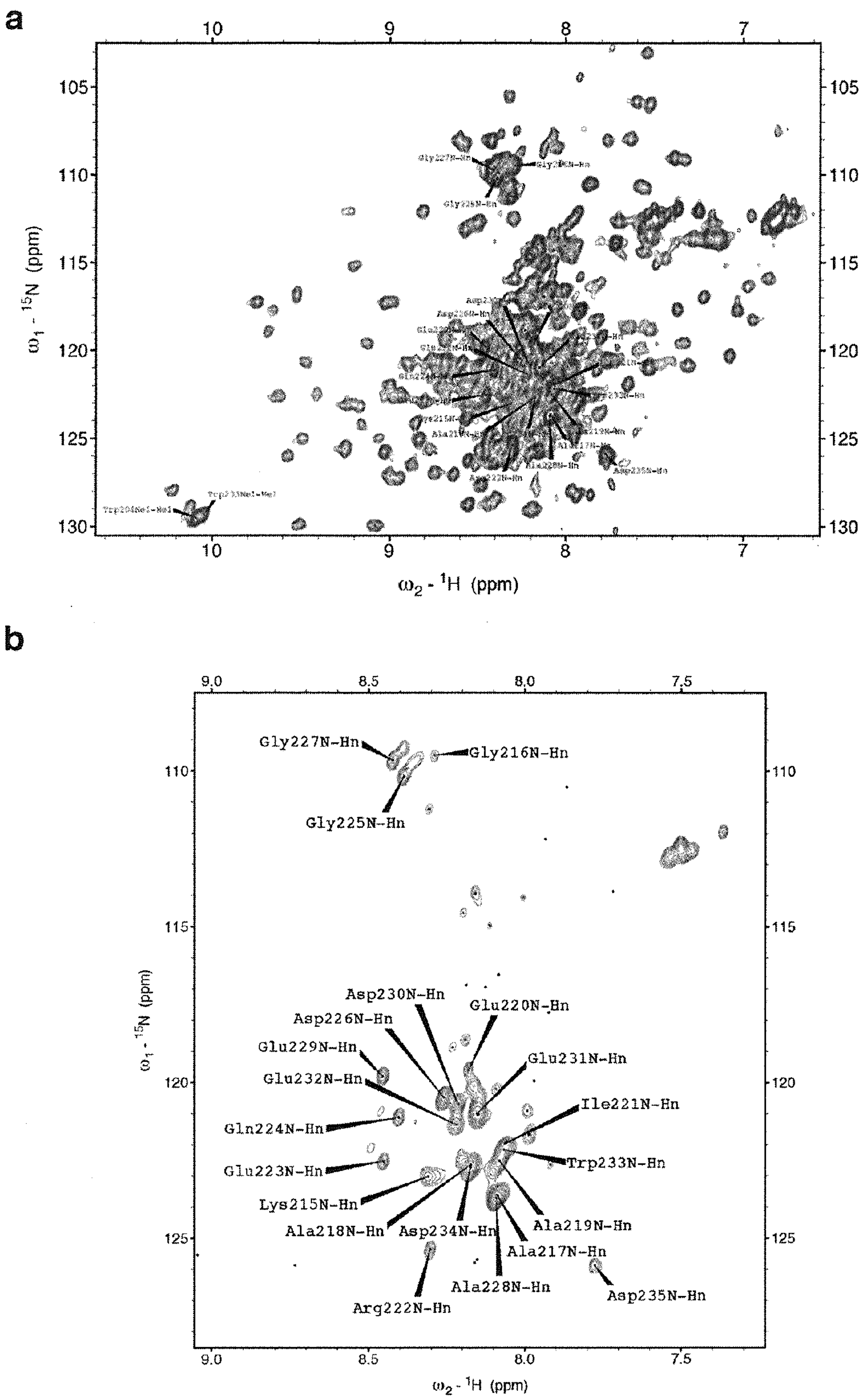
a
ω1 - 15N (ppm)
ω2 - 1H (ppm)
b
Gly227N-Hn
Gly216N-Hn
Gly225N-Hn
Asp230N-Hn
Glu220N-Hn
Asp226N-Hn
Glu229N-Hn
Glu231N-Hn
Glu232N-Hn
Ile221N-Hn
Gln224N-Hn
Trp233N-Hn
Glu223N-Hn
Lys215N-Hn
Ala219N-Hn
Ala218N-Hn
Asp234N-Hn
Ala217N-Hn
Ala228N-Hn
Asp235N-Hn
Arg222N-Hn
ω1 - 15N (ppm)
ω2 - 1H (ppm)

FIGURE 4

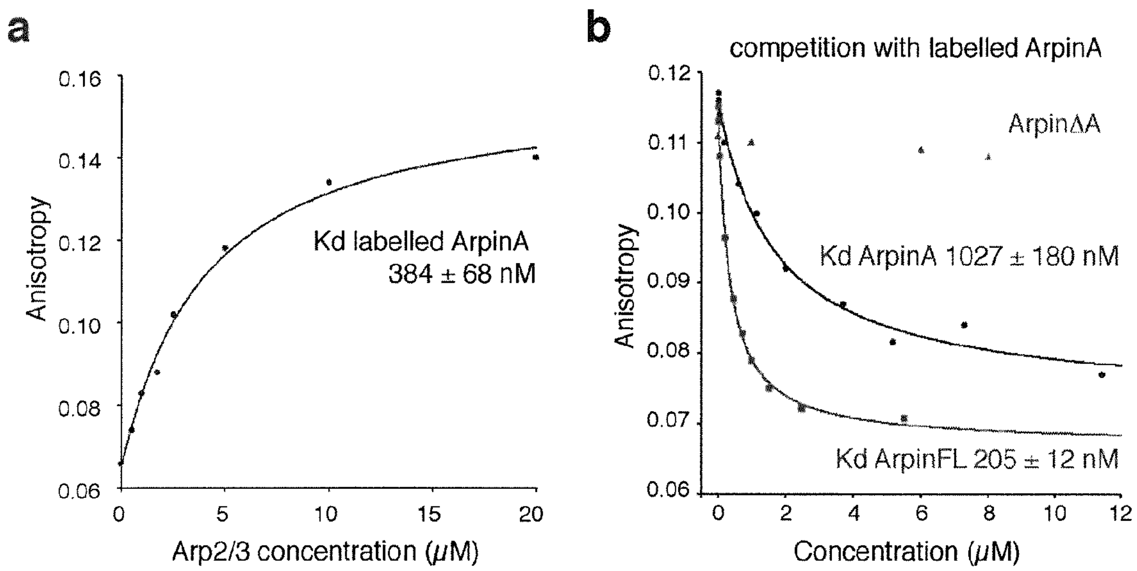
a
0.16
0.14
0.12
0.10
0.08
0.06
Anisotropy
Kd labelled ArpinA
384 ± 68 nM
0
5
10
15
20
Arp2/3 concentration (μM)
b
competition with labelled ArpinA
0.12
0.11
0.10
0.09
0.08
0.07
0.06
Anisotropy
ArpinΔA
Kd ArpinA 1027 ± 180 nM
Kd ArpinFL 205 ± 12 nM
0
2
4
6
8
10
12
Concentration (μM)

FIGURE 5

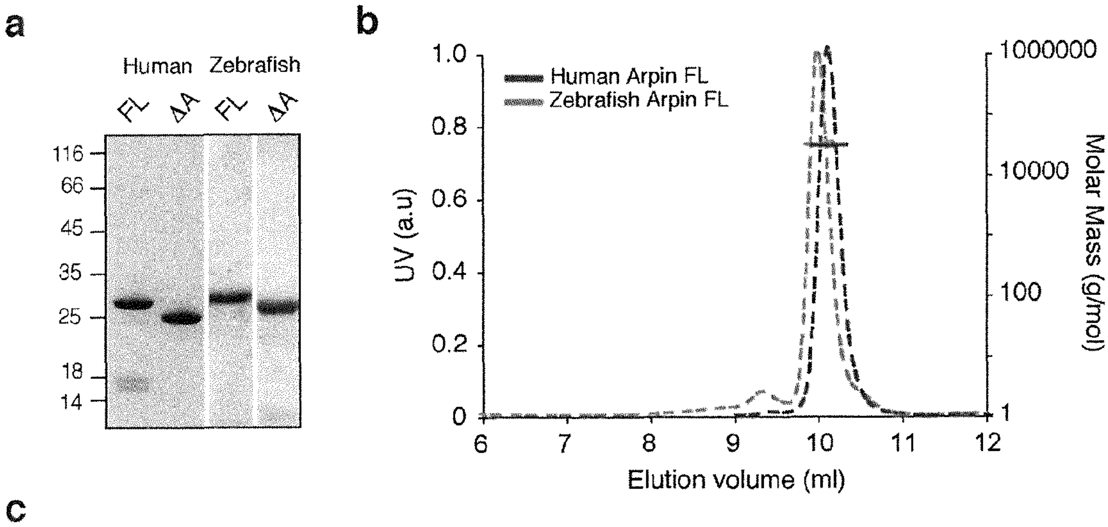


c

| Arpin FL | Theoretical mass (g/mol) | Measured mass (g/mol) | Hydrodynamic radius (nm) |
|---|---|---|---|
| Human | 25852.1 | 24860 ± 50 | 2.1 ± 0.21 |
| Zebrafish | 26454.6 | 26620 ± 100 | 2.5 ± 0.18 |

**FIGURE 6**

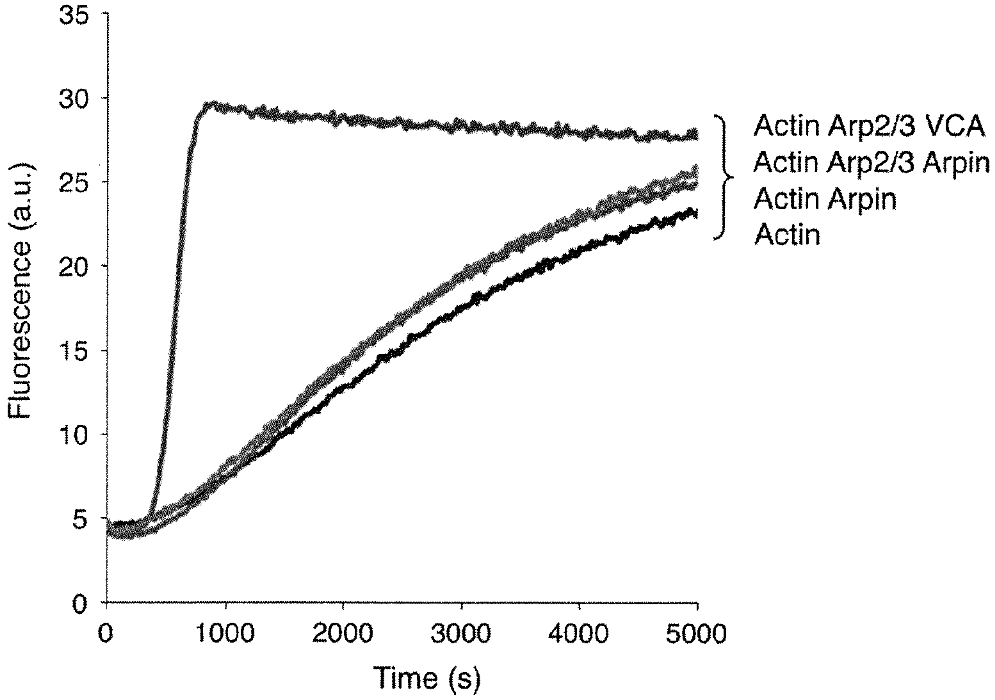
35
30
25
20
15
10
5
0
Fluorescence (a.u.)
0
1000
2000
3000
4000
5000
Time (s)
Actin Arp2/3 VCA
Actin Arp2/3 Arpin
Actin Arpin
Actin

**FIGURE 7**

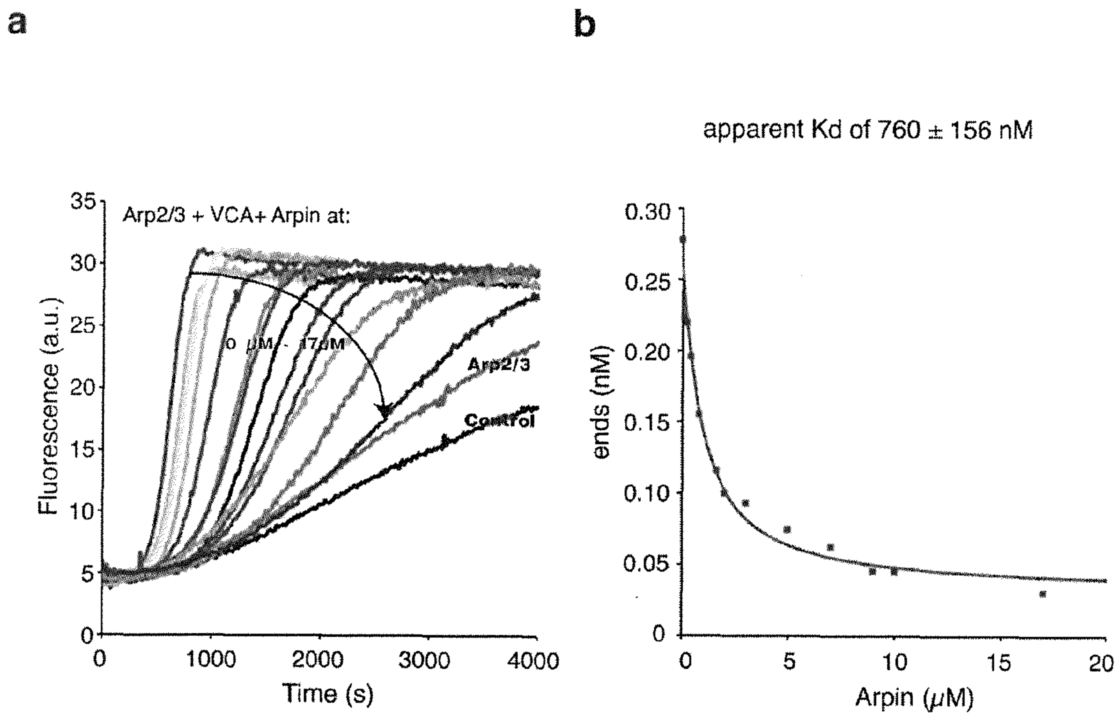
a
b
apparent Kd of 760 ± 156 nM
Arp2/3 + VCA+ Arpin at:
Arp2/3
Control
35
30
25
20
15
10
5
0
Fluorescence (a.u.)
0
1000
2000
3000
4000
Time (s)
0.30
0.25
0.20
0.15
0.10
0.05
0
ends (nM)
0
5
10
15
20
Arpin (μM)

FIGURE 8

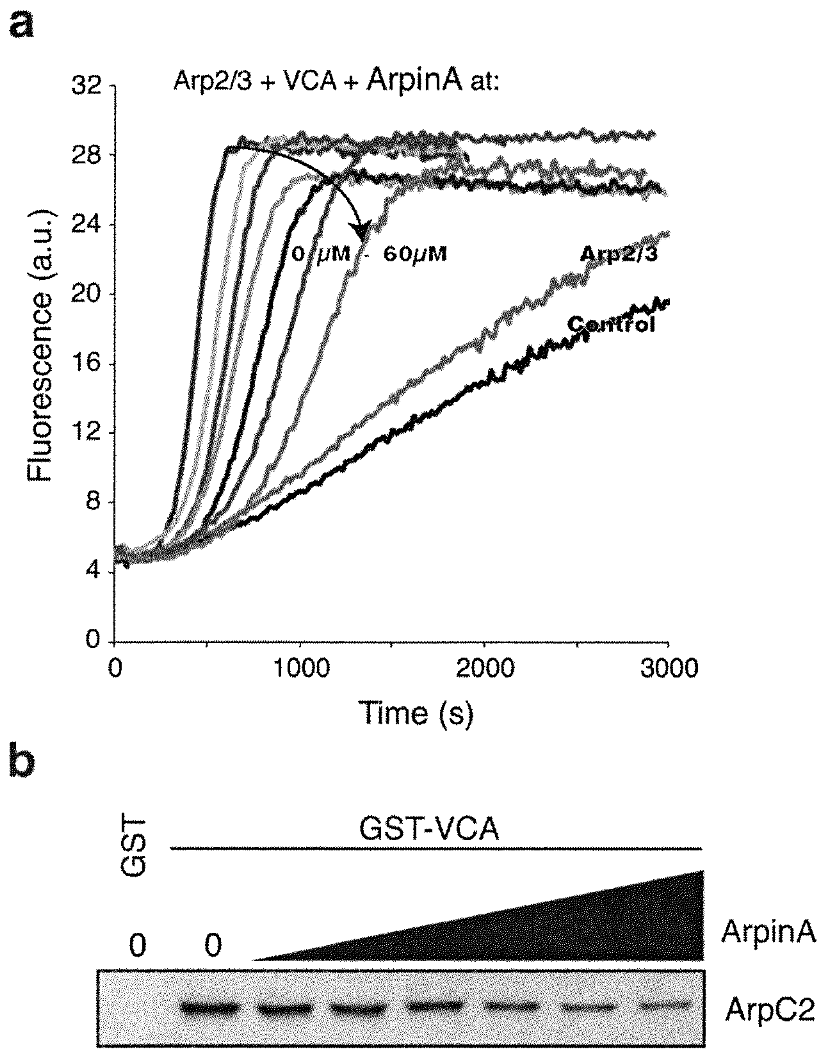
a
Arp2/3 + VCA + ArpinA at:
32
28
24
20
16
12
8
4
0
Fluorescence (a.u.)
0 μM - 60μM
Arp2/3
Control
0
1000
2000
3000
Time (s)
b
GST
GST-VCA
0
0
ArpinA
ArpC2

FIGURE 9

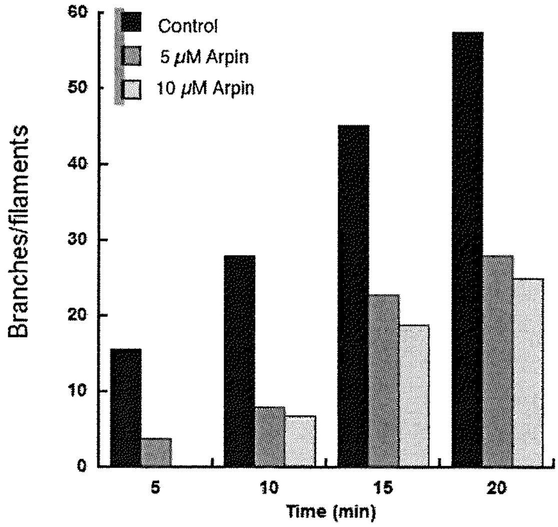
Control
5 μM Arpin
10 μM Arpin
Branches/filaments
60
50
40
30
20
10
0
5
10
15
20
Time (min)

FIGURE 10

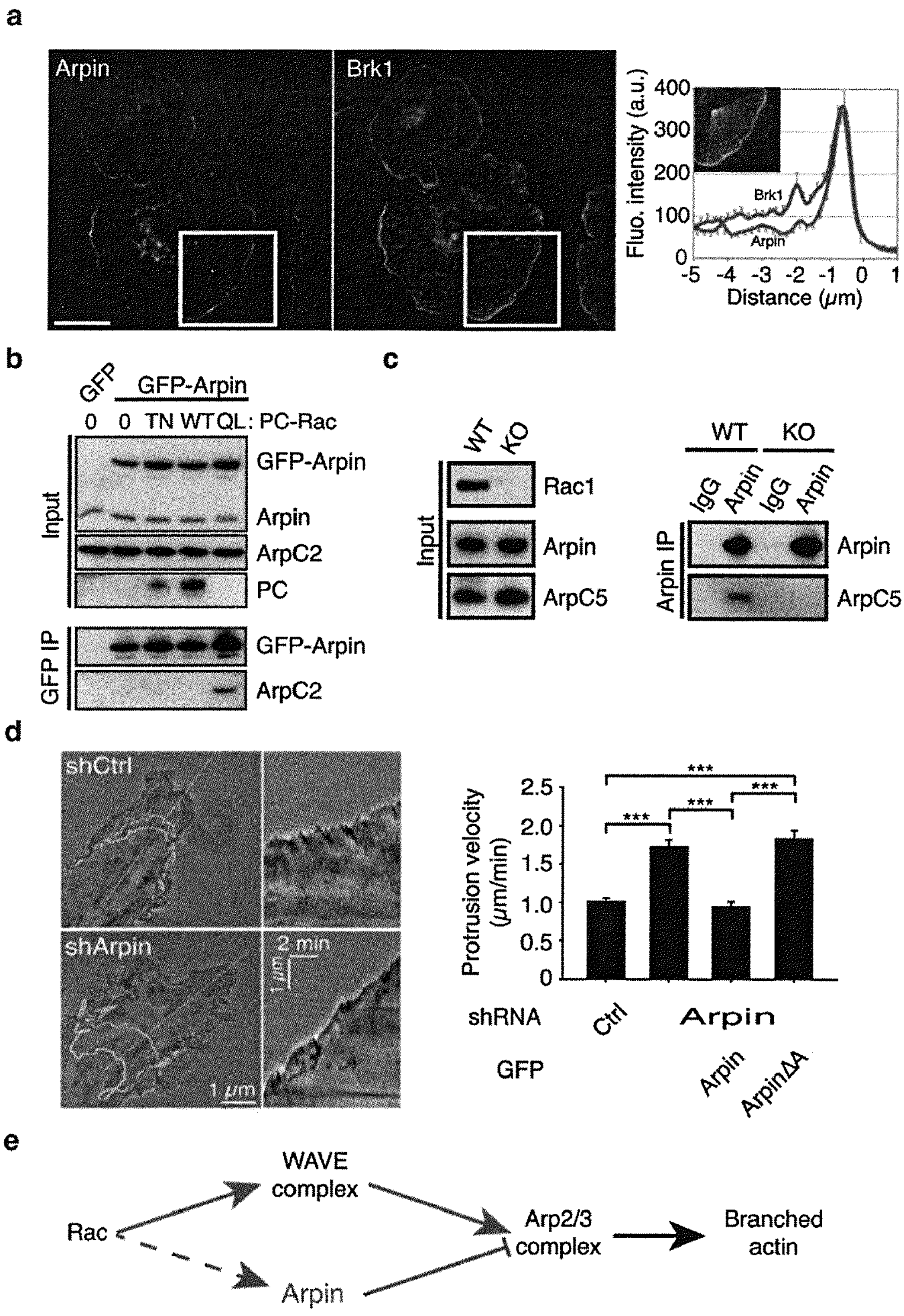
a
Arpin
Brk1
Fluo. intensity (a.u.)
400
300
200
100
0
Brk1
Arpin
-5 -4 -3 -2 -1 0 1
Distance (μm)
b
GFP
GFP-Arpin
0 0 TN WT QL: PC-Rac
Input
GFP-Arpin
Arpin
ArpC2
PC
GFP IP
GFP-Arpin
ArpC2
c
WT KO
Input
Rac1
Arpin
ArpC5
WT KO
IgG Arpin IgG Arpin
Arpin IP
Arpin
ArpC5
d
shCtrl
shArpin
2 min
1 μm
1 μm
Protrusion velocity (μm/min)
2.5
2.0
1.5
1.0
0.5
0
***
shRNA
Ctrl
Arpin
GFP
Arpin
ArpinΔA
e
Rac
WAVE complex
Arpin
Arp2/3 complex
Branched actin

FIGURE 11

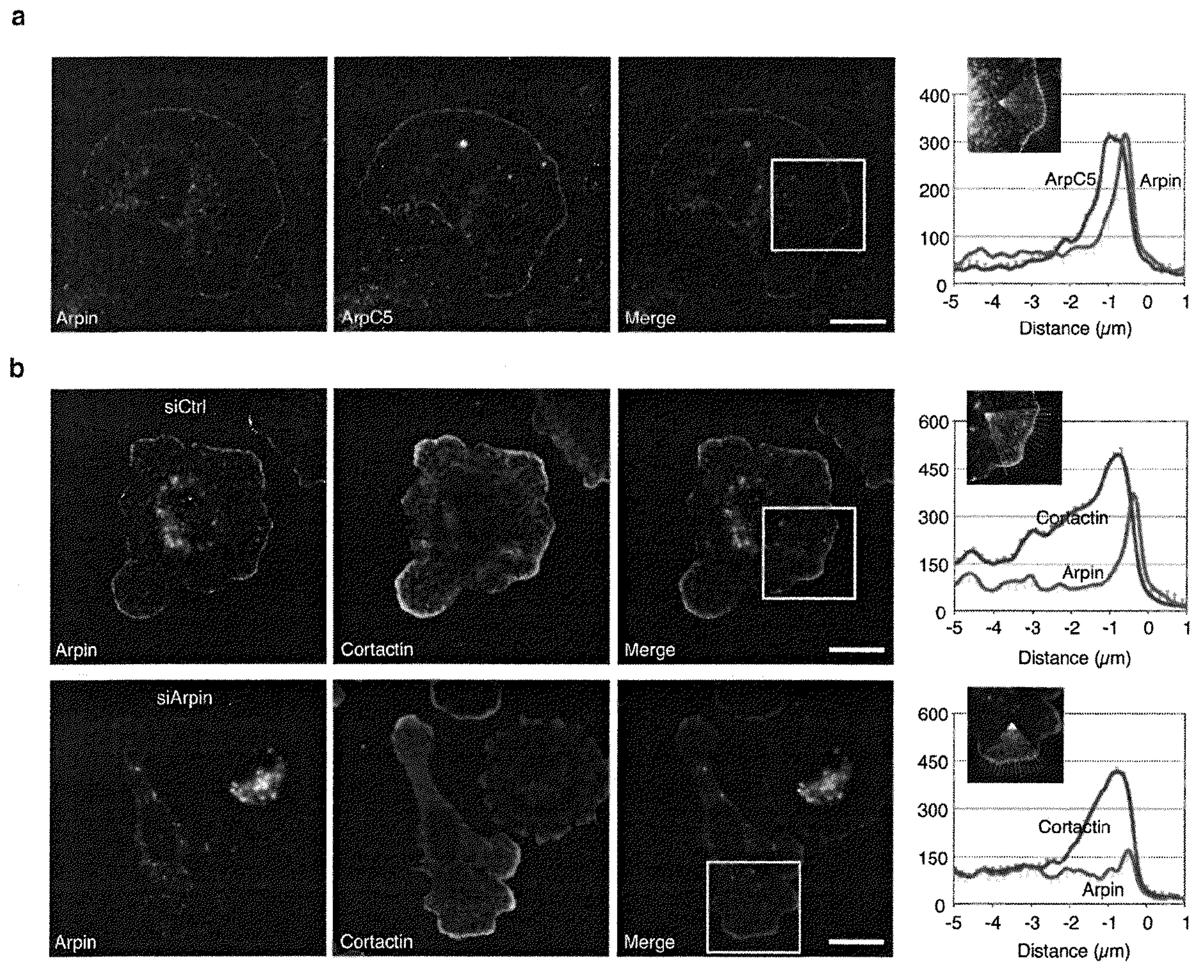
a
Arpin
ArpC5
Merge
400
300
200
100
0
ArpC5
Arpin
-5 -4 -3 -2 -1 0 1
Distance (μm)
b
siCtrl
Arpin
Cortactin
Merge
600
450
300
150
0
Cortactin
Arpin
-5 -4 -3 -2 -1 0 1
Distance (μm)
siArpin
Arpin
Cortactin
Merge
600
450
300
150
0
Cortactin
Arpin
-5 -4 -3 -2 -1 0 1
Distance (μm)

FIGURE 12

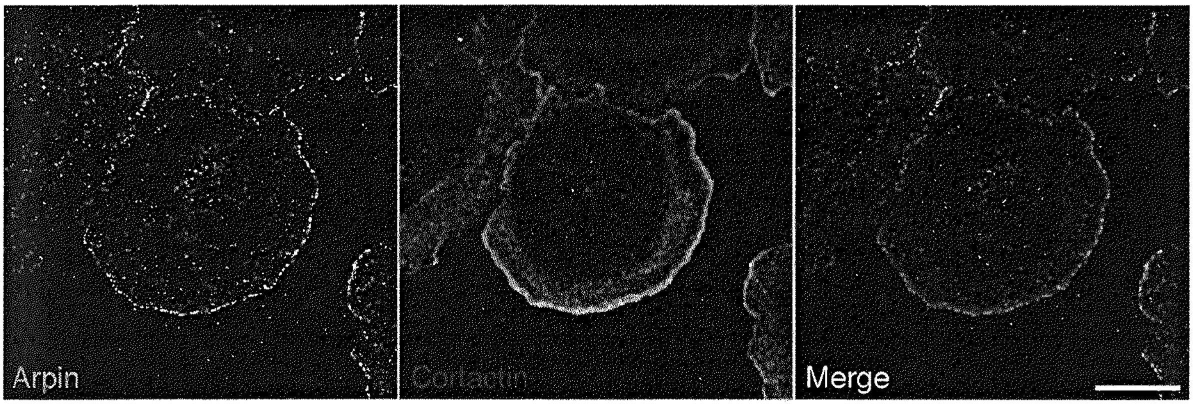


FIGURE 13

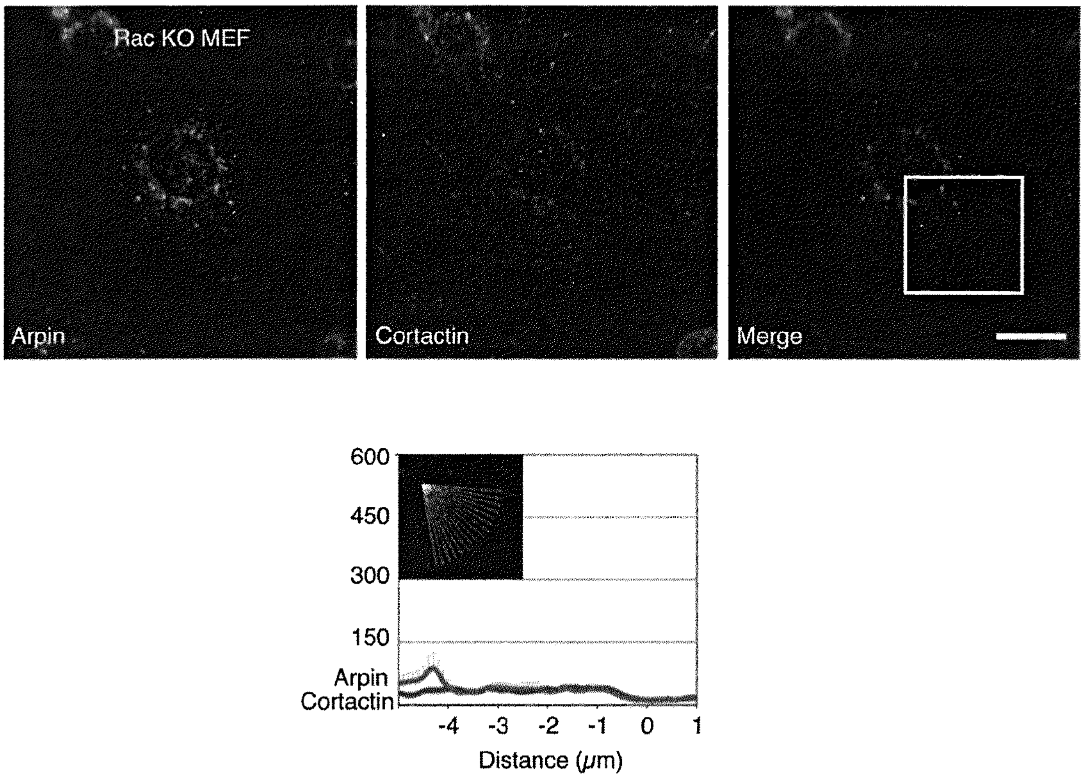
Rac KO MEF
Arpin
Cortactin
Merge
600
450
300
150
Arpin
Cortactin
-4 -3 -2 -1 0 1
Distance (μm)

**FIGURE 14**

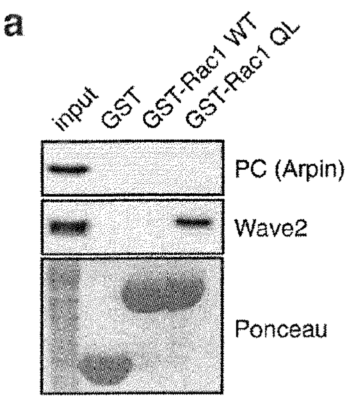


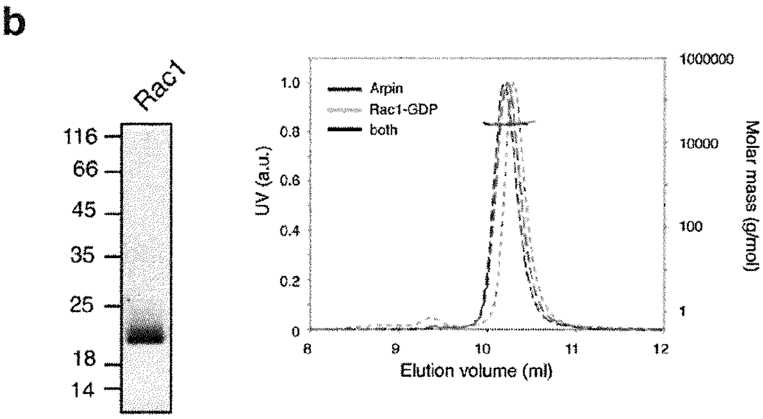


| | Theoretical mass (g/mol) | Measured mass (g/mol) |
|---|---|---|
| Arpin | 25852 | 26290 ± 140 |
| Rac1-GDP | 21893 | 28180 ± 170 |
| Arpin:Rac1-GDP | 47745 | 26970 ± 160 |
| Rac1-GTPγS | 21989 | 28390 ± 150 |
| Arpin:Rac1-GTPγS | 47841 | 26760 ± 110 |

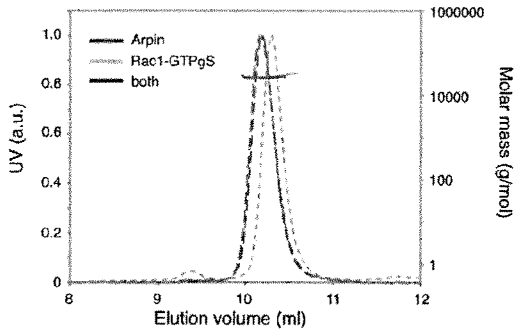


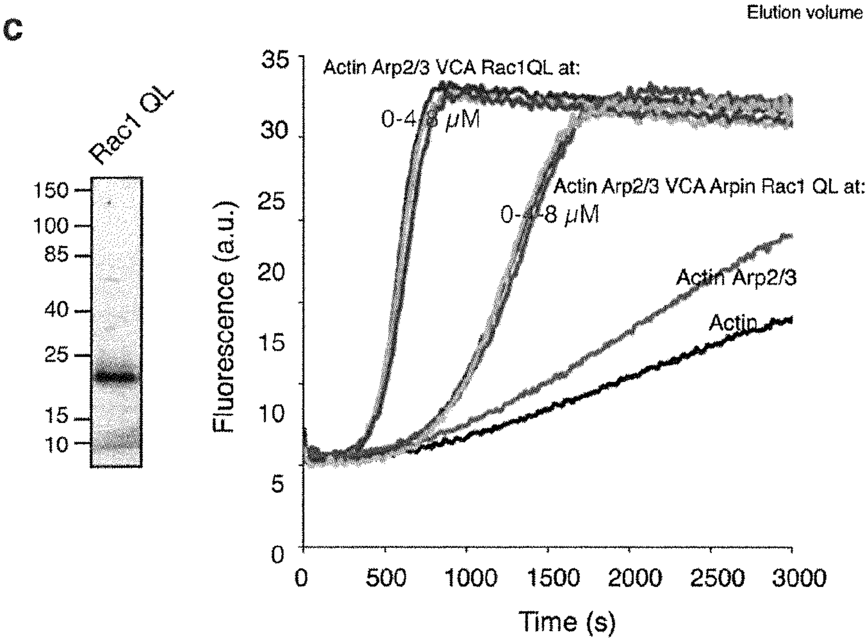


FIGURE 15

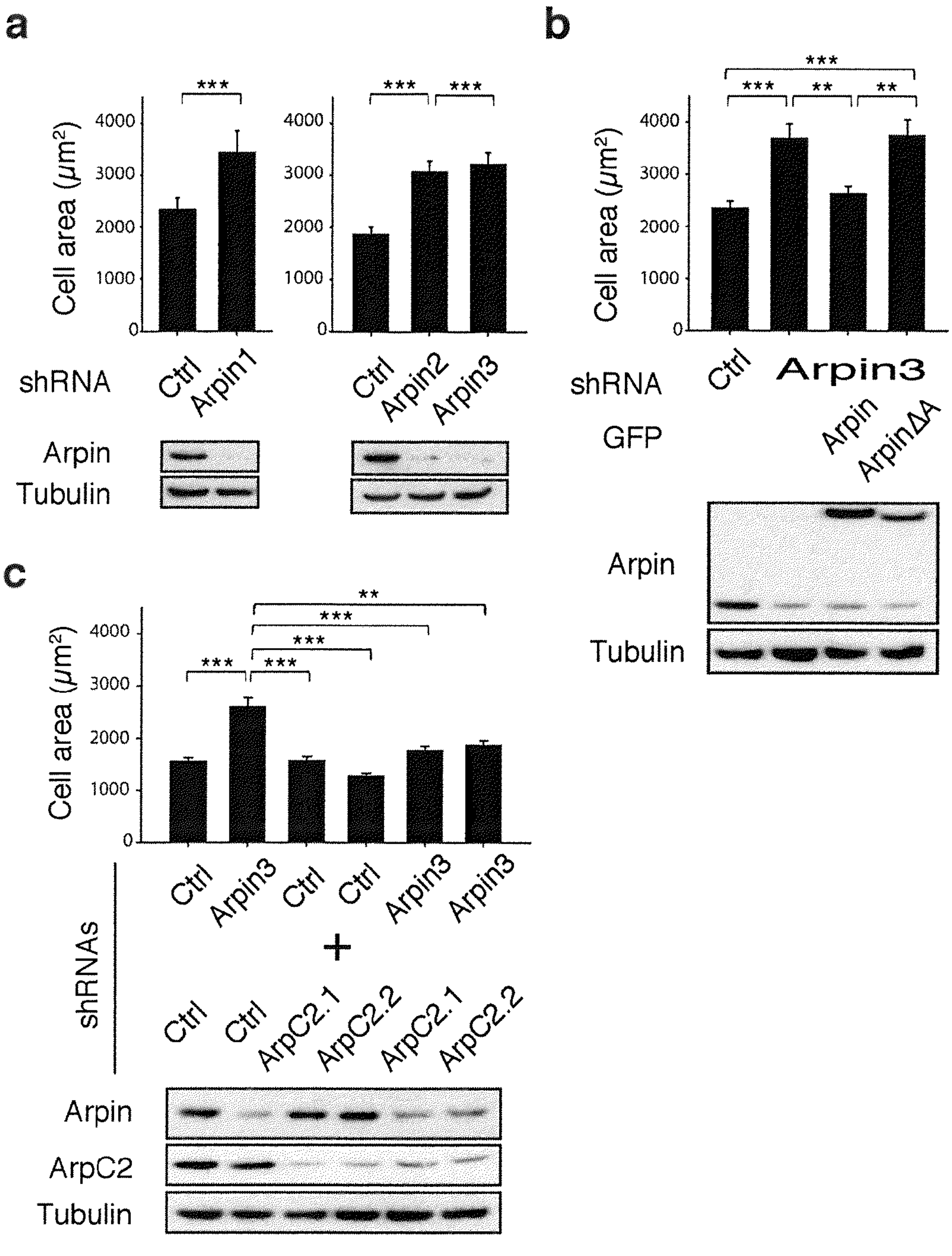
a
b
c
***
***
***
***
**
**
Cell area (μm²)
4000
3000
2000
1000
0
shRNA
Ctrl
Arpin1
Arpin2
Arpin3
GFP
Arpin
ArpinΔA
Tubulin
shRNAs
+
ArpC2.1
ArpC2.2
ArpC2

FIGURE 16

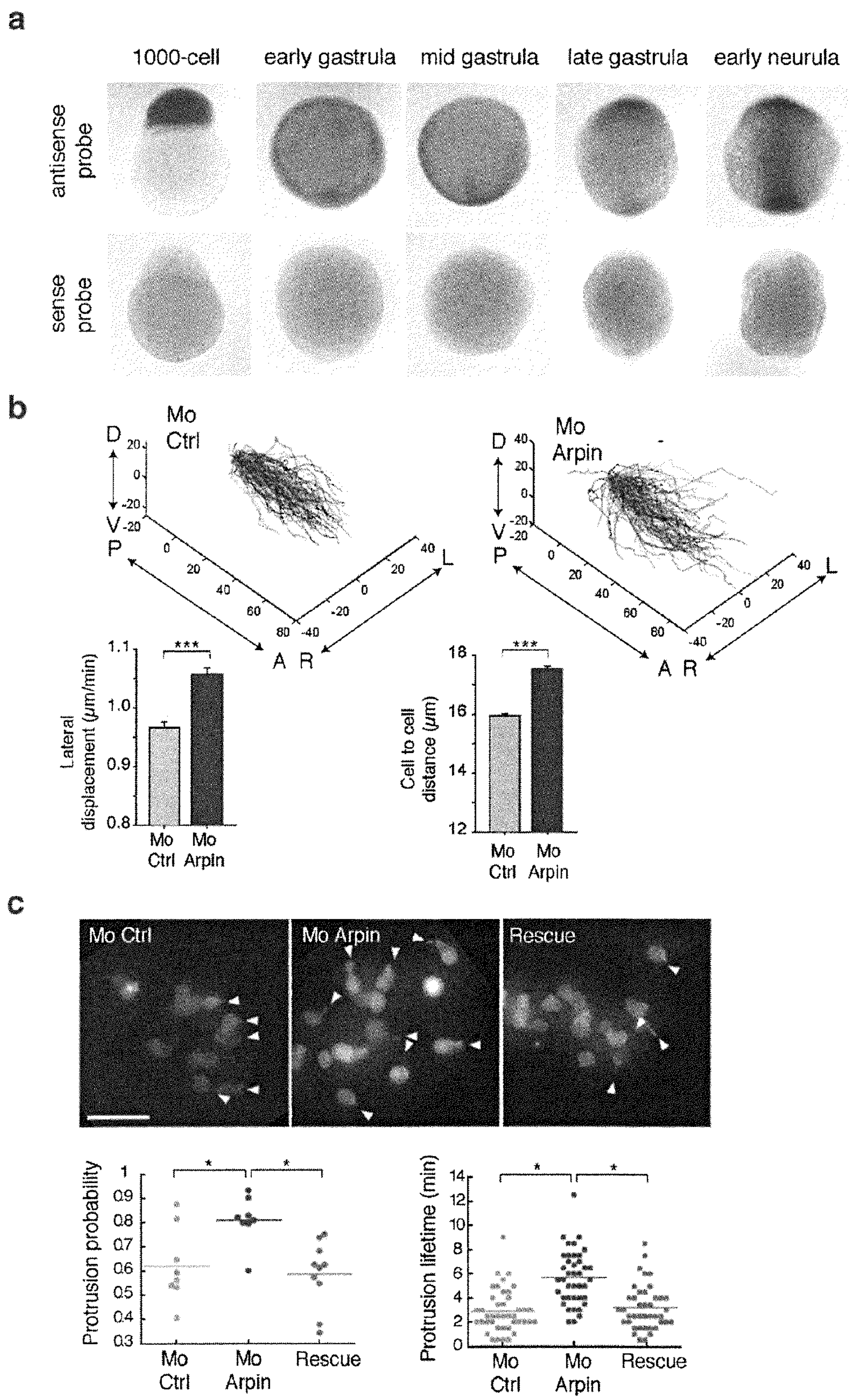
a
1000-cell
early gastrula
mid gastrula
late gastrula
early neurula
antisense probe
sense probe
b
Mo Ctrl
Mo Arpin
D
V
P
A
R
L
Lateral displacement (µm/min)
Cell to cell distance (µm)
c
Mo Ctrl
Mo Arpin
Rescue
Protrusion probability
Protrusion lifetime (min)

FIGURE 17

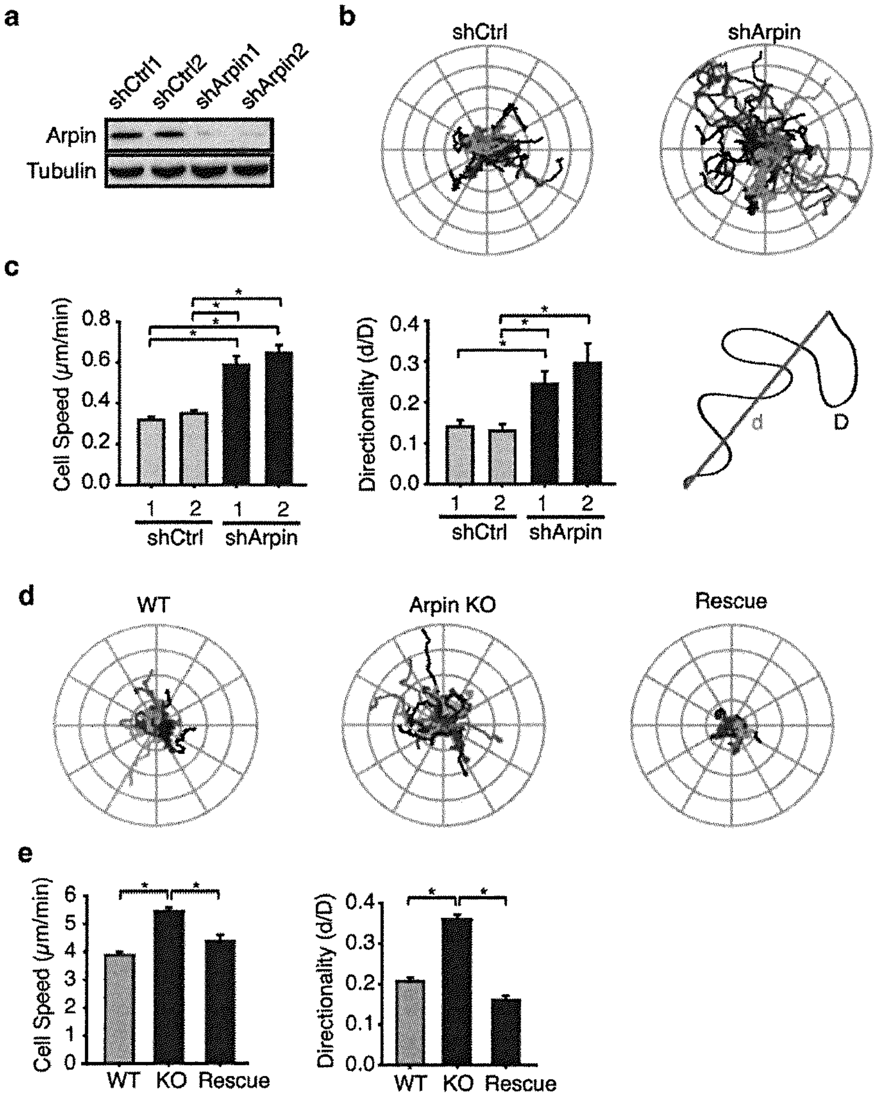
a
shCtrl1
shCtrl2
shArpin1
shArpin2
Arpin
Tubulin
b
shCtrl
shArpin
c
Cell Speed (μm/min)
0.8
0.6
0.4
0.2
0.0
1 2 1 2
shCtrl shArpin
Directionality (d/D)
0.4
0.3
0.2
0.1
0.0
1 2 1 2
shCtrl shArpin
d
D
d
WT
Arpin KO
Rescue
e
Cell Speed (μm/min)
6
5
4
3
2
1
0
WT KO Rescue
Directionality (d/D)
0.4
0.3
0.2
0.1
0.0
WT KO Rescue

FIGURE 18

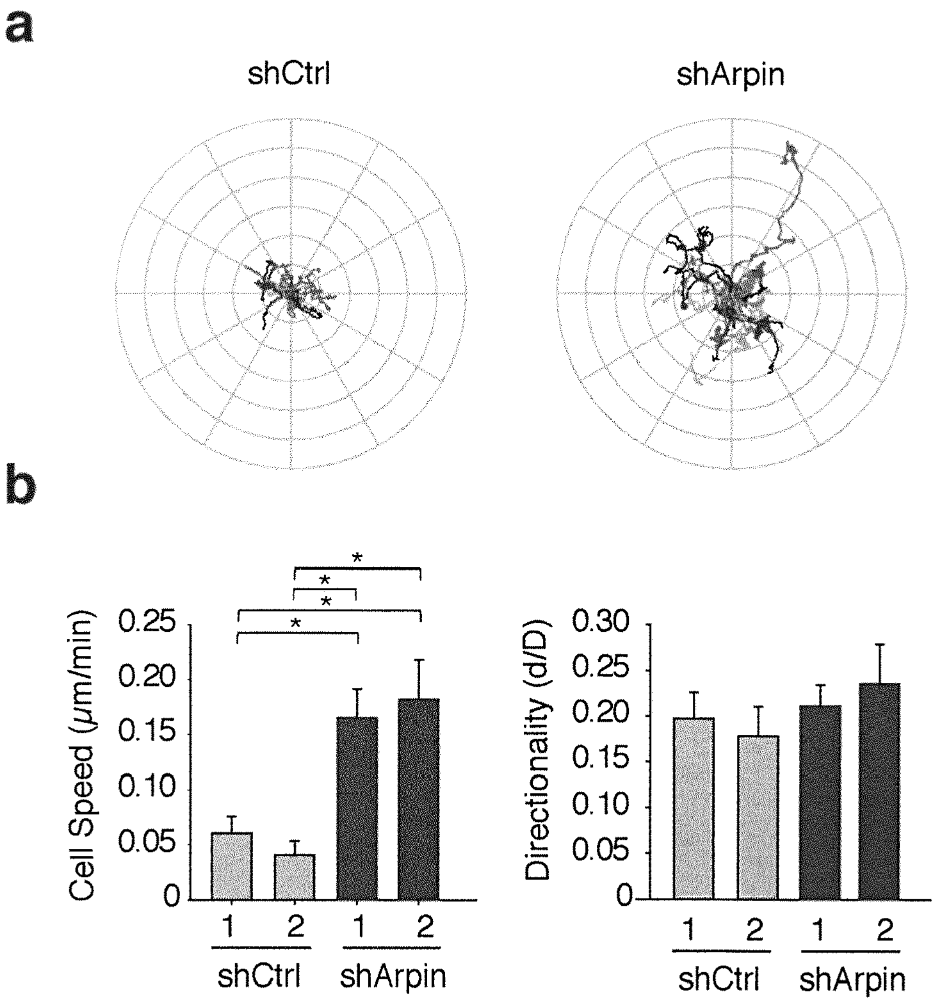
a
shCtrl
shArpin
b
Cell Speed (μm/min)
0.25
0.20
0.15
0.10
0.05
0
*
1
2
1
2
shCtrl
shArpin
Directionality (d/D)
0.30
0.25
0.20
0.15
0.10
0.05
0
1
2
1
2
shCtrl
shArpin

**FIGURE 19**

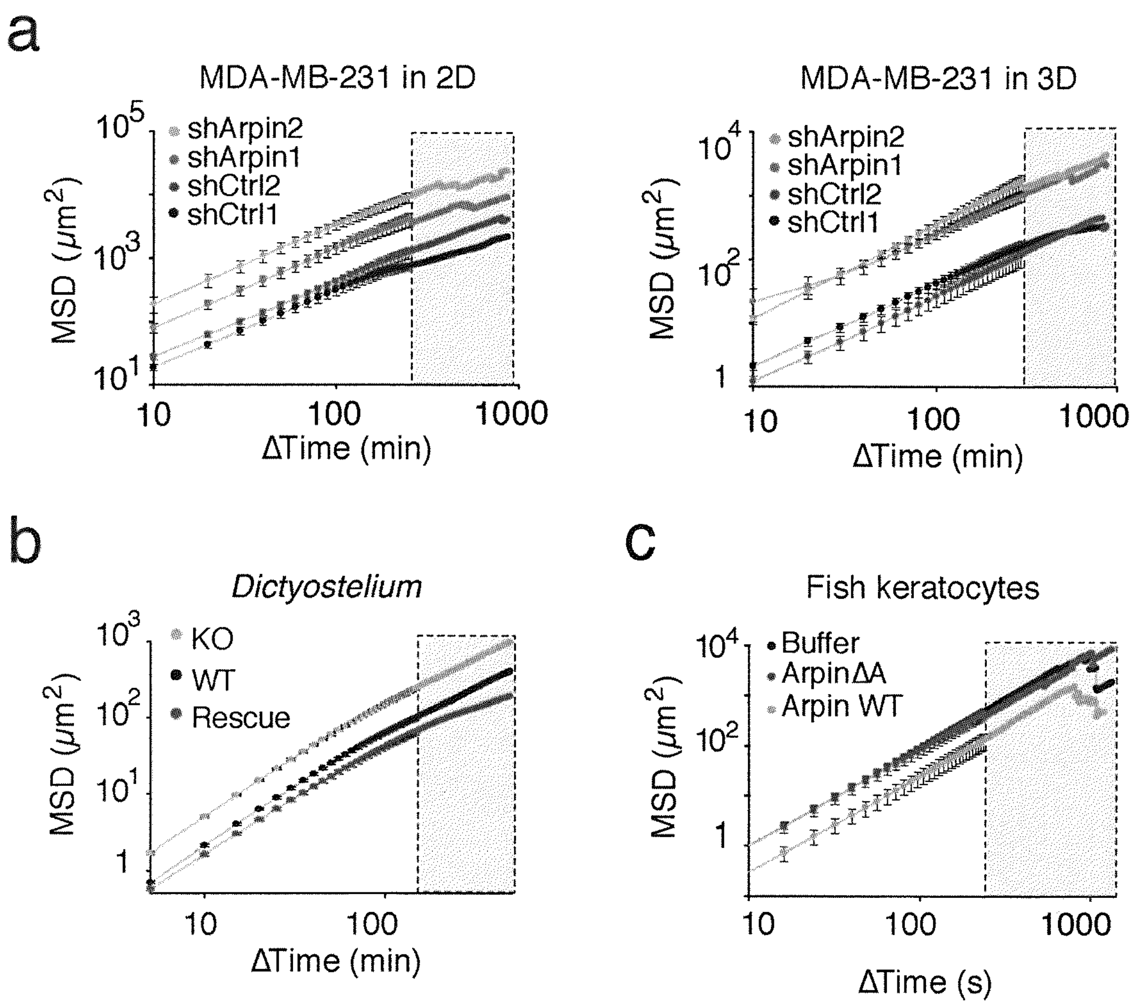
a
MDA-MB-231 in 2D
shArpin2
shArpin1
shCtrl2
shCtrl1
MSD ($\mu m^2$)
$10^5$
$10^3$
$10^1$
10
100
1000
ΔTime (min)
MDA-MB-231 in 3D
shArpin2
shArpin1
shCtrl2
shCtrl1
MSD ($\mu m^2$)
$10^4$
$10^2$
1
10
100
1000
ΔTime (min)
b
Dictyostelium
KO
WT
Rescue
MSD ($\mu m^2$)
$10^3$
$10^2$
$10^1$
1
10
100
ΔTime (min)
c
Fish keratocytes
Buffer
ArpinΔA
Arpin WT
MSD ($\mu m^2$)
$10^4$
$10^2$
1
10
100
1000
ΔTime (s)

FIGURE 20

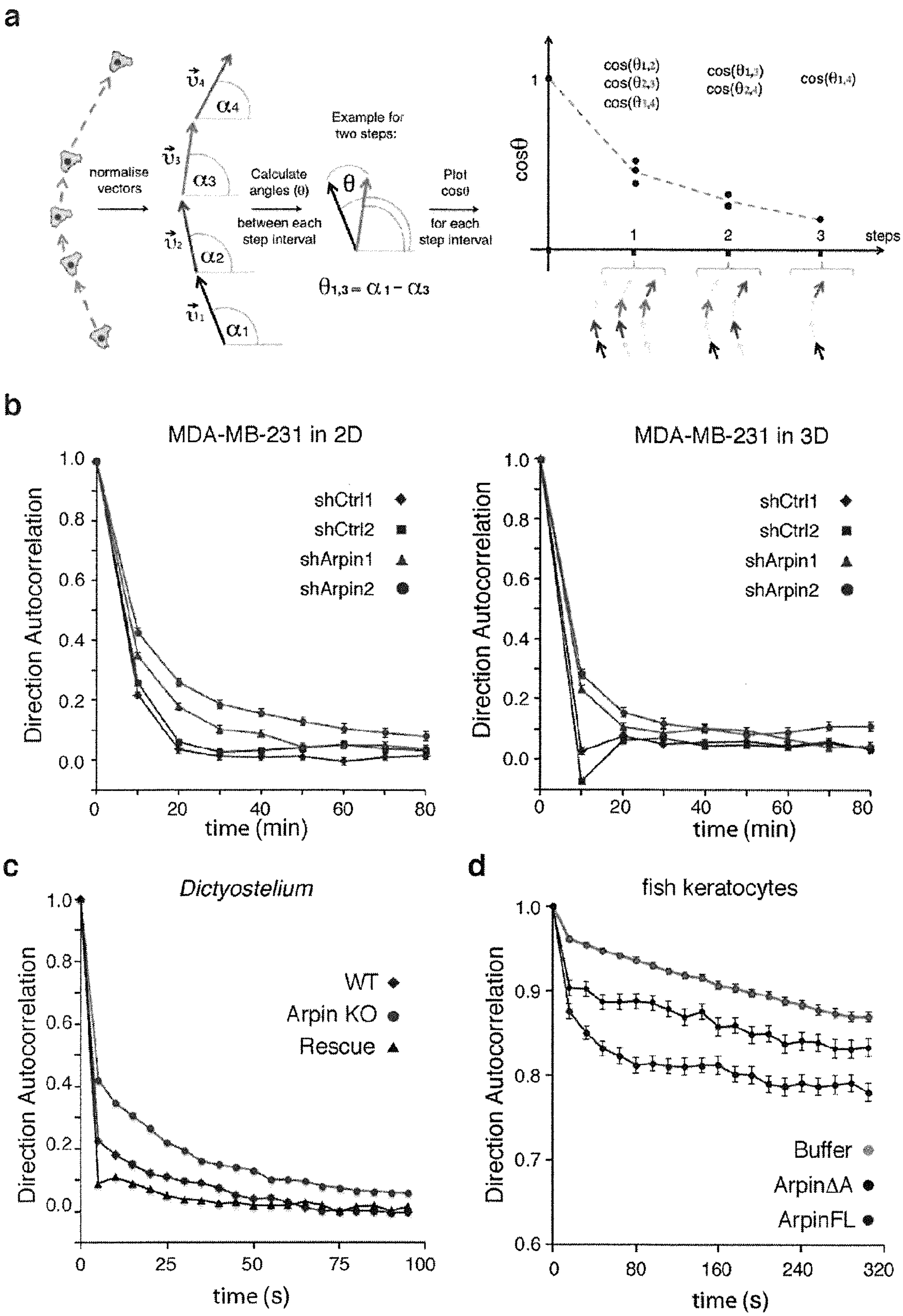
a
normalise vectors
Calculate angles (θ) between each step interval
Example for two steps:
$\theta_{1,3} = \alpha_1 - \alpha_3$
Plot cosθ for each step interval
cosθ
steps
b
MDA-MB-231 in 2D
MDA-MB-231 in 3D
shCtrl1
shCtrl2
shArpin1
shArpin2
Direction Autocorrelation
time (min)
c
Dictyostelium
WT
Arpin KO
Rescue
time (s)
d
fish keratocytes
Buffer
ArpinΔA
ArpinFL

**FIGURE 21**

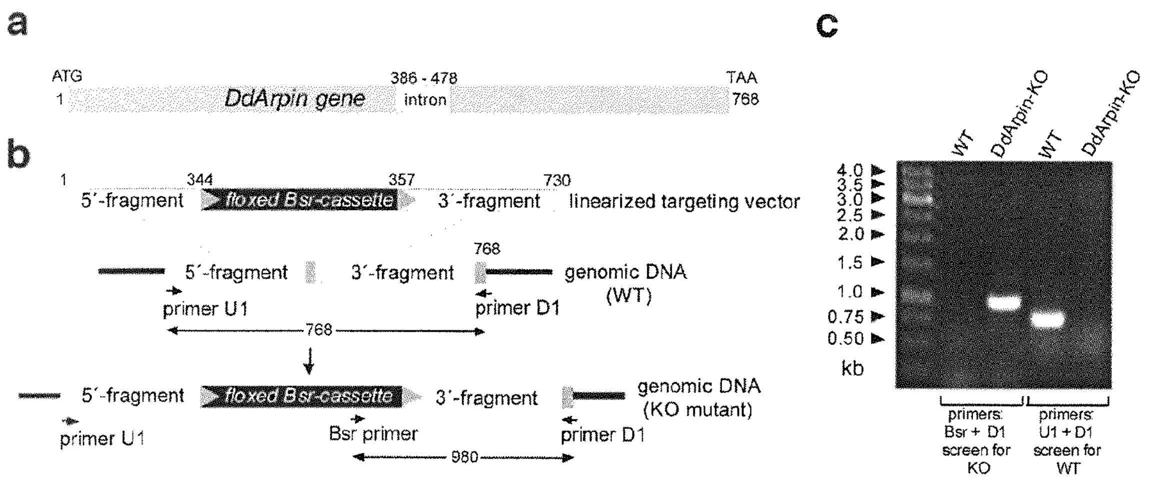
a
ATG
1
DdArpin gene
386 - 478
intron
TAA
768
b
1
344
357
730
5´-fragment
floxed Bsr-cassette
3´-fragment
linearized targeting vector
768
5´-fragment
3´-fragment
genomic DNA
(WT)
primer U1
primer D1
768
5´-fragment
floxed Bsr-cassette
3´-fragment
genomic DNA
(KO mutant)
primer U1
Bsr primer
primer D1
980
c
WT
DdArpin-KO
WT
DdArpin-KO
4.0
3.5
3.0
2.5
2.0
1.5
1.0
0.75
0.50
kb
primers: Bsr + D1 screen for KO
primers: U1 + D1 screen for WT

FIGURE 22

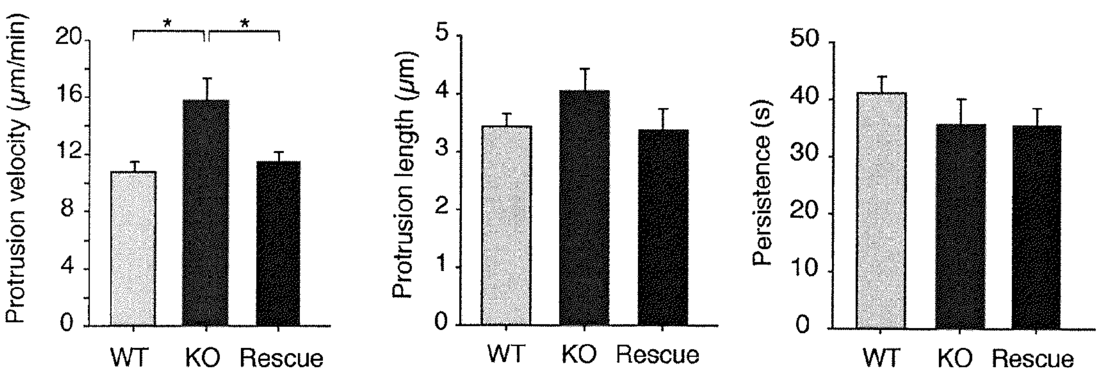
Protrusion velocity (μm/min)
20
16
12
8
4
0
*
*
WT
KO
Rescue
Protrusion length (μm)
5
4
3
2
1
0
WT
KO
Rescue
Persistence (s)
50
40
30
20
10
0
WT
KO
Rescue

FIGURE 23

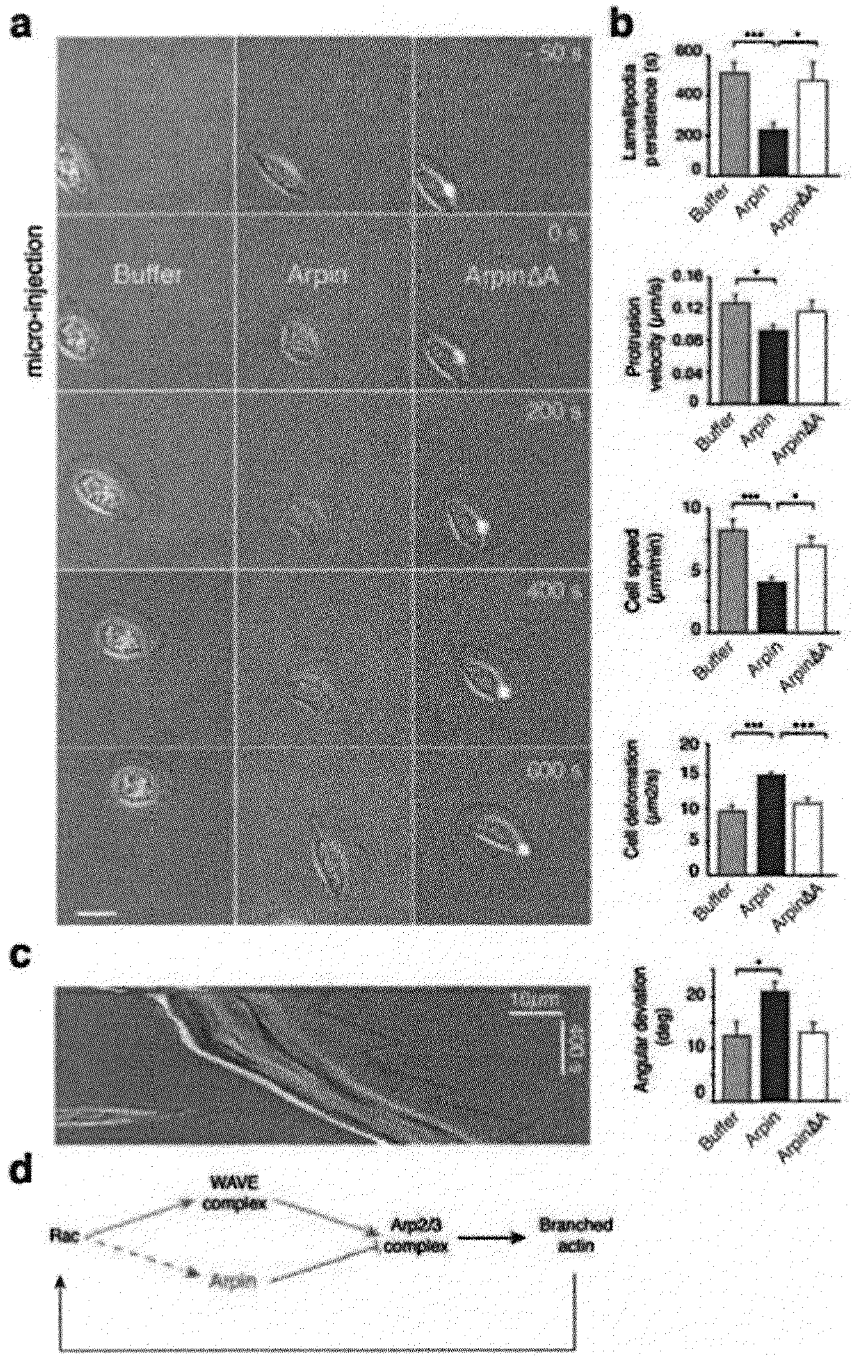
a
micro-injection
-50 s
0 s
Buffer
Arpin
ArpinΔA
200 s
400 s
600 s
b
Lamellipodia persistence (s)
Protrusion velocity (µm/s)
Cell speed (µm/min)
Cell deformation (µm2/s)
Angular deviation (deg)
Buffer
Arpin
ArpinΔA
c
10µm
100 s
d
WAVE complex
Rac
Arpin
Arp2/3 complex
Branched actin

FIGURE 24

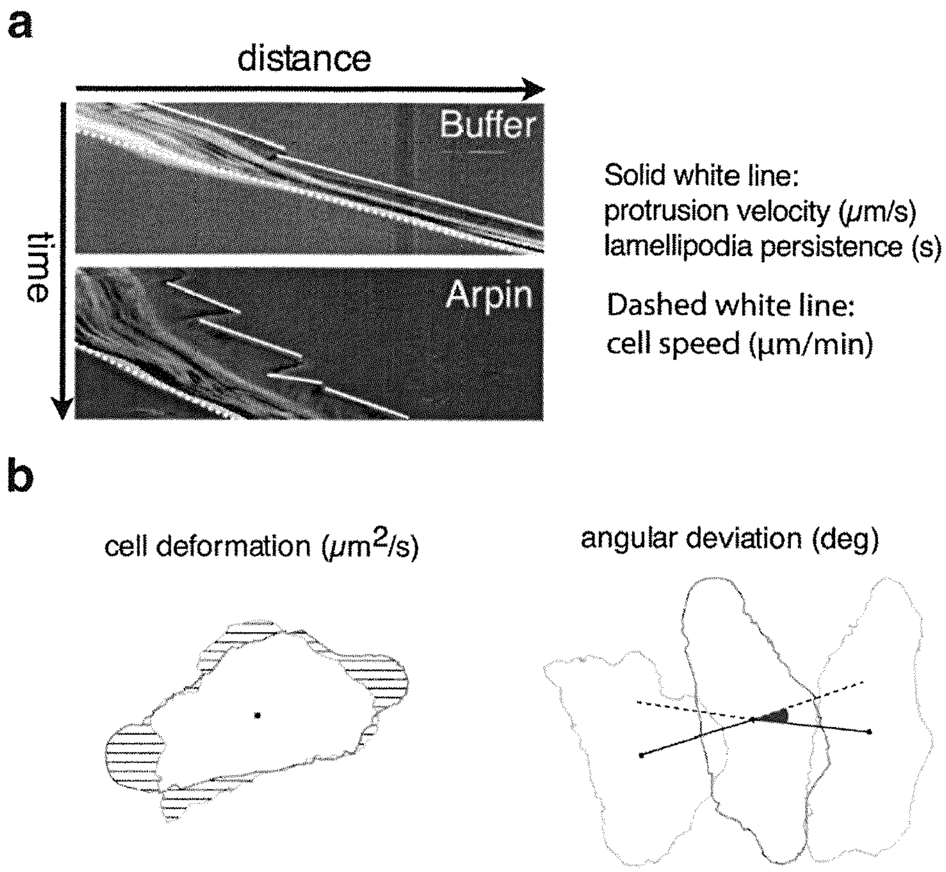
a
distance
time
Buffer
Arpin
Solid white line:
protrusion velocity (μm/s)
lamellipodia persistence (s)
Dashed white line:
cell speed (μm/min)
b
cell deformation (μm2/s)
angular deviation (deg)

FIGURE 25

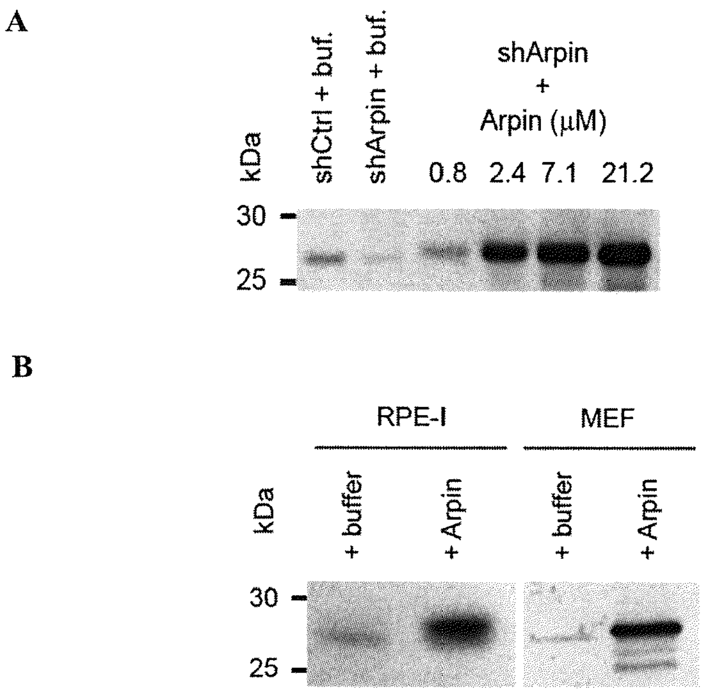
A
kDa
shCtrl + buf.
shArpin + buf.
shArpin
+
Arpin (μM)
0.8
2.4
7.1
21.2
30
25
B
RPE-I
MEF
kDa
+ buffer
+ Arpin
+ buffer
+ Arpin
30
25

FIGURE 26

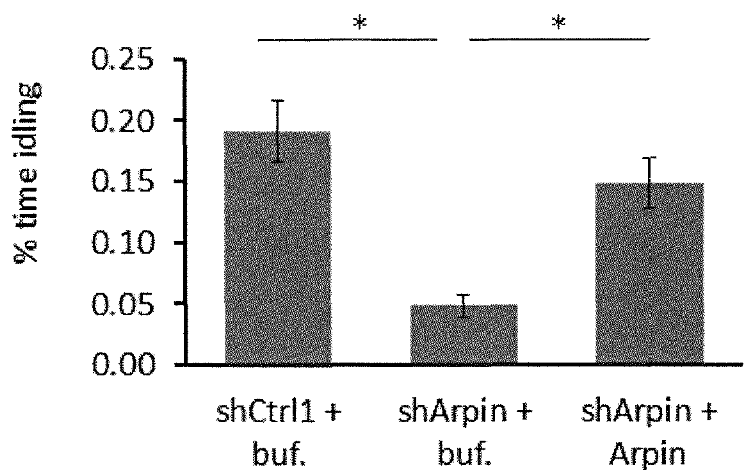
*
*
% time idling
0.25
0.20
0.15
0.10
0.05
0.00
shCtrl1 + buf.
shArpin + buf.
shArpin + Arpin

FIGURE 27

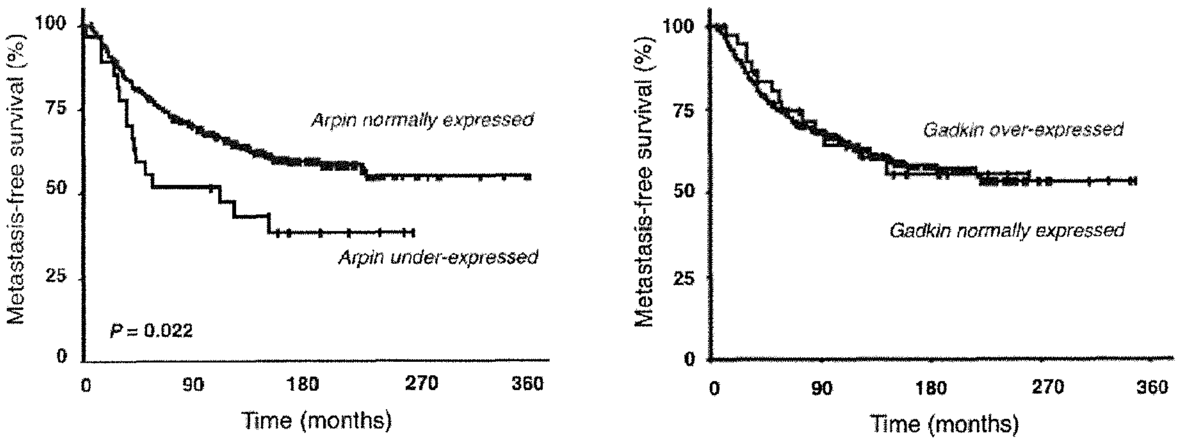

Metastasis-free survival (%)
100
75
50
25
0
Arpin normally expressed
Arpin under-expressed
P = 0.022
0
90
180
270
360
Time (months)
Metastasis-free survival (%)
100
75
50
25
0
Gadkin over-expressed
Gadkin normally expressed
0
90
180
270
360
Time (months)


FIGURE 28

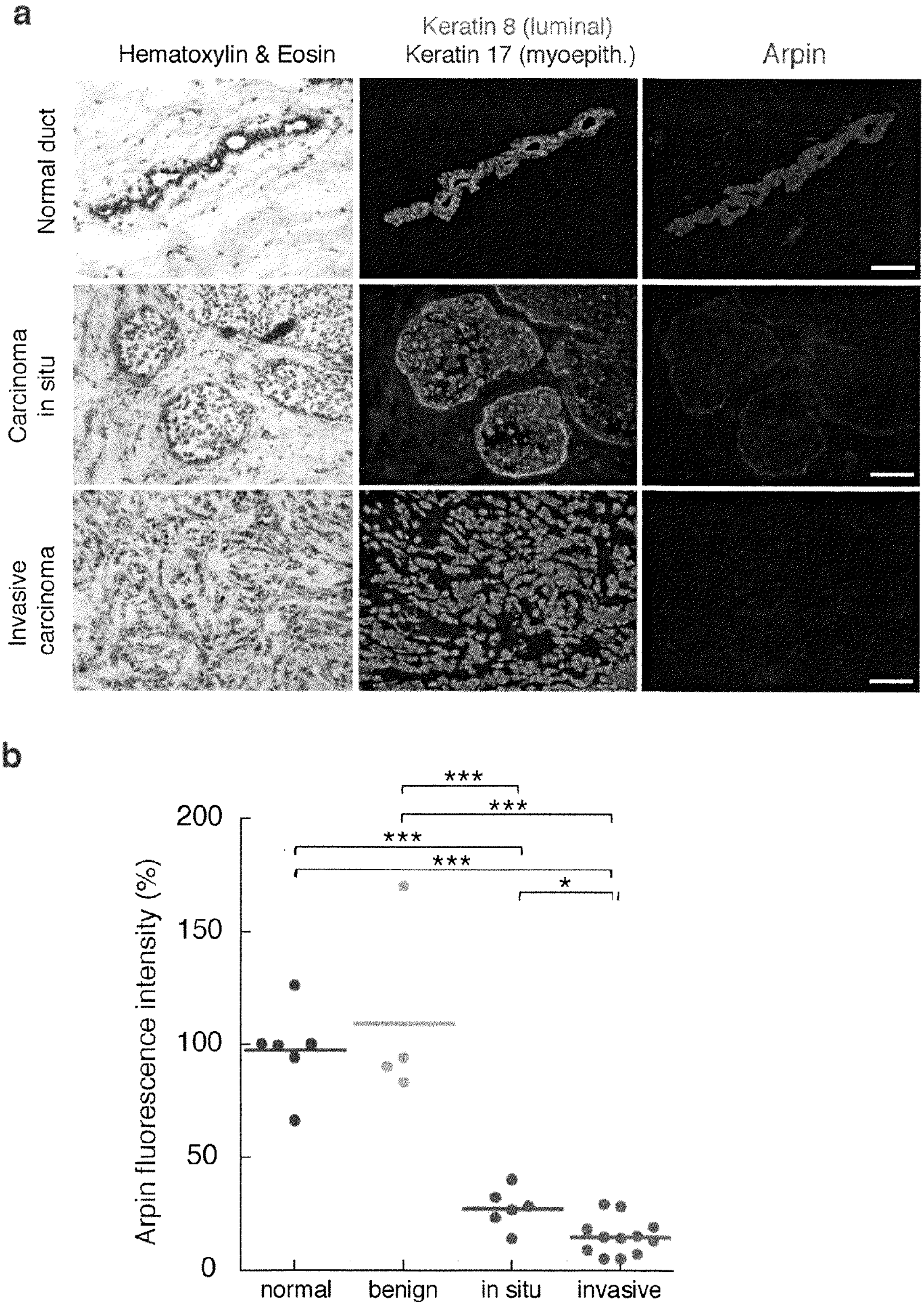
a
Keratin 8 (luminal)
Hematoxylin & Eosin
Keratin 17 (myoepith.)
Arpin
Normal duct
Carcinoma in situ
Invasive carcinoma
b
***
***
***
***
*
200
150
100
50
0
Arpin fluorescence intensity (%)
normal
benign
in situ
invasive

FIGURE 29

Europäisches Patentamt
European Patent Office
Office européen des brevets

# EUROPEAN SEARCH REPORT

Application Number
EP 13 30 6198

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | WO 2010/053772 A2 (UNIV CALIFORNIA [US]; FONG LAWRENCE H [US]; MACPHEE SERENA KWEK [US]) 14 May 2010 (2010-05-14)<br>* paragraph [0072]; claims 1,2,9; sequence 24 *<br>----- | 1-7,12 | INV.<br>A61K38/17<br>C07K14/47<br>G01N33/50<br>A61P25/00 |
| X | WO 03/046152 A2 (INCYTE GENOMICS INC [US]; TANG Y TOM [US]; CHAWLA NARINDER K [US]; LU) 5 June 2003 (2003-06-05)<br>* claims 1, 3, 11, 17-19, 20,23; sequence 16 *<br>----- | 1-14 | |
| X | Sousa C; Linkner J; Dang I; Guerin C; Nemethova M; Derivery E; Lakisic G; Small J; Blanchoin L; Faix J; Gautreau A: "Abstract 116: Arpin, a Novel Protein that Inhibits the Arp2/3 Complex, Controls Lamellipodium Protrusion and Cell Migration.",<br>Annual Meeting of the American-Society-for-Cell-Biology (ASCB) - TUESDAY- ORAL PRESENTATIONS; Denver, CO, USA<br>Molecular Biology of the Cell,<br>vol. 22<br>3 July 2011 (2011-07-03), XP002716381,<br>ISSN: 1059-1524<br>Retrieved from the Internet:<br>URL:http://ascb.org/files/Past-AM-Meetings/2011_Abstracts.pdf<br>[retrieved on 2013-11-14]<br>* Abstract 116;<br>page 64 - page 65 *<br>-----<br>-/-- | 1,15 | TECHNICAL FIELDS SEARCHED (IPC)<br>A61K<br>C07K<br>G01N<br>A61P |

The present search report has been drawn up for all claims

| Place of search | Date of completion of the search | Examiner |
|---|---|---|
| Munich | 20 November 2013 | Habedanck, Robert |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 30 6198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | C. TO ET AL: "Synthetic Triterpenoids Target the Arp2/3 Complex and Inhibit Branched Actin Polymerization", JOURNAL OF BIOLOGICAL CHEMISTRY, vol. 285, no. 36, 3 September 2010 (2010-09-03), pages 27944-27957, XP055088394, ISSN: 0021-9258, DOI: 10.1074/jbc.M110.103036 * abstract page 27944, right column, paragraph 2 * ----- | 1-15 | |
| T | IRENE DANG ET AL: "Inhibitory signalling to the Arp2/3 complex steers cell migration", NATURE, vol. 503, no. 7475, 16 October 2013 (2013-10-16), pages 281-284, XP055088178, ISSN: 0028-0836, DOI: 10.1038/nature12611 * abstract * ----- | | TECHNICAL FIELDS SEARCHED (IPC) |

The present search report has been drawn up for all claims

| Place of search | Date of completion of the search | Examiner |
|---|---|---|
| Munich | 20 November 2013 | Habedanck, Robert |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 30 6198

This annex lists the patent family members relating to the patent documents cited in the above-mentioned European search report. The members are as contained in the European Patent Office EDP file on
The European Patent Office is in no way liable for these particulars which are merely given for the purpose of information.

20-11-2013

| Patent document cited in search report | Publication date | Patent family member(s) | Publication date |
|---|---|---|---|
| WO 2010053772 A2 | 14-05-2010 | US 2011250218 A1 | 13-10-2011 |
| | | WO 2010053772 A2 | 14-05-2010 |
| WO 03046152 A2 | 05-06-2003 | AU 2002359567 A1 | 10-06-2003 |
| | | EP 1487989 A2 | 22-12-2004 |
| | | WO 03046152 A2 | 05-06-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080234183 A


### Non-patent literature cited in the description


- **ALTSCHUL et al.** *J. Mol. Biol.,* 1990, vol. 215, 403
- **MERRIFIELD et al.** *J. Am. Chem. Soc.,* 1964, vol. 85, 2149
- **STEFFEN et al.** *J. Cell. Sci.,* 31 July 2013
- **WU, C. et al.** Arp2/3 is critical for lamellipodia and response to extracellular matrix cues but is dispensable for chemotaxis. *Cell,* 2012, vol. 148, 973-987
- **SURANENI, P. et al.** The Arp2/3 complex is required for lamellipodia extension and directional fibroblast cell migration. *J Cell Biol,* 2012, vol. 197, 239-251
- **MULLINS, R. D. ; HEUSER, J. A. ; POLLARD, T. D.** The interaction of Arp2/3 complex with actin: nucleation, high affinity pointed end capping, and formation of branching networks of filaments. *Proc Natl Acad Sci USA,* 1998, vol. 95, 6181-6186
- **INSALL, R. H. ; MACHESKY, L. M.** Actin dynamics at the leading edge: from simple machinery to complex networks. *Dev Cell,* 2009, vol. 17, 310-322
- **PADRICK, S. B. ; ROSEN, M. K.** Physical mechanisms of signal integration by WASP family proteins. *Annu Rev Biochem,* 2010, vol. 79, 707-735
- **RIDLEY, A. J.** Life at the leading edge. *Cell,* 2011, vol. 145, 1012-1022
- **DERIVERY, E. et al.** The Arp2/3 activator WASH controls the fission of endosomes through a large multiprotein complex. *Dev Cell,* 2009, vol. 17, 712-723
- **SUETSUGU, S. ; GAUTREAU, A.** Synergistic BAR-NPF interactions in actin-driven membrane remodeling. *Trends Cell Biol,* 2012, vol. 22, 141-150
- **POLLARD, T. D.** Regulation of actin filament assembly by Arp2/3 complex and formins. *Annu Rev Biophys Biomol Struct,* 2007, vol. 36, 451-477
- **ROCCA, D. L. ; MARTIN, S. ; JENKINS, E. L. ; HANLEY, J. G.** Inhibition of Arp2/3-mediated actin polymerization by PICK1 regulates neuronal morphology and AMPA receptor endocytosis. *Nat Cell Biol,* 2008, vol. 10, 259-271
- **MARITZEN, T. et al.** Gadkin negatively regulates cell spreading and motility via sequestration of the actin-nucleating ARP2/3 complex. *Proc Natl Acad Sci U S A,* 2012, vol. 109, 10382-10387
- **LAI, F. P. et al.** Arp2/3 complex interactions and actin network turnover in lamellipodia. *Embo J,* 2008, vol. 27, 982-992
- **CAI, L. ; MAKHOV, A. M. ; SCHAFER, D. A. ; BEAR, J. E.** Coronin 1B antagonizes cortactin and remodels Arp2/3-containing actin branches in lamellipodia. *Cell,* 2008, vol. 134, 828-842
- **HART, Y. ; ALON, U.** The utility of paradoxical components in biological circuits. *Mol Cell,* 2013, vol. 49, 213-221
- **NEILSON, M. P. et al.** Chemotaxis: a feedback-based computational model robustly predicts multiple aspects of real cell behaviour. *PLoS Biol,* 2011, vol. 9, e1000618
- **CASTRO-CASTRO, A. et al.** Coronin 1A promotes a cytoskeletal-based feedback loop that facilitates Rac1 translocation and activation. *Embo J,* 2011, vol. 30, 3913-3927
- **MACHACEK, M. ; DANUSER, G.** Morphodynamic profiling of protrusion phenotypes. *Biophys J,* 2006, vol. 90, 1439-1452
- **WEINER, O. D. ; MARGANSKI, W. A. ; WU, L. F. ; ALTSCHULER, S. J. ; KIRSCHNER, M. W.** An actin-based wave generator organizes cell motility. *PLoS Biol,* 2007, vol. 5, e221
- **BRANDMAN, O. ; MEYER, T.** Feedback loops shape cellular signals in space and time. *Science,* 2008, vol. 322, 390-395
- **ALLARD, J. ; MOGILNER, A.** Traveling waves in actin dynamics and cell motility. *Curr Opin Cell Biol,* 2012
- **GOSH, M. et al.** Cofilin promotes actin polymerization and defines the direction of cell motility. *Science,* 2004, vol. 304, 743-746
- **PETRIE, R. J. ; DOYLE, A. D. ; YAMADA, K. M.** Random versus directionally persistent cell migration. *Nat Rev Mol Cell Biol,* 2009, vol. 10, 538-549
- **PANKOV, R. et al.** A Rac switch regulates random versus directionally persistent cell migration. *J Cell Biol,* 2005, vol. 170, 793-802

- **SIDANI, M. et al.** Cofilin determines the migration behavior and turning frequency of metastatic cancer cells. *J Cell Biol,* 2007, vol. 179, 777-791
- **LI, A. et al.** Rac1 drives melanoblast organization during mouse development by orchestrating pseudopod- driven motility and cell-cycle progression. *Dev Cell,* 2011, vol. 21, 722-734
- **LOISEL, T. P. ; BOUJEMAA, R. ; PANTALONI, D. ; CARLIER, M. F.** Reconstitution of actin-based motility of Listeria and Shigella using pure proteins. *Nature,* 1999, vol. 401, 613-616
- **EDGAR, R. C.** MUSCLE: multiple sequence alignment with high accuracy and high throughput. *Nucleic Acids Res,* 2004, vol. 32, 1792-1797
- **CLAMP, M. ; CUFF, J. ; SEARLE, S. M. ; BARTON, G. J.** The Jalview Java alignment editor. *Bioinformatics,* 2004, vol. 20, 426-427
- **MCGUFFIN, L. J. ; BRYSON, K. ; JONES, D. T.** The PSIPRED protein structure prediction server. *Bioinformatics,* 2000, vol. 16, 404-405
- **WARD, J. J. ; MCGUFFIN, L. J. ; BRYSON, K. ; BUXTON, B. F. ; JONES, D. T.** The DISOPRED server for the prediction of protein disorder. *Bioinformatics,* 2004, vol. 20, 2138-2139
- **BLANCHOIN, L. et al.** Direct observation of dendritic actin filament networks nucleated by Arp2/3 complex and WASP/Scar proteins. *Nature,* 2000, vol. 404, 1007-1011
- **DUMORTIER, J. G. ; MARTIN, S. ; MEYER, D. ; ROSA, F. M. ; DAVID, N. B.** Collective mesendoderm migration relies on an intrinsic directionality signal transmitted through cell contacts. *Proc Natl Acad Sci USA,* 2012, vol. 109, 16945-16950
- **MONTERO, J. A. ; KILIAN, B. ; CHAN, J. ; BAYLISS, P. E. ; HEISENBERG, C. P.** Phosphoinositide 3-kinase is required for process outgrowth and cell polarization of gastrulating mesendodermal cells. *Curr Biol,* 2003, vol. 13, 1279-1289
- **LOMMEL, S. et al.** Actin pedestal formation by enteropathogenic Escherichia coli and intracellular motility of Shigella flexneri are abolished in N-WASP-defective cells. *EMBO Rep,* 2001, vol. 2, 850-857
- **MILLARD, T. H. ; BEHRENDT, B. ; LAUNAY, S. ; FUTTERER, K. ; MACHESKY, L. M.** Identification and characterisation of a novel human isoform of Arp2/3 complex subunit p16-ARC/ARPC5. *Cell Motil Cytoskeleton,* 2003, vol. 54, 81-90
- **DUMONTIER, M. ; HOCHT, P. ; MINTERT, U. ; FAIX, J.** Rac1 GTPases control filopodia formation, cell motility, endocytosis, cytokinesis and development in Dictyostelium. *J Cell Sci,* 2000, vol. 113, 2253-2265
- **DERIVERY, E. et al.** Free Brickl is a trimeric precursor in the assembly of a functional wave complex. *PLoS ONE,* 2008, vol. 3, e2462
- **MICHELOT, A. et al.** Actin-filament stochastic dynamics mediated by ADF/cofilin. *Curr Biol,* 2007, vol. 17, 825-833
- **MACHESKY, L. M. et al.** Scar, a WASp-related protein, activates nucleation of actin filaments by the Arp2/3 complex. *Proc Natl Acad Sci USA,* 1999, vol. 96, 3739-3744
- **MARCHAND, J. B. ; KAISER, D. A. ; POLLARD, T. D. ; HIGGS, H. N.** Interaction of WASP/Scar proteins with actin and vertebrate Arp2/3 complex. *Nat Cell Biol,* 2001, vol. 3, 76-82
- **URBAN, E. ; JACOB, S. ; NEMETHOVA, M ; RESCH, G. P. ; SMALL, J. V.** Electron tomography reveals unbranched networks of actin filaments in lamellipodia. *Nat Cell Biol,* 2010, vol. 12, 429-435
- **DOITSIDOU, M. et al.** Guidance of primordial germ cell migration by the chemokine SDF-1. *Cell,* 2002, vol. 111, 647-659
- **HAUPTMANN, G. ; GERSTER, T.** Two-color whole-mount in situ hybridization to vertebrate and Drosophila embryos. *Trends Genet,* 1994, vol. 10, 266
- **SCHIRENBECK, A. ; BRETSCHNEIDER, T. ; ARASADA, R. ; SCHLEICHER, M. ; FAIX, J.** The Diaphanous-related formin dDia2 is required for the formation and maintenance of filopodia. *Nat Cell Biol,* 2005, vol. 7, 619-625
- **FAIX, J. ; KREPPEL, L. ; SHAULSKY, G. ; SCHLEICHER, M. ; KIMMEL, A. R.** A rapid and efficient method to generate multiple gene disruptions in Dictyostelium discoideum using a single selectable marker and the Cre-loxP system. *Nucleic Acids Res,* 2004, vol. 32, e143
- **BIECHE, I. et al.** Quantification of estrogen receptor alpha and beta expression in sporadic breast cancer. *Oncogene,* 2001, vol. 20, 8109-8115
- **TROYANOVSKY, S. M. ; GUELSTEIN, V. I. ; TCHIPYSHEVA, T. A. ; KRUTOVSKIKH, V. A. ; BANNIKOV, G. A.** Patterns of expression of keratin 17 in human epithelia: dependency on cell position. *J Cell Sci,* 1989, vol. 93, 419-426

SEQUENCE LISTING

<110> CENTRE NATIONAL DE LA RECHERCHE SCIENTIFIQUE

<120> USE OF ARPIN A NEW INHIBITOR OF THE ARP2/3 COMPLEX FOR THE DIAGNOSIS AND TREATMENT OF DISEASES

<130> 644ep369

<160> 41

<170> PatentIn version 3.5

<210> 1
<211> 226
<212> PRT
<213> Homo sapiens

<400> 1

```
Met Ser Arg Ile Tyr His Asp Gly Ala Leu Arg Asn Lys Ala Val Gln
1               5                   10                  15

Ser Val Arg Leu Pro Gly Ala Trp Asp Pro Ala Ala His Gln Gly Gly
            20                  25                  30

Asn Gly Val Leu Leu Glu Gly Glu Leu Ile Asp Val Ser Arg His Ser
        35                  40                  45

Ile Leu Asp Thr His Gly Arg Lys Glu Arg Tyr Tyr Val Leu Tyr Ile
    50                  55                  60

Arg Pro Ser His Ile His Arg Arg Lys Phe Asp Ala Lys Gly Asn Glu
65                  70                  75                  80

Ile Glu Pro Asn Phe Ser Ala Thr Arg Lys Val Asn Thr Gly Phe Leu
                85                  90                  95

Met Ser Ser Tyr Lys Val Glu Ala Lys Gly Asp Thr Asp Arg Leu Thr
            100                 105                 110

Pro Glu Ala Leu Lys Gly Leu Val Asn Lys Pro Glu Leu Leu Ala Leu
        115                 120                 125

Thr Glu Ser Leu Thr Pro Asp His Thr Val Ala Phe Trp Met Pro Glu
    130                 135                 140

Ser Glu Met Glu Val Met Glu Leu Glu Leu Gly Ala Gly Val Arg Leu
145                 150                 155                 160

Lys Thr Arg Gly Asp Gly Pro Phe Leu Asp Ser Leu Ala Lys Leu Glu
                165                 170                 175
```

Ala Gly Thr Val Thr Lys Cys Asn Phe Thr Gly Asp Gly Lys Thr Gly
180 185 190

Ala Ser Trp Thr Asp Asn Ile Met Ala Gln Lys Cys Ser Lys Gly Ala
195 200 205

Ala Ala Glu Ile Arg Glu Gln Gly Asp Gly Ala Glu Asp Glu Glu Trp
210 215 220

Asp Asp
225

<210> 2
<211> 226
<212> PRT
<213> Mus musculus

<400> 2

Met Ser Arg Ile Tyr Gln Asp Ser Ala Leu Arg Asn Lys Ala Val Gln
1 5 10 15

Ser Ala Arg Leu Pro Gly Thr Trp Asp Pro Ala Thr His Gln Gly Gly
20 25 30

Asn Gly Ile Leu Leu Glu Gly Glu Leu Val Asp Val Ser Arg His Ser
35 40 45

Ile Leu Asp Ala His Gly Arg Lys Glu Arg Tyr Tyr Val Leu Tyr Ile
50 55 60

Gln Pro Ser Cys Ile His Arg Arg Lys Phe Asp Pro Lys Gly Asn Glu
65 70 75 80

Ile Glu Pro Asn Phe Ser Ala Thr Arg Lys Val Asn Thr Gly Phe Leu
85 90 95

Met Ser Ser Tyr Lys Val Glu Ala Lys Gly Asp Thr Asp Arg Leu Thr
100 105 110

Leu Glu Ala Leu Lys Ser Leu Val Asn Lys Pro Gln Leu Leu Glu Leu
115 120 125

Thr Glu Ser Leu Thr Pro Asp Gln Ala Val Ala Phe Trp Met Pro Glu
130 135 140

Ser Glu Met Glu Val Met Glu Leu Glu Leu Gly Thr Gly Val Arg Leu
145 150 155 160

```
Lys Thr Arg Gly Asp Gly Pro Phe Ile Asp Ser Leu Ala Lys Leu Glu
                165                 170                 175

Leu Gly Thr Val Thr Lys Cys Asn Phe Ala Gly Asp Gly Lys Thr Gly
            180                 185                 190

Ala Ser Trp Thr Asp Asn Ile Met Ala Gln Lys Ser Ser Glu Arg Asn
        195                 200                 205

Thr Ala Glu Ile Arg Glu Gln Gly Asp Gly Ala Glu Asp Glu Glu Trp
    210                 215                 220

Asp Asp
225
```

<210> 3
<211> 226
<212> PRT
<213> Xenopus tropicalis

<400> 3

```
Met Ser Arg Ile Tyr His Asn Thr Ser Leu Arg Asn Lys Pro Val His
1               5                   10                  15

Asp Glu Arg Ile Ala Gly Ser Trp Glu Pro Thr Ala Phe Gln Arg Gly
            20                  25                  30

Ala Gly Val Leu Leu Glu Gly Thr Leu Leu Asp Phe Ser Arg His Ser
        35                  40                  45

Leu Thr Asp Ser Lys Gly Lys Lys Glu Arg Trp Tyr Ile Leu Tyr Leu
    50                  55                  60

Met Pro Ser Lys Ile His Arg Arg His Phe Asp Ser Lys Gly Asn Glu
65                  70                  75                  80

Ile Glu Pro Asn Phe Ser Asp Thr Lys Lys Val Asn Thr Gly Phe Leu
                85                  90                  95

Met Ser Ser Tyr Lys Val Glu Ala Lys Gly Glu Ser Asp Lys Ile Ser
            100                 105                 110

Val Glu Glu Leu Ile Gln Leu Val Asn Lys Val Glu Leu Met Lys Ile
        115                 120                 125

Ser Glu Lys Tyr Thr Pro Arg Glu Thr Val Ala Phe Trp Leu Pro Glu
    130                 135                 140
```

Val Asp Val Glu Lys Thr Glu Leu Glu Leu Gly Glu Gln Leu Arg Val
145                 150                 155                 160

Lys Thr Met Gly Asp Ser Pro Phe Val Phe Ser Leu Ala Lys Val Asp
                165                 170                 175

Ser Gly Thr Val Thr Lys Cys Asn Phe Ala Gly Asp Ala Gln Ala Gly
            180                 185                 190

Ala Ser Trp Thr Asp Asn Ile Met Ala Gln Lys Ser Gln Ser Thr Ser
        195                 200                 205

Ala Cys Phe Glu Pro Arg Gly Gln Gly Asp Gly Ala Glu Asp Asp Glu
    210                 215                 220

Trp Asp
225

<210> 4
<211> 226
<212> PRT
<213> Danio rerio

<400> 4

Met Ser Arg Ile Tyr Asp Asn Thr Ala Leu Leu Asn Lys Pro Val His
1               5                   10                  15

Asn Glu Lys Leu Ser Phe Thr Trp Asp Pro Ile Val His Gln Ser Gly
            20                  25                  30

His Gly Val Ile Leu Glu Gly Thr Val Val Asp Phe Ser Arg His Ala
        35                  40                  45

Ile Thr Asp Val Lys Asn Arg Lys Glu Arg Tyr Asn Val Leu Tyr Ile
    50                  55                  60

Lys Pro Ser Arg Val His Arg Arg Lys Tyr Asp Ser Lys Gly Asn Glu
65                  70                  75                  80

Ile Glu Pro Asn Phe Ser Asp Thr Lys Lys Val Asn Thr Gly Phe Leu
                85                  90                  95

Met Ser Ser Phe Lys Val Glu Ala Lys Gly Glu Thr Asp Cys Leu Asp
            100                 105                 110

Glu Arg Gln Leu Arg Glu Ile Val Asn Lys Glu Gln Leu Val Lys Val
        115                 120                 125

Thr Ile Lys His Cys Pro Arg Glu Ala Phe Ala Phe Trp Ile Ser Glu
    130                 135                 140

Ala Glu Met Asp Lys Thr Glu Leu Glu Pro Gly Gln Glu Val Arg Leu
145                 150                 155                 160

Lys Thr Lys Gly Asp Gly Pro Phe Ile Phe Ser Ser Ala Lys Leu Asp
                165                 170                 175

Ser Gly Thr Val Thr Lys Cys Asn Phe Ala Gly Asp Glu Asn Ala Gly
            180                 185                 190

Ala Ser Trp Thr Glu Lys Ile Met Ala Asn Lys Ser Asn Gln Glu Asn
        195                 200                 205

Thr Gly Lys Ser Ala Ala Gln Gly Glu Gly Ala Asp Asp Asp Glu Trp
    210                 215                 220

Asp Asp
225

<210> 5
<211> 226
<212> PRT
<213> Strongylocentrotus purpuratus

<400> 5

Met Ser Arg Ile Tyr Asp Asn Lys Pro Leu Gln Ser Val Pro Val Gln
1               5                   10                  15

Asn Gln Thr Gly Asn Trp Asn Pro Gln Gln Phe Gln Ser Gly Ser Gly
            20                  25                  30

Ala Ile Leu Glu Gly Lys Ile Asp Ser Arg Ser Arg His Val Ile Thr
        35                  40                  45

Asp Ser Lys Gln Asn Lys Ser Arg Tyr Phe Val Leu His Val Arg Val
    50                  55                  60

Lys Ile Ala His Arg Arg Gln Phe Asn Ser Lys Gly Glu Glu Ile Glu
65                  70                  75                  80

Ser Asn Met Asp Ala Thr Gln Lys Val Asn Thr Gly Tyr Leu Met Ser
                85                  90                  95

Ser Tyr Lys Val Glu Ala Lys Gly Gln Thr Asp Arg Leu Thr Gln Ser
            100                 105                 110

```
Gln Leu Ser Ala Leu Val Asn Lys Pro Ser Leu Thr Ser Leu Thr Gln
        115                 120                 125

Arg His Thr Pro Glu Gly Cys Leu Ala Phe Trp Met Thr Glu Leu Glu
    130                 135                 140

Leu Lys Asp Val Glu Phe Glu Asp Gly Asp Val Ile Met Leu Arg Thr
145                 150                 155                 160

Lys Gly Asp Gly Pro Phe Ile Asp Ser Val Gly Lys Leu Asp Thr Ser
                165                 170                 175

Ser Lys Glu Met Thr Asn Met Ala Gly Ser Gly Gln Leu Gly Glu Ser
            180                 185                 190

Trp Thr Asp Lys Ile Met Thr Ile Lys Ser Ser Gln Asp Pro Pro Asn
        195                 200                 205

Pro Lys Pro Gly Gln Glu Glu Asn Glu Gly Ala Gly Asp Asp Glu Trp
    210                 215                 220

Asp Asp
225


<210>  6
<211>  224
<212>  PRT
<213>  Dictyostelium discoideum

<400>  6

Met Ser Ser Ser Thr Asn Tyr Ser Gly Thr Phe Thr Lys Glu Arg Phe
1               5                   10                  15

Cys Gly Gly Asp Gly Phe Ile Val Glu Gly Ile Leu Gln Lys His Asn
            20                  25                  30

Ile His Thr Val Phe Leu Gln Ala Lys Gln Pro Leu Gln Asp Thr Val
        35                  40                  45

Leu Pro Thr Arg Tyr Val Ala Ile Thr Ile Glu Phe Asp Asn Ala Val
    50                  55                  60

Lys Arg Asn Trp Lys Asp Gly Val Glu Ile Pro Pro Lys Met Thr Arg
65                  70                  75                  80

Ala Thr Ala Ile Thr Lys Gly Tyr Leu Asn Lys Phe Asp Arg Val Gln
                85                  90                  95
```

Ala Thr Leu Val Asp Lys Ile Asn Ala Asp Gln Leu Ser Val Ala Ile
            100                 105                 110

Ser Asp Gly Asn Lys Asn Ser Ser Leu Pro Ser Asn Phe His Asp Asp
        115                 120                 125

Ala Ile Lys Asn Leu His Pro Asn Lys Lys Asn Tyr Phe Glu Phe Trp
    130                 135                 140

Ala Asp Val Asp Lys Phe Ser His Lys Ile Phe Lys Ser Gly Asp Glu
145                 150                 155                 160

Val Arg Ile Lys Thr Ile Gly Asp Ser Ile Phe Val Asp Ser Val Val
                165                 170                 175

Lys Met Asp Ser Asn Asn Ile Ala Asn Phe Ser Gly Asp Ser Asn Leu
            180                 185                 190

Gly Asp Ser Phe Ala Ser Asn Ile Met Lys Arg Tyr Asp Gln Ile Glu
        195                 200                 205

Ser Asn Asn Gln Gln Gln Gln Glu Glu Glu Asp Asp Asp Glu Trp Lys
    210                 215                 220

<210> 7
<211> 16
<212> PRT
<213> artificial sequence

<220>
<223> A motif from human and mouse Arpin

<400> 7

Glu Ile Arg Glu Gln Gly Asp Gly Ala Glu Asp Glu Glu Trp Asp Asp
1               5                   10                  15

<210> 8
<211> 15
<212> PRT
<213> artificial sequence

<220>
<223> A motif from frog Arpin

<400> 8

Glu Pro Arg Gly Gln Gly Asp Gly Ala Glu Asp Asp Glu Trp Asp
1               5                   10                  15

<210> 9
<211> 16

<212> PRT
<213> artificial sequence

<220>
<223> A motif from zebrafish Arpin

<400> 9

Lys Ser Ala Ala Gln Gly Glu Gly Ala Asp Asp Asp Glu Trp Asp Asp
1               5                   10                  15

<210> 10
<211> 17
<212> PRT
<213> artificial sequence

<220>
<223> A motif from sea urchin Arpin

<400> 10

Lys Pro Gly Gln Glu Glu Asn Glu Gly Ala Gly Asp Asp Glu Trp Asp
1               5                   10                  15

Asp

<210> 11
<211> 13
<212> PRT
<213> artificial sequence

<220>
<223> A motif from amoeba Arpin

<400> 11

Gln Gln Gln Gln Glu Glu Glu Asp Asp Asp Glu Trp Lys
1               5                   10

<210> 12
<211> 681
<212> DNA
<213> Homo sapiens

<400> 12
atgagccgca tctaccacga cggcgcgctc cggaacaagg cggtgcagag cgtccggctg 60

ccaggggcct gggaccccgc cgcccaccag gggggaaatg gtgtcctgct ggagggagaa 120

ctgatcgatg tatctcggca cagcatcttg gacactcatg gcaggaagga gcgctactac 180

gtgctgtata tccggcccag tcacatccat cgccgtaaat tcgacgccaa gggaaatgaa 240

atcgagccca acttcagcgc caccaggaag gtgaacacgg gcttcctcat gtcgtcctac 300

aaggtggaag ccaaggggga cactgacagg ctcacgcccg aggcgctgaa ggggctggtc 360

aacaagccag agctgctcgc gctgacagag agcctcaccc ccgaccacac agtggcgttc 420

tggatgcccg agtcagagat ggaggtgatg gaactcgagc tgggggccgg ggtacggctg 480

aagactcggg gcgatggtcc cttcctggat tcattggcca aacttgaggc tggaacagtg 540

accaagtgta atttcactgg tgatggaaag acaggggcat cctggacaga caacatcatg 600

gcccaaaagt gttcgaaggg ggctgcagcg gagatccgag agcaggggga tggggcagag 660

gacgaggagt gggatgactg a 681

<210> 13
<211> 21
<212> DNA
<213> artificial sequence

<220>
<223> Arpin mRNA target for shArpin#1

<400> 13
ggagaactga tcgatgtatc t 21

<210> 14
<211> 21
<212> DNA
<213> artificial sequence

<220>
<223> Arpin mRNA target for shArpin#2

<400> 14
gcttcctcat gtcgtcctac a 21

<210> 15
<211> 21
<212> DNA
<213> artificial sequence

<220>
<223> Arpin mRNA target for shArpin#3

<400> 15
gccttcctag acattacatg a 21

<210> 16
<211> 19
<212> DNA
<213> artificial sequence

<220>
<223> mRNA target for shArpC2#1

<400> 16
ccatgtatgt tgagtctaa 19

<210> 17
<211> 19
<212> DNA

<213> artificial sequence

<220>
<223> mRNA target for shArpC2#2

<400> 17
gctctaaggc ctatattca 19

<210> 18
<211> 21
<212> DNA
<213> artificial sequence

<220>
<223> non-targeting sh control

<400> 18
gcatatgtgc gtacctagca t 21

<210> 19
<211> 25
<212> DNA
<213> artificial sequence

<220>
<223> morpholino targeting Arpin

<400> 19
gttgtcataa atacgactca tcttc 25

<210> 20
<211> 25
<212> DNA
<213> artificial sequence

<220>
<223> control morpholino

<400> 20
cctcttacct cagttacaat ttata 25

<210> 21
<211> 35
<212> DNA
<213> artificial sequence

<220>
<223> DdArpin_BU primer

<400> 21
cgcggatccg catgagttca agtacaaatt atagt 35

<210> 22
<211> 34
<212> DNA
<213> artificial sequence

<220>

<223>  DdArpin_SD primer

<400>  22
cgcgtcgact ttatttccat tcatcatcat cttc                                   34

<210>  23
<211>  34
<212>  DNA
<213>  artificial sequence

<220>
<223>  oligonucleotide primer

<400>  23
gcgctgcagc atctgaaatt gcaactgata gttg                                   34

<210>  24
<211>  33
<212>  DNA
<213>  artificial sequence

<220>
<223>  oligonucleotide primer

<400>  24
gcgaagcttt cttctttacc ttcaaatttt cat                                    33

<210>  25
<211>  34
<212>  DNA
<213>  artificial seqeunce

<220>
<223>  oligonucleotide primer

<400>  25
cgcgtcgacg ttggttattt gattctattt gatc                                   34

<210>  26
<211>  25
<212>  DNA
<213>  artificial sequence

<220>
<223>  Primer Arpin-U

<400>  26
cttcctcatg tcgtcctaca aggtg                                             25

<210>  27
<211>  20
<212>  DNA
<213>  artificial sequence

<220>
<223>  Primer Arpin-L

<400>  27

ctgtcagcgc gagcagctct 20

<210> 28
<211> 21
<212> DNA
<213> artificial sequence

<220>
<223> Primer TBP-U

<400> 28
tgcacaggag ccaagagtga a 21

<210> 29
<211> 20
<212> DNA
<213> artificial sequence

<220>
<223> Primer TBP-L

<400> 29
cacatcacag ctccccacca 20

<210> 30
<211> 16
<212> PRT
<213> artificial sequence

<220>
<223> N-WASP A motif

<400> 30

Asp Glu Asp Asp Glu Glu Asp Phe Glu Asp Asp Asp Glu Trp Glu Asp
1 5 10 15

<210> 31
<211> 16
<212> PRT
<213> artificial sequence

<220>
<223> WAVE2 A motif

<400> 31

Asp Ser Glu Asp Asp Ser Ser Glu Phe Asp Glu Asp Asp Trp Ser Asp
1 5 10 15

<210> 32
<211> 16
<212> PRT
<213> artificial sequence

<220>
<223> WASH1 A motif

<400> 32

Pro Pro Gln Gln Pro Gln Ala Glu Glu Asp Glu Asp Asp Trp Glu Ser
1 5 10 15

<210> 33
<211> 16
<212> PRT
<213> artificial sequence

<220>
<223> human WASP A-motif

<400> 33

Gly Glu Asp Gln Ala Gly Asp Glu Asp Glu Asp Asp Glu Trp Asp Asp
1 5 10 15

<210> 34
<211> 16
<212> PRT
<213> artificial sequence

<220>
<223> human WASF2 A motif

<400> 34

Asp Ser Glu Asp Asp Ser Ser Glu Phe Asp Glu Asp Asp Trp Ser Asp
1 5 10 15

<210> 35
<211> 16
<212> PRT
<213> artificial sequence

<220>
<223> human WASF1 A motif

<400> 35

Ser Asp Ser Glu Asp Asp Ser Glu Phe Asp Glu Val Asp Trp Leu Glu
1 5 10 15

<210> 36
<211> 16
<212> PRT
<213> artificial sequence

<220>
<223> human WASF3 A-motif

<400> 36

Ser Asp Ser Asp Asp Asp Ser Glu Phe Asp Glu Asn Asp Trp Ser Asp
1 5 10 15

<210> 37

<211> 16
<212> PRT
<213> artificial sequence

<220>
<223> human WASF4 A-motif

<400> 37

Asp Ser Glu Asp Asp Ser Ser Glu Phe Asp Gly Asp Asp Trp Ser Asn
1 5 10 15

<210> 38
<211> 16
<212> PRT
<213> artificial sequence

<220>
<223> human FKB15 A-motif

<400> 38

Pro Leu Phe Gly Asp Asp Asp Asp Asp Asp Asp Ile Asp Trp Leu Gly
1 5 10 15

<210> 39
<211> 16
<212> PRT
<213> artificial sequence

<220>
<223> human WASH4 A-motif

<400> 39

Pro Pro Pro Gln Gln Pro Gln Ala Glu Asp Glu Asp Asp Trp Glu Ser
1 5 10 15

<210> 40
<211> 16
<212> PRT
<213> artificial sequence

<220>
<223> human STRAD A-motif

<400> 40

Gly Leu Val Thr Asn Leu Glu Glu Leu Glu Val Asp Asp Trp Glu Phe
1 5 10 15

<210> 41
<211> 16
<212> PRT
<213> artificial sequence

<220>
<223> human SACA1 A-motif

<400> 41

Pro Thr Glu Met Pro Gly Glu Asp Asp Ala Leu Ser Glu Trp Asn Glu
1               5                   10                  15